# EUROPÄISCHE PATENTSCHRIFT

(11) **EP 2 195 290 B1**
(45) Veröffentlichungstag und Bekanntmachung des Hinweises auf die Patenterteilung: **11.03.2015**
(21) Anmeldenummer: 08802077.1
(22) Anmeldetag: 12.09.2008
(51) Int. Cl.: C07D 209/54, A01N 43/38

(54) **HALOGENALKOXYSPIROCYCLISCHE TETRAM- UND TETRONSÄURE-DERIVATE**
HALOGEN ALKOXY SPIROCYCLIC TETRAMIC AND TETRONIC ACID DERIVATIVES
DÉRIVÉS HALOGÉNOALCOXYSPIROCYCLIQUES D'ACIDE TÉTRAMIQUE ET TÉTRONIQUE

(30) Priorität: 25.09.2007 EP 07117104
(43) Veröffentlichungstag der Anmeldung: 16.06.2010
(73) Patentinhaber: Bayer Intellectual Property GmbH, 40789 Monheim am Rhein (DE)
(72) Erfinder: FISCHER, Reiner, 40789 Monheim (DE); BRETSCHNEIDER, Thomas, 53797 Lohmar (DE); LEHR, Stefan, 65835 Liederbach (DE); ARNOLD, Christian, 40764 Langenfeld (DE); DITTGEN, Jan, 60316 Frankfurt (DE); FEUCHT, Dieter, 65760 Eschborn (DE); KEHNE, Heinz, 65719 Hofehim (DE); MALSAM, Olga, 51503 Rösrath (DE); ROSINGER, Christopher, Hugh, 65719 Hofheim (DE); FRANKEN, Eva-Maria, 69760 Limonest (FR); GÖRGENS, Ulrich, 40882 Ratingen (DE)
(74) Vertreter: BIP Patents
(86) Internationale Anmeldenummer: PCT/EP2008/007517
(87) Internationale Veröffentlichungsnummer: WO 2009/039975

(56) Entgegenhaltungen:
- EP-A- 0 528 156
- WO-A-2004/024688
- WO-A-2006/000355

## Beschreibung

Die vorliegende Erfindung betrifft neue halogenalkoxyspirocyclische Tetram- und Tetronsäure-Derivate, mehrere Verfahren zu ihrer Herstellung und ihre Verwendung als Schädlingsbekämpfungsmittel und/oder Herbizide. Auch Gegenstand der Erfindung sind selektiv herbizide Mittel, die Halogenalkoxyspirocyclische Tetram- und Tetronsäure-Derivate einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere halogenalkoxyspirocyclische Tetram- und Tetronsäure-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschtem Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyr-rolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049596, WO 05/066125, WO 05/092897, WO 06/000355, WO 06/029799, WO 06/056281, WO 06/056282, WO 06/089633, WO 07/048545, WO 07/073856, DE-A-2005/059892, WO 07/096058, WO 07/121868, WO 07/140881, WO 08/067873, WO 08/067910 und WO 08/067911. Außerdem sind ketalsubstituierte 1-H-Aryl-pyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et. al Bioscience, Biotechnology and Biochemistry 67, 1230-1238, (2003) bekannt. Der Zusatz von Safenem zu Ketoenolen ist ebenfalls prinzipiell aus der WO 03/013249 bekannt. Außerdem sind aus WO 06/024411 herbizide Mittel enthaltend Ketoenole bekannt.

Es ist bekannt, dass bestimmte Δ³-Dihydrofuran-2-on Derivate herbizide, insektizide oder akarizide Eigenschaften aufweisen: EP-A-528 156, EP-A-647 637, WO 95/26 954, WO 96/20 196, WO 96/25 395, WO 96/35 664, WO 97/01 535, WO 97/02 243, WO 97/36 868, WO 98/05 638, WO 98/06 721, WO 99/16 748, WO 98/25 928, WO 99/43 649, WO 99/48 869, WO 99/55 673, WO 01/23354, WO 01/74 770, WO 01/17 972, WO 04/024 688, WO 04/080 962, WO 04/111 042, WO 05/092 897, WO 06/000 355, WO 06/029 799, WO 06/089633, WO 07/048545 und WO 07/073856 und WO 08/067911.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) 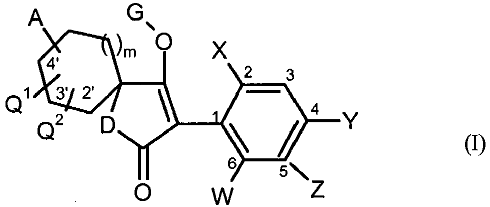 gefunden,
in welcher
- W: steht bevorzugt für Wasserstoff, C₁-C₆-Alkyl, C₂-C₆-Allcenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, Halogen, C₁-C₆-Alkoxy, C₁-C₄-Halogenalkyl, C₁-C₄-Halogenalkoxy oder Cyano,
- X: steht bevorzugt für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloallcyl substituiertes C₃-C₆-Cycloalkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenallcenyloxy, Nitro oder Cyano,
- Y und Z: stehen bevorzugt unabhängig voneinander für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenallcyl, C₁-C₆-Halogenalkoxyl, Cyano, C₂-C₆-Alkenyl, C₂-C₆-Alkinyl oder für einen der (Het)-arylreste 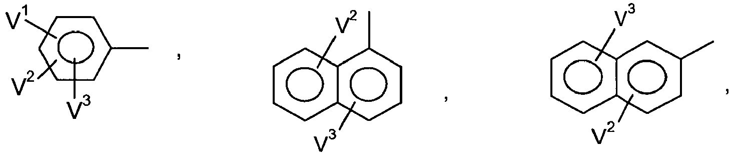 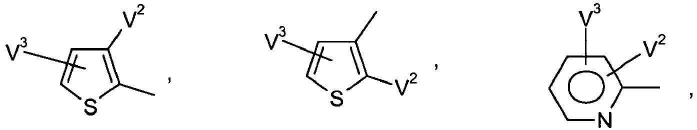 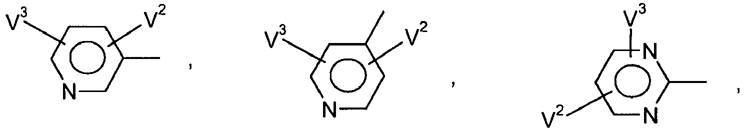 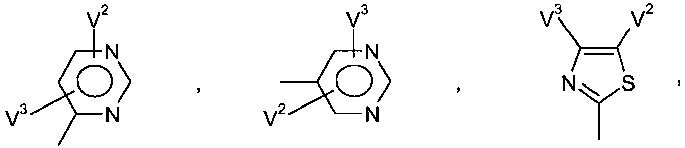 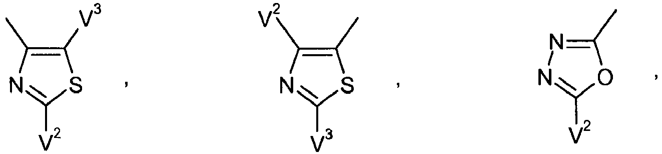 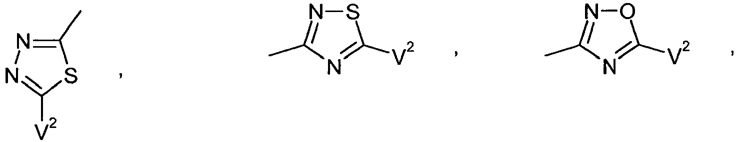 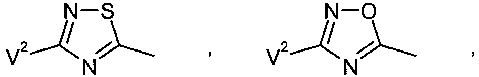 wobei im Falle von (Het)-aryl nur einer der Reste Y oder Z für (Het)-aryl stehen darf,
- V¹: steht bevorzugt für Wasserstoff, Halogen, C₁-C₁₂-Allcyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
- V² und V³: stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy,
- A: steht bevorzugt für einfach bis siebenfach durch Fluor, Chlor, Brom und/oder Jod substituiertes C₁-C₄-Alkoxy oder für einfach bis fünffach durch Fluor, Chlor und/oder Brom substituiertes C₃-C₆-Cycloalkyl-C₁-C₂-alkoxy, welches gegebenenfalls durch C₁-C₂-Alkyl, C₁-C₂-Halogenalkyl oder C₁-C₂-Alkoxy substituiert sein kann,
- D: steht bevorzugt für NH (1) oder Sauerstoff (2),
- Q¹ und Q²: stehen bevorzugt unabhängig voneinander für Wasserstoff, C₁-C₆-Alkyl, C₁-C₂-Halogenalkyl oder C₁-C₄-Alkoxy,
- m: steht bevorzugt für die Zahl 0 oder 1,
- G: steht bevorzugt für Wasserstoff (a) oder für eine der Gruppen 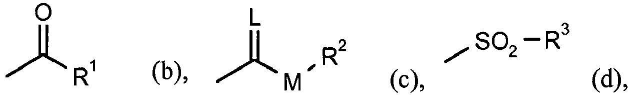 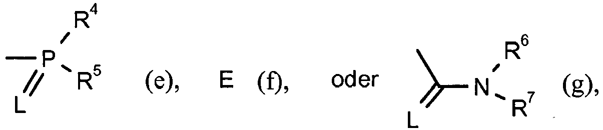 in welchen
E für ein Metallion oder ein Ammoniumion steht,
L für Sauerstoff oder Schwefel steht und
M für Sauerstoff oder Schwefel steht,
R¹ steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff,
R² steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
R³ steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
R⁴ und R⁵ Stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogen-alkylthio, C₁-C₄-Alkyl oder C₁-C₄-Halogenalkyl substituiertes Phenyl, Phenoxy oder Phenylthio,
R⁶ und R⁷ stehen unabhängig voneinander bevorzugt für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Al-kylenrest, in welchem gegebenenfalls eine Methylengruppe durch Sauerstoff oder Schwefel ersetzt ist.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung von D für NH (1) und D für O (2) ergeben sich folgende hauptsächliche Strukturen (I-1) bis (1-2): 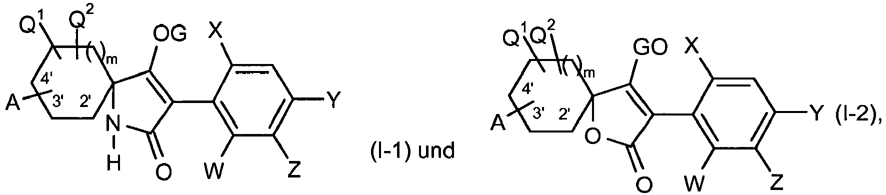 worin
A, G, Q¹, Q², m, W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn D für NH (1) steht, 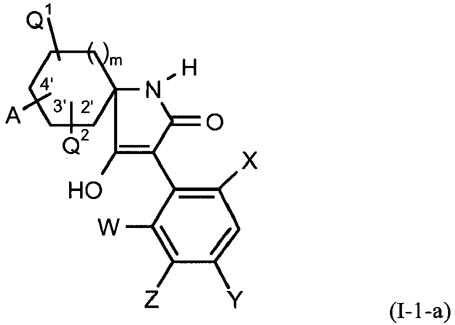 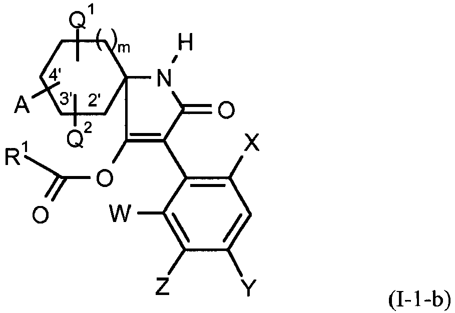 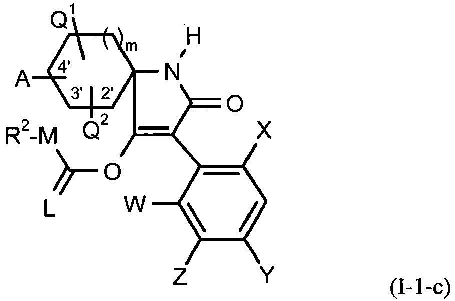 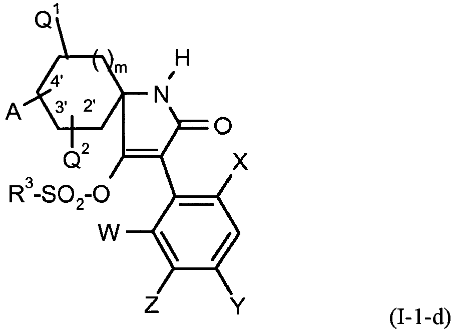 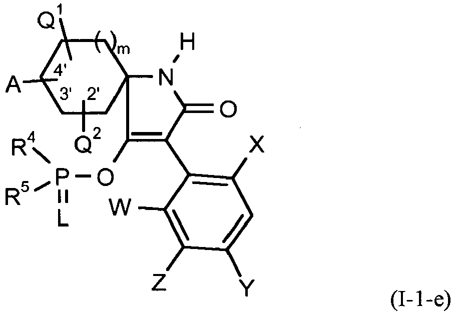 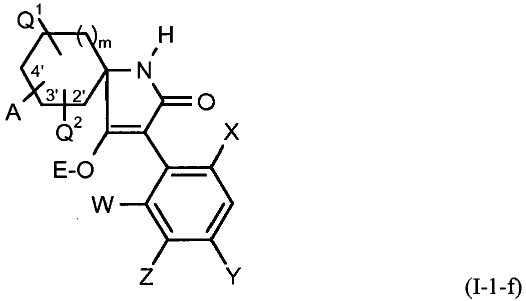 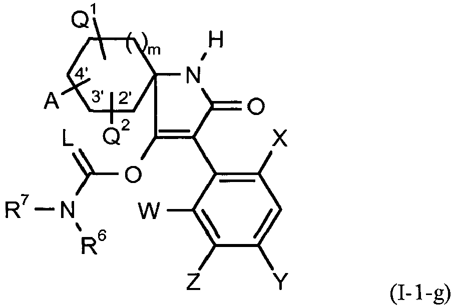 worin
A, E, L, M, Q¹, Q², m, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn D für O (2) steht, 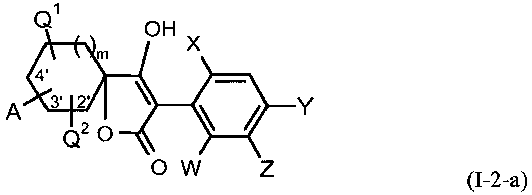 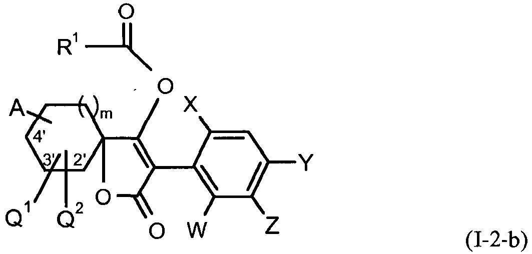 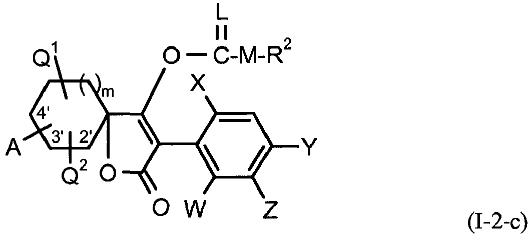 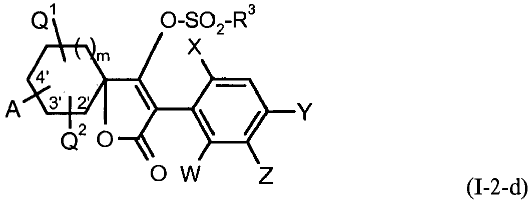 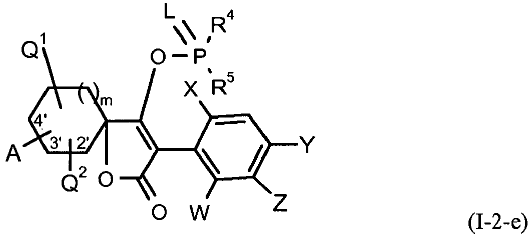 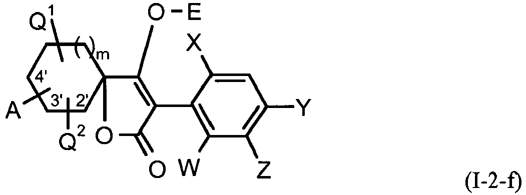 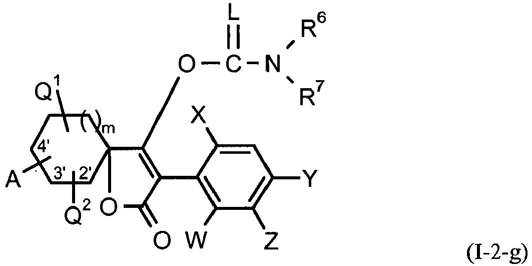 worin
A, E, L, M, Q¹, Q², m, W, X, Y, Z, R¹, R², R³, R⁴, R⁵, R⁶ und R⁷ die oben angegebene Bedeutung haben.

Weiterhin wurde gefunden, dass man die neuen Verbindungen der Formel (I) nach den im Folgenden beschriebenen Verfahren erhält:
(A) Man erhält Verbindungen der Formel (I-1-a) 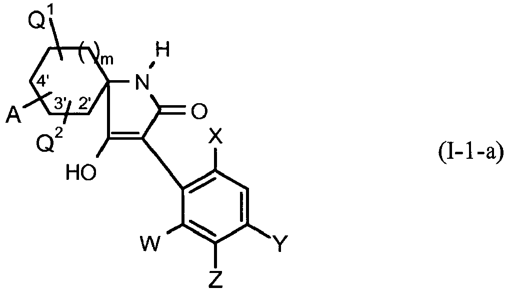 in welcher
   A, Q¹, Q², m, W, X, Y und Z die oben angegebenen Bedeutungen haben,
   wenn man
   Verbindungen der Formel (II) 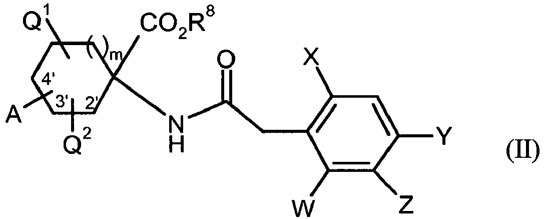 in welcher
   A, Q¹, Q², m, W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man Verbindungen der Formel (I-2-a) 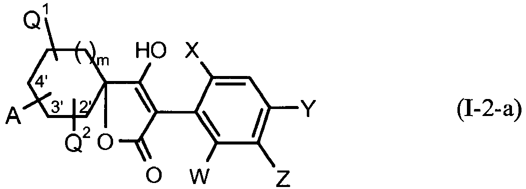 in welcher
   A, Q¹, Q², m, W, X, Y und Z die oben angegebenen Bedeutungen haben,
   erhält, wenn man
   Verbindungen der Formel (III) 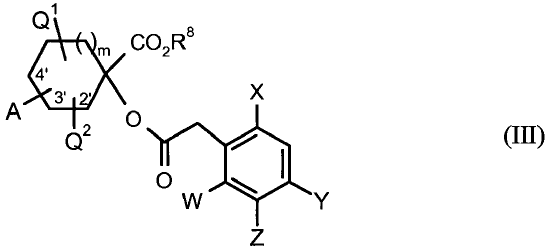 in welcher
   A, Q¹, Q², m, W, X, Y, Z und R⁸ die oben angegebenen Bedeutungen haben,
   in Gegenwart eines Verdünnungsmittels und in Gegenwart einer Base intramolekular kondensiert.
   Außerdem wurden gefunden
(C) dass man die Verbindungen der oben gezeigten Formeln (I-1-b) bis (I-2-b), in welchen R¹, A, Q¹, Q², m, W, X, Y und Z die oben angegebenen Bedeutungen haben, erhält, wenn man Verbindungen der oben gezeigten Formeln (I-1-a) bis (I-2-a), in welchen A, Q¹, Q², m, W, X, Y und Z die oben angegebenen Bedeutungen haben, jeweils
   α) mit Verbindungen der Formel (IV) 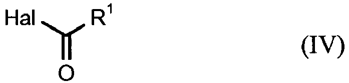 in welcher
      R¹ die oben angegebene Bedeutung hat und
      Hal für Halogen (insbesondere Chlor oder Brom) steht
      oder
   β) mit Carbonsäureanhydriden der Formel (V)

      R¹-CO-O-CO-R¹ (V)

      in welcher
      R¹ die oben angegebene Bedeutung hat,
      gegebenenfalls in Gegenwart eines Verdünnungsmittels und gegebenenfalls in Gegenwart eines Säurebindemittels umsetzt;
(D) dass man die Verbindungen der oben gezeigten Formeln (I-1-c) bis (I-2-c), in welchen R², A, Q¹, Q², m, W, M, X, Y und Z die oben angegebenen Bedeutungen haben und L für Sauerstoff steht, erhält, wenn man Verbindungen der oben gezeigten Formeln (I-1-a) bis (I-2-a), in welchen A, Q¹, Q², m, W, X, Y und Z die oben angegebenen Bedeutungen haben, jeweils
   mit Chlorameisensäureestern oder Chlorameisensäurethioestern der Formel (VI)

   R²-M-CO-Cl (VI)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formeln (I-1-c) bis (I-2-c), in welchen R², A, Q¹, Q², m, W, M, X, Y und Z die oben angegebenen Bedeutungen haben und L für Schwefel steht, erhält, wenn man Verbindungen der oben gezeigten Formeln (I-1-a) bis (1-2-a), in welchen A, Q¹, Q², m, W, X, Y und Z die oben angegebenen Bedeutungen haben, jeweils
   mit Chlormonothioameisensäureestern oder Chlordithioameisensäureestern der Formel (VII) 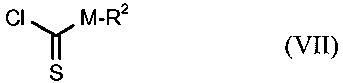 in welcher
   M und R² die oben angegebenen Bedeutungen haben,
   gegebenenfalls in Gegenwart eines Verdünnungsmittels und gegebenenfalls in Gegenwart
   eines Säurebindemittels umsetzt,
(F) dass man Verbindungen der oben gezeigten Formeln (I-1-d) bis (I-2-d), in welchen R³, A, W, Q¹, Q², m, X, Y und Z die oben angegebenen Bedeutungen haben, erhält, wenn man Verbindungen der oben gezeigten Formeln (I-1-a) bis (I-2-a), in welchen A, B, Q¹, Q², m, W, X, Y und Z die oben angegebenen Bedeutungen haben, jeweils
   mit Sulfonsäurechloriden der Formel (VIII)

   R³-SO₂-Cl (VIII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formeln (I-1-e) bis (I-2-e), in welchen L, R⁴, R⁵, A, Q¹, Q², m, W, X, Y und Z die oben angegebenen Bedeutungen haben, erhält, wenn man Verbindungen der oben gezeigten Formeln (I-1-a) bis (I-2-a), in welchen A, Q¹, Q², m, W, X, Y und Z die oben angegebenen Bedeutungen haben, jeweils
   mit Phosphorverbindungen der Formel (IX) 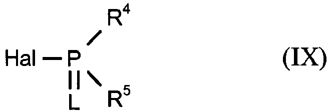 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-f) bis (I-2-f), in welchen E, A, Q¹, Q², m, W, X, Y und Z die oben angegebenen Bedeutungen haben, erhält, wenn man Verbindungen der Formeln (I-1-a) bis (I-2-a), in welchen A, Q¹, Q², m, W, X, Y und Z die oben angegebenen Bedeutungen haben, jeweils
   mit Metallverbindungen oder Aminen der Formeln (X) oder (XI)

   Me(OR¹⁰)ₜ (X)

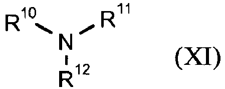

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(I) dass man Verbindungen der oben gezeigten Formeln (I-1-g) bis (I-2-g), in welchen L, R⁶, R⁷, A, Q¹, Q², m, W, X, Y und Z die oben angegebenen Bedeutungen haben, erhält, wenn man Verbindungen der oben gezeigten Formeln (I-1-a) bis (I-2-a), in welchen A, Q¹, Q², m, W, X, Y und Z die oben angegebenen Bedeutungen haben, jeweils
   α) mit Isocyanaten oder Isothiocyanaten der Formel (XII)

      R⁶-N=C=L (XII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIII) 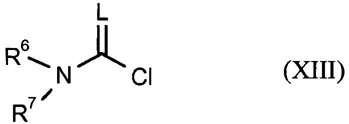 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt,
   (Jα) dass man Verbindungen der oben gezeigten Formeln (I-1a) bis (I-1-g), in welchen A, D, G, Q¹, Q², m, W, X, Y und Z die oben angegebene Bedeutung haben, erhält, wenn man Verbindungen der Formeln (I-1-a') bis (I-2-g'), in welchen A, D, G, Q¹, Q², m, W, X und Y die oben genannte Bedeutung haben und Z' bevorzugt für Brom oder Iod steht 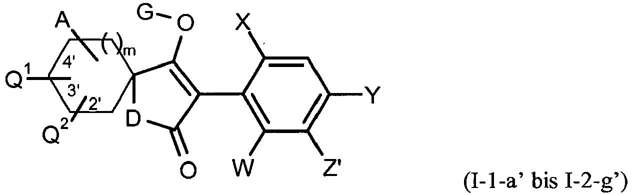 und
   (Jβ) dass man Verbindungen der oben gezeigten Formeln (I-1-a) bis (I-2-g), in welchen A, D, G, Q¹ Q², m, W, X, Y und Z die oben angegebene Bedeutung haben, erhält, wenn man Verbindungen der Formeln (I-1-a") bis (I-2-g"), in welchen, D, G, Q¹, Q², m, W, X und Z die oben genannte Bedeutung haben und Y' bevorzugt für Brom oder Iod steht 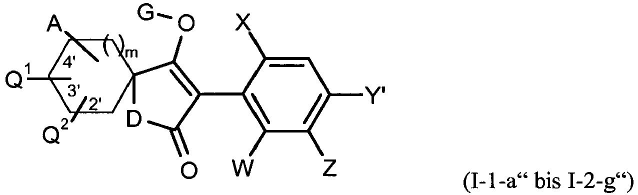 mit kupplungsfähigen (Het)-arylderivaten, z.B. Phenylboronsäuren der Formeln (XVα) und (XVβ) 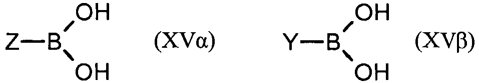 oder deren Ester in Gegenwart eines Lösungsmittels, in Gegenwart eines Katalysators (z. B. Pd-Salze oder Pd-Komplexe) und in Gegenwart einer Base (z.B. Natriumcarbonat, Kaliumphosphat) kuppelt. Die Verbindungen der Formel (XV) sind teilweise bekannt, teilweise käuflich oder lassen sich nach im Prinzip bekannten Verfahren herstellen.

Weiterhin wurde gefunden, dass die neuen Verbindungen der Formel (I) eine gute Wirksamkeit als Schädlingsbekämpfungsmittel, vorzugsweise als Insektizide und/oder Akarizide und/oder Herbizide aufweisen und darüber hinaus häufig sehr gut pflanzenverträglich, insbesondere gegenüber Kulturpflanzen, sind.

Überraschenderweise wurde nun auch gefunden, dass bestimmte halogenalkoxyspirocyclische Tetram- und Tetronsäure-Derivate bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens eine Verbindung der Formel (I), in welcher A, D, G, Q¹, Q², m, W, X, Y und Z die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-pro-penylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethyl-ester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyl-oxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-iso-propyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethyl-ester (vgl. auch verwandte Verbindungen in EP-A-269806 und EP-A-333131), 5-(2,4-Dichlorbenzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chin-olin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethyl-sulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxy-benzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 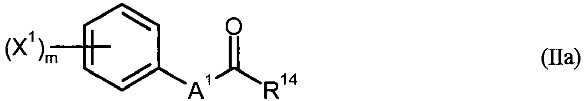 oder der allgemeinen Formel (IIb) 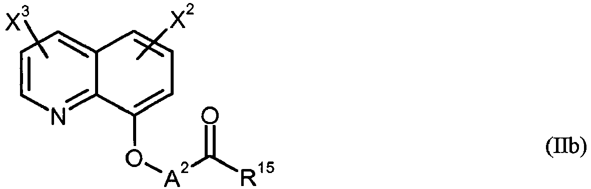 oder der Formel (IIc) 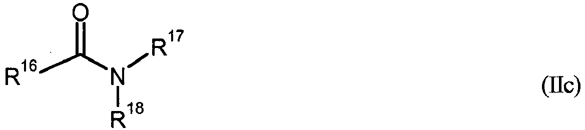 wobei
- m: für eine Zahl 0, 1, 2, 3, 4 oder 5 steht,
- A¹: für eine der nachstehend skizzierten divalenten heterocyclischen Gruppierungen steht, 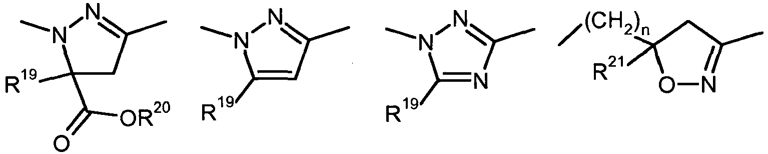
- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
- R¹⁵: für Hydroxy, Mercapto, Amino, C₁-C₇-Alkoxy, C₁-C₆-Alkylthio, C₁-C₆-Alkenyloxy, C₁-C₆-Alkenyloxy-C₁-C₆-alkoxy, C₁-C₆-Alkylamino oder Di-(C₁-C₄-alkyl)-amino steht,
- R¹⁶: für gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C_{I}-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, R ¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5-oder 6-gliedrigen Carboxyclus bilden, substituiertes C₃-C₆-Alkandiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 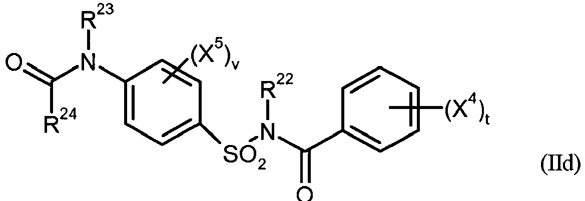 oder der allgemeinen Formel (IIe) 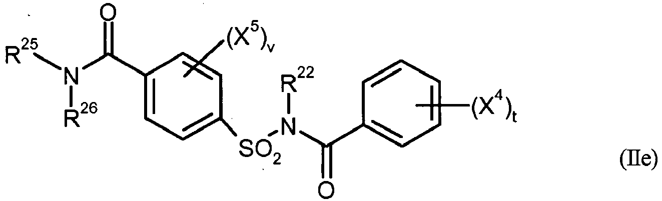 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cyclo-alkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
In den als bevorzugt genannten Restedefinitionen steht Halogen für Fluor, Chlor, Brom und Iod, insbesondere für Fluor, Chlor und Brom.
   - W: steht besonders bevorzugt für Wasserstoff, Chlor, Brom, C₁-C4-Alkyl, C₂-C₄-Alkenyl, C₂-C₄-Allcinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy,
   - X: steht besonders bevorzugt für Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano,
   - Y und Z: stehen besonders bevorzugt unabhängig voneinander für Wasserstoff, Fluor, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste, 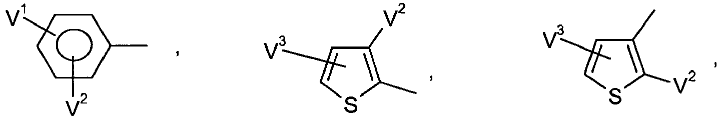 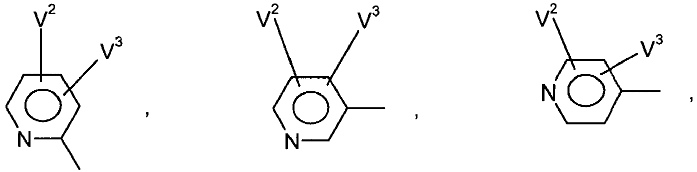 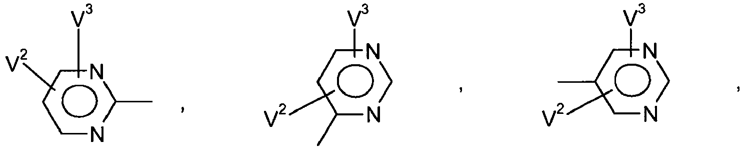 wobei im Falle von (Het)-aryl nur einer der Reste Y oder Z für (Het)-aryl stehen darf,
   - V¹: steht besonders bevorzugt für Wasserstoff, Fluor, Chlor, Brom, C₁-C₆-Alkyl, C₁-C₄-Alkoxy, C₁-C₂- Halogenalkyl, C₁-C₂-Halogenalkoxy, Nitro, Cyano oder jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
   - V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy,
   - A: steht besonders bevorzugt für einfach bis fünffach durch Fluor, Chlor und/oder Brom substituiertes C₁-C₄-Alkoxy oder für einfach bis dreifach durch Fluor und/oder Chlor substituiertes C₃-C₆-Cycloalkyl-C₁-C₂-alkoxy,
   - D: steht besonders bevorzugt für NH (1) oder Sauerstoff (2),
   - Q¹ und Q²: stehen besonders bevorzugt unabhängig voneinander für Wasserstoff, Methyl, Ethyl, Trifluormethyl, Methoxy oder Ethoxy,
   - m: steht besonders bevorzugt für die Zahl 0 oder 1,
   - G: steht besonders bevorzugt für Wasserstoff (a) oder für eine der Gruppen 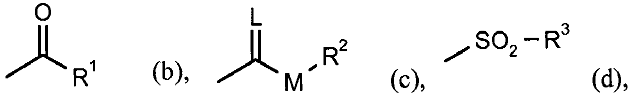 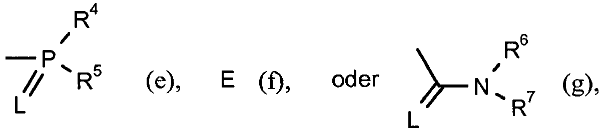 in welchen
   E für ein Metallion oder ein Ammoniumion steht,
   L für Sauerstoff oder Schwefel steht und
   M für Sauerstoff oder Schwefel steht,
   R¹ steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
   für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
   für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
   für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
   für gegebenenfalls einfach bis zweifach durch Fluor, Chlor, Brom oder C₁-C₄-Alkyl substituiertes Phenoxy-C₁-C₅-alkyl oder
   für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁ -C₅-alkyl,
   R² steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
   für gegebenenfalls einfach bis zweifach durch Fluor, Chlor, C₁-C₄-Alkyl oder C₁-C₄-Alkoxy substituiertes C₃-C₇-Cycloalkyl oder
   für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl,
   R³ steht besonders bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl,
   R⁴ und R⁵ stehen unabhängig voneinander besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio,
   R⁶ und R⁷ stehen unabhängig voneinander besonders bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Brom, Methyl, Ethyl, Vinyl, Ethinyl, Propinyl, Cyclopropyl, Methoxy, Ethoxy oder Trifluormethyl,
- X: steht ganz besonders bevorzugt für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano,
- Y und Z: stehen ganz besonders bevorzugt unabhängig voneinander für Wasserstoff, Fluor, Chlor, Brom, Methyl, Ethyl, Vinyl, Ethinyl, Propinyl, Cyclopropyl, Methoxy, Trifluormethyl, Trifluormethoxy, Cyano oder einen Phenylrest, 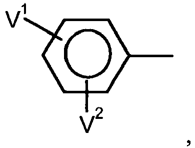 wobei im Falle von Phenyl nur einer der Reste Y oder Z für Phenyl stehen darf,
- V¹: steht ganz besonders bevorzugt für Wasserstoff, Fluor, Chlor, Brom, Methyl, Ethyl, n-Propyl, iso-Propyl, tert.-Butyl, Methoxy, Ethoxy, n-Propoxy, iso-Propoxy, Trifluormethyl oder Trifluormethoxy,
- V²: steht ganz besonders bevorzugt für Wasserstoff, Fluor, Chlor, Methyl, Ethyl, n-Propyl, iso-Propyl, Methoxy, Ethoxy oder Trifluormethyl,
- A: steht ganz besonders bevorzugt für jeweils einfach bis dreifach durch Fluor und/oder Chlor substituiertes Methoxy, Ethoxy, Propoxy, Butoxy oder Isobutoxy oder für jeweils einfach bis dreifach durch Fluor und/oder Chlor substituiertes Cyclopropylmethoxy oder Cyclopropylethoxy,
- D: steht ganz besonders bevorzugt für NH (1) oder Sauerstoff (2),
- Q¹ und Q²: stehen ganz besonders bevorzugt für Wasserstoff,
- m: steht ganz besonders bevorzugt für die Zahl 1
- G: steht ganz besonders bevorzugt für Wasserstoff (a) oder für eine der Gruppen 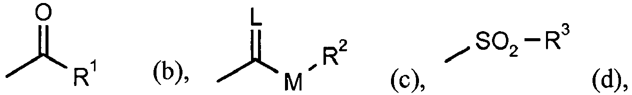 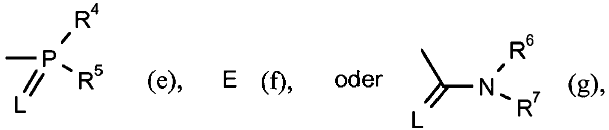 in welchen
E für ein Metallion oder ein Ammoniumion steht,
L für Sauerstoff oder Schwefel steht und
M für Sauerstoff oder Schwefel steht,
R¹ steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl,
R² steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl,
R³ steht ganz besonders bevorzugt für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl,
R⁴ und R⁵ stehen unabhängig voneinander ganz besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio,
R⁶ und R⁷ stehen unabhängig voneinander ganz besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
W steht insbesondere bevorzugt für Wasserstoff, Chlor, Brom, Methyl, Ethyl oder Methoxy, (hervorgehoben für Wasserstoff, Methyl oder Ethyl),
X steht insbesondere bevorzugt für Chlor, Brom, Methyl, Ethyl, Methoxy oder Ethoxy,
Y und Z stehen insbesondere bevorzugt unabhängig voneinander für Wasserstoff, Chlor, Brom, Methyl, Methoxy, Cyclopropy oder für die Reste 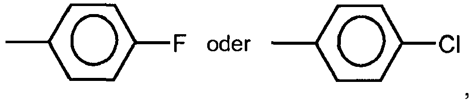 wobei in diesem Falle nur einer der Reste Y oder Z für einen substituierten Phenylrest stehen darf, (hervorgehoben steht Z für Wasserstoff, Methyl, 4-Cl-Phenyl oder 4-F-Phenyl)
A steht insbesondere bevorzugt für jeweils einfach bis dreifach durch Fluor und/oder Chlor substituiertes Methoxy, Ethoxy oder Propoxy, (hervorgehoben für OCH₂CF₃ oder OCH₂CF₂CF₃),
D steht insbesondere bevorzugt für NH (1) oder Sauerstoff (2),
Q¹ und Q² stehen insbesondere bevorzugt für Wasserstoff,
m steht insbesondere bevorzugt für die Zahl 1,
G steht insbesondere bevorzugt für Wasserstoff (a) oder für eine der Gruppen 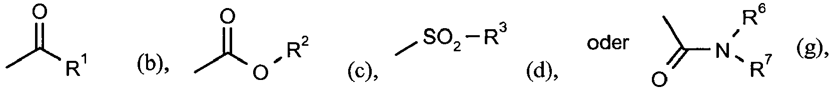 (hervorgehoben für die Gruppen a, b oder c),
R¹ steht insbesondere bevorzugt für C₁-C₁₀-Alkyl, C₁-C₄-Alkoxy-C₁-C₂-alkyl, C₃-C₆-Cycloalkyl,
für gegebenenfalls einfach durch Chlor substituiertes Phenyl, oder für Thienyl, (hervorgehoben für C₁-C₁₀-Alkyl),
R² steht insbesondere bevorzugt für C₁-C₁₀-Alkyl, C₂-C₁₀-Alkenyl, oder für Benzyl, (hervorgehoben für C₁-C₁₀-Alkyl),
R³ steht insbesondere bevorzugt für Methyl,
R⁶ und R⁷ stehen insbesondere bevorzugt zusammen für einen C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfmdungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Hervorgehoben sind Verbindungen der Formel (I) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

**Tabelle 1**

| | | | | |
|---|---|---|---|---|
| 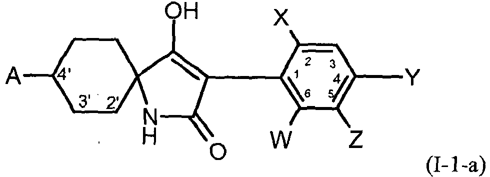 | | | | |

| **A** | **X** | **W** | **Y** | **Z** |
|---|---|---|---|---|
| -O-CH₂-CF₃ | CH₃ | H | H | H |
| -O-CH₂-CF₃ | Br | H | H | H |
| -O-CH₂-CF₃ | Cl | H | H | H |
| -O-CH₂-CF₃ | CF₃ | H | H | H |
| -O-CH₂-CF₃ | OCH₃ | H | H | H |
| -O-CH₂-CF₃ | Br | H | Cl | H |
| -O-CH₂-CF₃ | Cl | H | Br | H |
| -O-CH₂-CF₃ | Cl | H | Cl | H |
| -O-CH₂-CF₃ | Cl | H | CH₃ | H |
| -O-CH₂-CF₃ | CH₃ | H | Cl | H |
| -O-CH₂-CF₃ | Cl | Cl | H | H |
| -O-CH₂-CF₃ | Cl | OCH₃ | H | H |
| -O-CH₂-CF₃ | Cl | CH₃ | H | H |
| -O-CH₂-CF₃ | Cl | OC₂H₅ | H | H |
| -O-CH₂-CF₃ | OCH₃ | OCH₃ | H | H |
| -O-CH₂-CF₃ | CH₃ | CH₃ | H | H |
| -O-CH₂-CF₃ | C₂H₅ | CH₃ | H | H |
| -O-CH₂-CF₃ | C₂H₅ | C₂H₅ | H | H |
| -O-CH₂-CF₃ | Br | C^{H}3 | Br | H |
| -O-CH₂-CF₃ | Cl | CH₃ | Cl | H |
| -O-CH₂-CF₃ | CH₃ | Br | CH₃ | H |
| -O-CH₂-CF₃ | CH₃ | Cl | CH₃ | H |
| -O-CH₂-CF₃ | OCH₃ | CH₃ | CH₃ | H |
| -O-CH₂-CF₃ | OC₂H₅ | CH₃ | CH₃ | H |
| -O-CH₂-CF₃ | 0C₃H₇ | CH₃ | CH₃ | H |
| -O-CH₂-CF₃ | CH₃ | CH₃ | CH₃ | H |
| -O-CH₂-CF₃ | Br | Br | CH₃ | H |
| -O-CH₂-CF₃ | Cl | Cl | CH₃ | H |
| -O-CH₂-CF₃ | CH₃ | CH₃ | Br | H |
| -O-CH₂-CF₃ | OCH₃ | C₂H₅ | CH₃ | H |
| -O-CH₂-CF₃ | OC₂H₅ | C₂H₅ | CH₃ | H |
| -O-CH₂-CF₃ | CH₃ | CH₃ | OCH₃ | H |
| -O-CH₂-CF₃ | Br | Cl | CH₃ | H |
| -O-CH_{z}-CF₃ | Br | CH₃ | Cl | H |
| -O-CH₂-CF₃ | Cl | CH₃ | Br | H |
| -O-CH₂-CF₃ | CH₃ | CH₃ | Cl | H |
| -O-CH₂-CF₃ | C₂H₅ | CH₃ | CH₃ | H |
| -O-CH₂-CF₃ | C₂H₅ | C₂H₅ | CH₃ | H |
| -O-CH₂-CF₃ | C₂^{H}5 | CH₃ | C₂H₅ | H |
| -O-CH₂-CF₃ | C₂H₅ | C₂H₅ | C₂H₅ | H |
| -O-CH₂-CF₃ | C₂H₅ | CH₃ | Cl | H |
| -O-CH₂-CF₃ | C₂H₅ | C₂H₅ | Cl | H |
| -O-CH₂-CF₃ | C₂H₅ | CH₃ | Br | H |
| -O-CH₂-CF₃ | C₂H₅ | C₂H₅ | Br | H |
| -O-CH₂-CF₃ | C₂H₅ | Cl | CH₃ | H |
| -O-CH₂-CF₃ | C₂H₅ | Br | CH₃ | H |
| -O-CH₂-CF₃ | C₂H₅ | Cl | Cl | H |
| -O-CH₂-CF₃ | C₂H₅ | Br | Br | H |
| -O-CH₂-CF₃ | C₂H₅ | Cl | Br | H |
| -O-CH₂-CF₃ | C₂H₅ | Br | Cl | H |
| -O-CH₂-CF₃ | OCH₃ | CH₃ | Cl | H |
| -O-CH₂-CF₃ | OCH₃ | C₂H₅ | Cl | H |
| -O-CH₂-CF₃ | OC₂H₅ | CH₃ | Cl | H |
| -O-CH₂-CF₃ | 0C₂H₅ | C₂H₅ | Cl | H |
| -O-CH₂-CF₃ | Cl | OCH₃ | CH₃ | H |
| -O-CH₂-CF₃ | Cl | OC₂H₅ | CH₃ | H |
| -O-CH₂-CF₃ | CH₃ | CH₃ | Cl | H |
| -O-CH₂-CF₃ | Cl | H | Cl | Cl |
| -O-CH₂-CF₃ | CH₃ | H | CH₃ | CH₃ |
| -O-CH₂-CF₃ | CH₃ | H | Cl | CH₃ |
| -O-CH₂-CF₃ | Br | H | Cl | CH₃ |
| -O-CH_{z}-CF₃ | Br | H | CH₃ | CH₃ |
| -O-CH₂-CF₃ | Cl | H | Br | CH₃ |
| -O-CH₂-CF₃ | Cl | H | Cl | CH₃ |
| -O-CH₂-CF₃ | CH₃ | H | Br | CH₃ |
| -O-CH₂-CF₃ | Cl | H | CH₃ | Cl |
| -O-CH₂-CF₃ | CH₃ | H | H | CH₃ |
| -O-CH₂-CF₃ | Cl | H | H | CH₃ |
| -O-CH₂-CF₃ | Br | H | H | CH₃ |
| -O-CH₂-CF₃ | CH₃ | H | H | Cl |
| -O-CH₂-CF₃ | CH₃ | H | H | Br |
| -O-CH₂-CF₃ | CH₃ | CH₃ | CH₃ | CH₃ |
| -O-CH₂-CF₃ | CH₃ | CH₃ | CH₃ | F |
| -O-CH₂-CF₃ | CH₃ | CH₃ | CH₃ | Cl |
| -O-CH₂-CF₃ | CH₃ | CH₃ | CH₃ | Br |
| -O-CH₂-CF₃ | CH₃ | CH₃ | H | Cl |
| -O-CH₂-CF₃ | CH₃ | CH₃ | H | Br |
| -O-CH₂-CF₃ | Cl | Cl | H | Br |
| -O-CH₂-CF₃ | CH₃ | CH₃ | 4-Cl-C₆H₄ | H |
| -O-CH₂-CF₃ | C₂H₅ | CH₃ | 4-Cl-C₆H₄ | H |
| -O-CH₂-CF₃ | C₂H₅ | C₂H₅ | 4-Cl-C₆H₄ | H |
| -O-CH₂-CF₃ | Cl | CH₃ | 4-Cl-C₆H₄ | H |
| -O-CH₂-CF₃ | Cl | C₂H₅ | 4-Cl-C₆H₄ | H |
| -O-CH₂-CF₃ | CH₃ | H | H | 4-Cl-C₆H₄ |
| -O-CH₂-CF₃ | CH₃ | CH₃ | H | 4-Cl-C₆H₄ |
| -O-CH₂-CF₃ | CH₃ | H | CH₃ | 4-Cl-C₆H₄ |
| -O-CH₂-CF₃ | CH₃ | CH₃ | CH₃ | 4-Cl-C₆H₄ |
| -O-CH₂-CF₃ | Cl | H | H | 4-Cl-C₆H₄ |
| -O-CH₂-CF₃ | J | H | H | H |
| -O-CH₂-CF₃ | J | H | CH₃ | H |
| -O-CH₂-CF₃ | J | CH₃ | H | H |
| -O-CH₂-CF₃ | J | C₂H₅ | H | H |
| -O-CH₂-CF₃ | CH₃ | H | H | J |
| -O-CH₂-CF₃ | CH₃ | H | CH₃ | J |
| -O-CH₂-CF₃ | J | CH₃ | CH₃ | H |
| -O-CH₂-CF₃ | J | C₂H₅ | CH₃ | H |
| -O-CH₂-CF₃ | J | CH₃ | Cl | H |
| -O-CH₂-CF₃ | J | C₂H₅ | Cl | H |
| -O-CH₂-CF₃ | J | Cl | CH₃ | H |
| -O-CH₂-CF₃ | J | H | CH₃ | CH₃ |
| -O-CH₂-CF₃ | CH₃ | H | J | H |
| -O-CH₂-CF₃ | C₂H₅ | H | J | H |
| -O-CH₂-CF₃ | CH₃ | CH₃ | J | H |
| -O-CH₂-CF₃ | C₂H₅ | CH₃ | J | H |
| -O-CH₂-CF₃ | C₂H₅ | C₂H₅ | J | H |
| -O-CH₂-CF₃ | Cl | CH₃ | J | H |
| -O-CH₂-CF₃ | Cl | C₂H₅ | J | H |
| -O-CH₂-CF₃ | CH₃ | H | J | CH₃ |
| -O-CH₂-CF₃ | CH₃ | CH₃ | H | J |
| -O-CH₂-CF₃ | J | H | H | CH₃ |
| -O-CH₂-CF₃ | C₂H₅ | H | H | H |
| -O-CH₂-CF₃ | 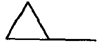 | H | H | H |
| -O-CH₂-CF₃ | 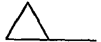 | CH₃ | H | H |
| -O-CH₂-CF₃ | 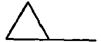 | H | CH₃ | H |
| -O-CH₂-CF₃ | 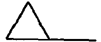 | C₂H₅ | H | H |
| -O-CH₂-CF₃ | 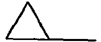 | CH₃ | CH₃ | H |
| -O-CH₂-CF₃ | 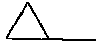 | C₂H₅ | CH₃ | H |
| -O-CH₂-CF₃ | 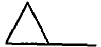 | CH₃ | Cl | H |
| -O-CH₂-CF₃ | 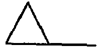 | C₂H₅ | Cl | H |
| -O-CH₂-CF₃ | 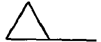 | Cl | CH₃ | H |
| -O-CH₂-CF₃ | CH₃ | H | 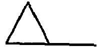 | H |
| -O-CH₂-CF₃ | C₂H₅ | H | 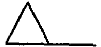 | H |
| -O-CH₂-CF₃ | CH₃ | CH₃ | 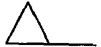 | H |
| -O-CH₂-CF₃ | C₂H₅ | CH₃ | 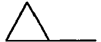 | H |
| -O-CH₂-CF₃ | C₂H₅ | C₂H₅ | 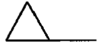 | H |
| -O-CH₂-CF₃ | Cl | CH₃ | 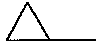 | H |
| -O-CH₂-CF₃ | Cl | C₂H₅ | 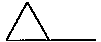 | H |

Im einzelnen seien außer den bei den Herstellungsbeispielen genannten Verbindungen die folgenden Verbindungen der Formel (I-2-a) genannt:

**Tabelle 2**

| | | | | |
|---|---|---|---|---|
| 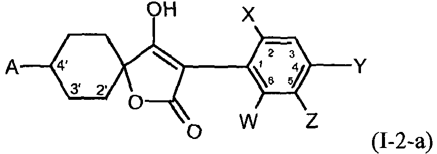 | | | | |

| **A** | **X** | **W** | **Y** | **Z** |
|---|---|---|---|---|
| -O-CH₂-CF₃ | CH₃ | H | H | H |
| -O-CH₂-CF₃ | Br | H | H | H |
| -O-CH₂-CF₃ | Cl | H | H | H |
| -O-CH₂-CF₃ | CF₃ | H | H | H |
| -O-CH₂-CF₃ | OCH₃ | H | H | H |
| -O-CH₂-CF₃ | Br | H | Cl | H |
| -O-CH₂-CF₃ | Cl | H | Br | H |
| -O-CH₂-CF₃ | Cl | H | Cl | H |
| -O-CH₂-CF₃ | Cl | H | CH₃ | H |
| -O-CH₂-CF₃ | CH₃ | H | Cl | H |
| -O-CH₂-CF₃ | Cl | Cl | H | H |
| -O-CH₂-CF₃ | Cl | OCH₃ | H | H |
| -O-CH₂-CF₃ | Cl | CH₃ | H | H |
| -O-CH₂-CF₃ | Cl | OC₂H₅ | H | H |
| -O-CH₂-CF₃ | OCH₃ | OCH₃ | H | H |
| -O-CH₂-CF₃ | CH₃ | CH₃ | H | H |
| -O-CH₂-CF₃ | C₂H₅ | CH₃ | H | H |
| -O-CH₂-CF₃ | C₂H₅ | C₂H₅ | H | H |
| -O-CH₂-CF₃ | Br | CH₃ | Br | H |
| -O-CH₂-CF₃ | Cl | CH₃ | Cl | H |
| -O-CH₂-CF₃ | CH₃ | Br | CH₃ | H |
| -O-CH₂-CF₃ | CH₃ | Cl | CH₃ | H |
| -O-CH₂-CF₃ | OCH₃ | CH₃ | CH₃ | H |
| -O-CH₂-CF₃ | OC₂H₅ | CH₃ | CH₃ | H |
| -O-CH₂-CF₃ | OC₃H₇ | CH₃ | CH₃ | H |
| -O-CH₂-CF₃ | CH₃ | CH₃ | CH₃ | H |
| -O-CH₂-CF₃ | Br | Br | CH₃ | H |
| -O-CH₂-CF₃ | Cl | Cl | CH₃ | H |
| -O-CH₂-CF₃ | CH₃ | CH₃ | Br | H |
| -O-CH₂-CF₃ | OCH₃ | C₂H₅ | CH₃ | H |
| -O-CH₂-CF₃ | OC₂H₅ | C₂H₅ | CH₃ | H |
| -O-CH₂-CF₃ | CH₃ | CH₃ | OCH₃ | H |
| -O-CH₂-CF₃ | Br | Cl | CH₃ | H |
| -O-CH₂-CF₃ | Br | CH₃ | Cl | H |
| -O-CH₂-CF₃ | Cl | CH₃ | Br | H |
| -O-CH₂-CF₃ | CH₃ | CH₃ | Cl | H |
| -O-CH₂-CF₃ | C₂H₅ | CH₃ | CH₃ | H |
| -O-CH₂-CF₃ | C₂H₅ | C₂H₅ | CH₃ | H |
| -O-CH₂-CF₃ | C₂H₅ | CH₃ | C₂H₅ | H |
| -O-CH₂-CF₃ | C₂H₅ | C₂H₅ | C₂H₅ | H |
| -O-CH₂-CF₃ | C₂H₅ | CH₃ | Cl | H |
| -O-CH₂-CF₃ | C₂H₅ | C₂H₅ | Cl | H |
| -O-CH₂-CF₃ | C₂H₅ | CH₃ | Br | H |
| -O-CH₂-CF₃ | C₂H₅ | C₂H₅ | Br | H |
| -O-CH₂-CF₃ | C₂H₅ | Cl | CH₃ | H |
| -O-CH₂-CF₃ | C₂H₅ | Br | CH₃ | H |
| -O-CH₂-CF₃ | C₂H₅ | Cl | Cl | H |
| -O-CH₂-CF₃ | C₂H₅ | Br | Br | H |
| -O-CH₂-CF₃ | C₂H₅ | Cl | Br | H |
| -O-CH₂-CF₃ | C₂H₅ | Br | Cl | H |
| -O-CH₂-CF₃ | OCH₃ | CH₃ | Cl | H |
| -O-CH₂-CF₃ | OCH₃ | C₂H₅ | Cl | H |
| -O-CH₂-CF₃ | OC₂H₅ | CH₃ | Cl | H |
| -O-CH₂-CF₃ | OC₂H₅ | C₂H₅ | Cl | H |
| -O-CH₂-CF₃ | Cl | OCH₃ | CH₃ | H |
| -O-CH₂-CF₃ | Cl | OC₂H₅ | CH₃ | H |
| -O-CH₂-CF₃ | CH₃ | CH₃ | Cl | H |
| -O-CH₂-CF₃ | Cl | H | Cl | Cl |
| -O-CH₂-CF₃ | CH₃ | H | CH₃ | CH₃ |
| -O-CH₂-CF₃ | CH₃ | H | Cl | CH₃ |
| -O-CH₂-CF₃ | Br | H | Cl | CH₃ |
| -O-CH₂-CF₃ | Br | H | CH₃ | CH₃ |
| -O-CH₂-CF₃ | Cl | H | Br | CH₃ |
| -O-CH₂-CF₃ | Cl | H | Cl | CH₃ |
| -O-CH₂-CF₃ | CH₃ | H | Br | CH₃ |
| -O-CH₂-CF₃ | Cl | H | CH₃ | Cl |
| -O-CH₂-CF₃ | CH₃ | H | H | CH₃ |
| -O-CH₂-CF₃ | Cl | H | H | CH₃ |
| -O-CH_{z}-CF₃ | Br | H | H | CH₃ |
| -O-CH₂-CF₃ | CH₃ | H | H | Cl |
| -O-CH₂-CF₃ | CH₃ | H | H | Br |
| -O-CH₂-CF₃ | CH₃ | CH₃ | CH₃ | CH₃ |
| -O-CH₂-CF₃ | CH₃ | CH₃ | CH₃ | F |
| -O-CH₂-CF₃ | CH₃ | CH₃ | CH₃ | Cl |
| -O-CH₂-CF₃ | CH₃ | CH₃ | CH₃ | Br |
| -O-CH₂-CF₃ | CH₃ | CH₃ | H | Cl |
| -O-CH₂-CF₃ | CH₃ | CH₃ | H | Br |
| -O-CH₂-CF₃ | Cl | Cl | H | Br |
| -O-CH₂-CF₃ | CH₃ | CH₃ | 4-Cl-C₆H₄ | H |
| -O-CH₂-CF₃ | C₂H₅ | CH₃ | 4-Cl-C₆H₄ | H |
| -O-CH₂-CF₃ | C₂H₅ | C₂H₅ | 4-Cl-C₆H₄ | H |
| -O-CH₂-CF₃ | Cl | CH₃ | 4-Cl-C₆H₄ | H |
| -O-CH₂-CF₃ | Cl | C₂H₅ | 4-Cl-C₆H₄ | H |
| -O-CH₂-CF₃ | CH₃ | H | H | 4-Cl-C₆H₄ |
| -O-CH₂-CF₃ | CH₃ | CH₃ | H | 4-Cl-C₆H₄ |
| -O-CH₂-CF₃ | CH₃ | H | CH₃ | 4-Cl-C₆H₄ |
| -O-CH₂-CF₃ | CH₃ | CH₃ | CH₃ | 4-Cl-C₆H₄ |
| -O-CH₂-CF₃ | Cl | H | H | 4-Cl-C₆H₄ |
| -O-CH₂-CF₃ | J | H | H | H |
| -O-CH₂-CF₃ | J | H | CH₃ | H |
| -O-CH₂-CF₃ | J | CH₃ | H | H |
| -O-CH₂-CF₃ | J | C₂H₅ | H | H |
| -O-CH₂-CF₃ | CH₃ | H | H | J |
| -O-CH₂-CF₃ | CH₃ | H | CH₃ | J |
| -O-CH₂-CF₃ | J | CH₃ | CH₃ | H |
| -O-CH₂-CF₃ | J | C₂H₅ | CH₃ | H |
| -O-CH₂-CF₃ | J | CH₃ | Cl | H |
| -O-CH₂-CF₃ | J | C₂H₅ | Cl | H |
| -O-CH₂-CF₃ | J | Cl | CH₃ | H |
| -O-CH₂-CF₃ | J | H | CH₃ | CH₃ |
| -O-CH₂-CF₃ | CH₃ | H | J | H |
| -O-CH₂-CF₃ | C₂H₅ | H | J | H |
| -O-CH₂-CF₃ | CH₃ | CH₃ | J | H |
| -O-CH₂-CF₃ | C₂H₅ | CH₃ | J | H |
| -O-CH₂-CF₃ | C₂H₅ | C₂H₅ | J | H |
| -O-CH₂-CF₃ | Cl | CH₃ | J | H |
| -O-CH₂-CF₃ | Cl | C₂H₅ | J | H |
| -O-CH₂-CF₃ | CH₃ | H | J | CH₃ |
| -O-CH₂-CF₃ | CH₃ | CH₃ | H | J |
| -O-CH₂-CF₃ | J | H | H | CH₃ |
| -O-CH₂-CF₃ | 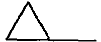 | H | H | H |
| -O-CH₂-CF₃ | 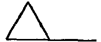 | CH₃ | H | H |
| -O-CH₂-CF₃ | 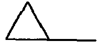 | H | CH₃ | H |
| -O-CH₂-CF₃ | 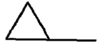 | C₂H₅ | H | H |
| -O-CH₂-CF₃ | 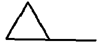 | CH₃ | CH₃ | H |
| -O-CH₂-CF₃ | 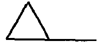 | C₂H₅ | CH₃ | H |
| -O-CH₂-CF₃ | 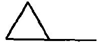 | CH₃ | Cl | H |
| -O-CH₂-CF₃ | 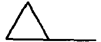 | C₂H₅ | Cl | H |
| -O-CH₂-CF₃ | 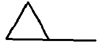 | Cl | CH₃ | H |
| -O-CH₂-CF₃ | CH₃ | H | 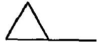 | H |
| -O-CH₂-CF₃ | C₂H₅ | H | 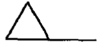 | H |
| -O-CH₂-CF₃ | CH₃ | CH₃ | 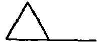 | H |
| -O-CH₂-CF₃ | C₂H₅ | CH₃ | 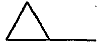 | H |
| -O-CH₂-CF₃ | C₂H₅ | C₂H₅ | 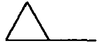 | H |
| -O-CH₂-CF₃ | Cl | CH₃ | 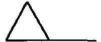 | H |
| -O-CH₂-CF₃ | Cl | C₂H₅ | 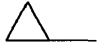 | H |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 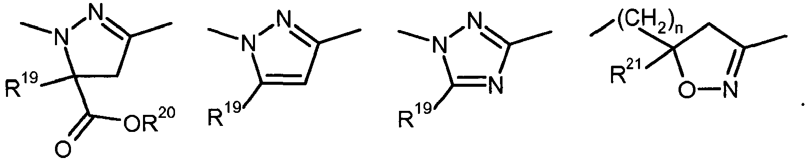
- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxycarbonyl oder Allyloxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormelhoxy.
- t: steht bevorzugt für die Zahlen 0, 1, 2, 3 oder 4.
- v: steht bevorzugt für die Zahlen 0, 1, 2 oder 3.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X^{S}: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle**

| | | | |
|---|---|---|---|
| Beispiele für die Verbindungen der Formel (IIa) | | | |
| 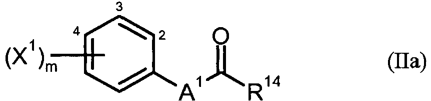 | | | |

| **Beispiel-Nr.** | **(Positionen) (X¹)ₘ** | **A¹** | **R¹⁴** |
|---|---|---|---|
| IIa-1 | (2) Cl, (4) Cl | 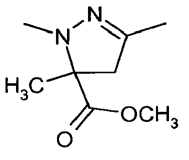 | OCH₃ |
| IIa-2 | (2) Cl, (4) Cl | 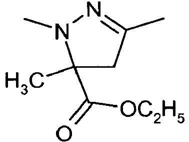 | OCH₃ |
| IIa-3 | (2) Cl, (4) Cl | 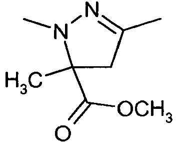 | OC₂H₅ |
| IIa-4 | (2) Cl, (4) Cl | 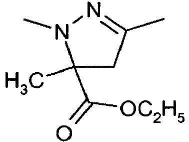 | OC₂H₅ |
| IIa-5 | (2) Cl | 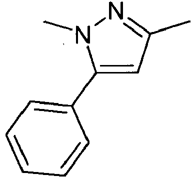 | OCH₃ |
| IIa-6 | (2) Cl, (4) Cl | 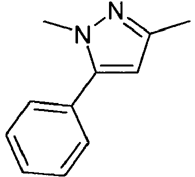 | OCH₃ |
| IIa-7 | (2) F | 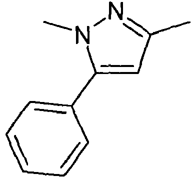 | OCH₃ |
| IIa-8 | (2)F | 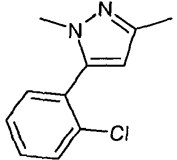 | OCH₃ |
| IIa-9 | (2) Cl, (4) Cl | 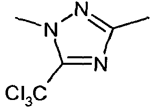 | OC₂H₅ |
| IIa-10 | (2) Cl, (4) CF₃ | 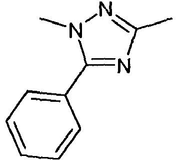 | OCH₃ |
| IIa-11 | (2) Cl | 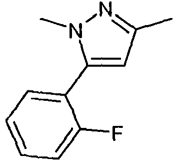 | OCH₃ |
| IIa-12 | - | 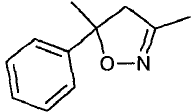 | OC₂H₅ |
| IIa-13 | (2) Cl, (4) Cl | 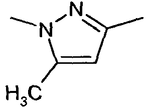 | OC₂H₅ |
| IIa-14 | (2) Cl, (4) Cl | 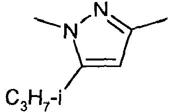 | OC₂H₅ |
| IIa-15 | (2) Cl, (4) Cl | 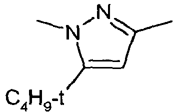 | OC₂H₅ |
| IIa-16 | (2) Cl, (4) Cl | 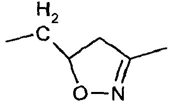 | OC₂H₅ |
| IIa-17 | (2) Cl, (4) Cl | 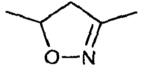 | OC₂H₅ |
| IIa-18 | - | 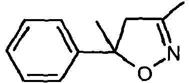 | OH |

Beispiele für die als erfindungsgemäße Herbizid-Safener ganz besonders bevorzugten Verbindungen der Formel (IIb) sind in der nachstehenden Tabelle aufgeführt.

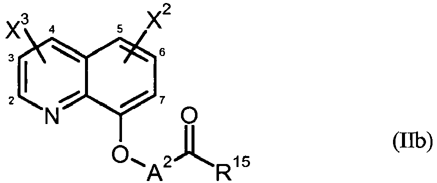

**Tabelle**

| Beispiele für die Verbindungen der Formel (IIb) | | | | |
|---|---|---|---|---|
| **Beispiel-Nr.** | **(Position) X²** | **(Position) X³** | **A²** | **R¹⁵** |
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| IIb-4 | (5) Cl | - | CH₂ | OC₃H₇-n |
| IIb-5 | (5) Cl | - | CH₂ | OC₃H₇₋ᵢ |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉₋ᵢ |
| IIb-12 | (5) Cl | - | CH₂ | 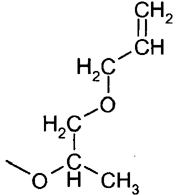 |
| IIb-13 | (5) Cl | - | 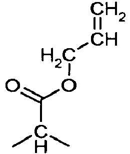 | OCH₂CH=CH₂ |
| IIb-14 | (5) Cl | - | 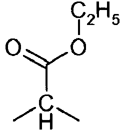 | OC₂H₅ |
| IIb-15 | (5) Cl | - | 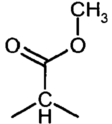 | OCH₃ |

Beispiele für die als erfindungsgemäße Herbizid-Safener ganz besonders bevorzugten Verbindungen der Formel (IIc) sind in der nachstehenden Tabelle aufgeführt.

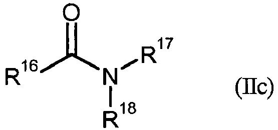

**Tabelle**

| Beispiele für die Verbindungen der Formel (IIc) | | |
|---|---|---|
| **Beispiel-Nr.** | **R¹⁶** | **N(R¹⁷,R¹⁸)** |
| IIc-1 | CHCl₂ | N(CH₂CH=CH₂)₂ |
| IIc-2 | CHCl₂ | 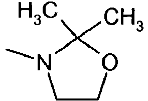 |
| IIc-3 | CHCl₂ | 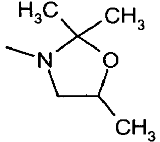 |
| IIc-4 | CHCl₂ | 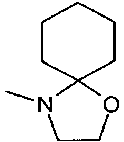 |
| IIc-5 | CHCl₂ | 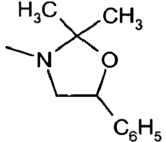 |
| IIc-6 | CHCl₂ | 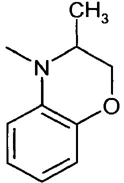 |
| IIc-7 | CHCl₂ | 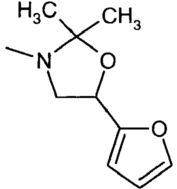 |

Beispiele für die als erfindungsgemäße Herbizid-Safener ganz besonders bevorzugten Verbindungen der Formel (IId) sind in der nachstehenden Tabelle aufgeführt.

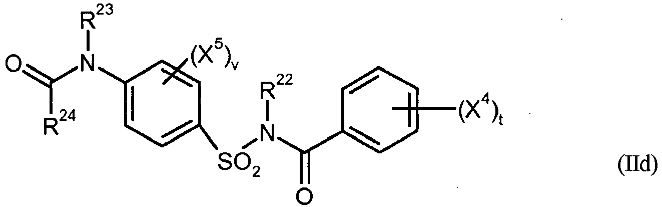

**Tabelle**

| Beispiele für die Verbindungen der Formel (IId) | | | | | |
|---|---|---|---|---|---|
| **Beispiel-Nr.** | **R²²** | **R²³** | **R²⁴** | **(Positionen) (X⁴)ₜ** | **(Positionen) (X⁵)ᵥ** |
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 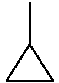 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 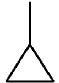 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 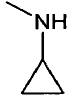 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

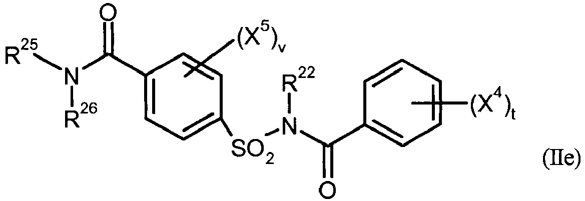

**Tabelle**

| Beispiele für die Verbindungen der Formel (IIe) | | | | | |
|---|---|---|---|---|---|
| **Beispiel-Nr.** | **R²²** | **R²⁵** | **R²⁶** | **(Positionen) (X⁴)ₜ** | **(Positionen) (X⁵)ᵥ** |
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 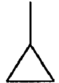 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 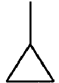 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795lCJS-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle**

| Beispiele für die erfindungsgemäßen Kombinationen | |
|---|---|
| **Wirkstoffe der Formel (I)** | **Safener** |
| I-1-a | Cloquintocet-mexyl |
| I-1-a | Fenchlorazole-ethyl |
| I-1-a | Isoxadifen-ethyl |
| I-1-a | Mefenpyr-diethyl |
| I-1-a | Furilazole |
| I-1-a | Fenclorim |
| I-1-a | Cumyluron |
| I-1-a | Daimuron /Dymron |
| I-1-a | Dimepiperate |
| I-1-a | IIe-11 |
| I-1-a | IIe-5 |
| I-1-b | Cloquintocet-mexyl |
| I-1-b | Fenchlorazole-ethyl |
| I-1-b | Isoxadifen-ethyl |
| I-1-b | Mefenpyr-diethyl |
| I-1-b | Furilazole |
| I-1-b | Fenclorim |
| I-1-b | Cumyluron |
| I-1-b | Daimuron /Dymron |
| I-1-b | Dimepiperate |
| I-1-b | Ile-11 |
| I-1-b | IIe-5 |
| I-1-c | Cloquintocet-mexyl |
| I-1-c | Fenchlorazole-ethyl |
| I-1-c | Isoxadifen-ethyl |
| I-1-c | Mefenpyr-diethyl |
| I-1-c | Furilazole |
| I-1-c | Fenclorim |
| I-1-c | Cumyluron |
| I-1-c | Daimuron /Dymron |
| I-1-c | Dimepiperate |
| I-1-c | IIe-5 |
| I-1-c | IIe-11 |
| I-1-d | Cloquintocet-mexyl |
| I-1-d | Fenchlorazole-ethyl |
| I-1-d | Isoxadifen-ethyl |
| I-1-d | Mefenpyr-diethyl |
| I-1-d | Furilazole |
| I-1-d | Fenclorim |
| I-1-d | Cumyluron |
| I-1-d | Daimuron /Dymron |
| I-1-d | Dimepiperate |
| I-1-d | IIe-11 |
| I-1-d | IIe-5 |
| I-1-e | Cloquintocet-mexyl |
| I-1-e | Fenchlorazole-ethyl |
| I-1-e | Isoxadifen-ethyl |
| I-1-e | Mefenpyr-diethyl |
| I-1-e | Furilazole |
| I-1-e | Fenclorim |
| I-1-e | Cumyluron |
| I-1-e | Daimuron /Dymron |
| I-1-e | Dimepiperate |
| I-1-e | IIe-5 |
| I-1-e | IIe-11 |
| I-1-f | Cloquintocet-mexyl |
| I-1-f | Fenchlorazole-ethyl |
| I-1-f | Isoxadifen-ethyl |
| I-1-f | Mefenpyr-diethyl |
| I-1-f | Furilazole |
| I-1-f | Fenclorim |
| I-1-f | Cumyluron |
| I-1-f | Daimuron /Dymron |
| I-1-f | Dimepiperate |
| I-1-f | IIe-5 |
| I-1-f | IIe-11 |
| I-1-g | Cloquintocet-mexyl |
| I-1-g | Fenehlorazole-ethyl |
| I-1-g | Isoxadifen-ethyl |
| I-1-g | Mefenpyr-diethyl |
| I-1-g | Furilazole |
| I-1-g | Fenclorim |
| I-1-g | Cumyluron |
| I-1-g | Daimuron /Dymron |
| I-1-g | Dimepiperate |
| I-1-g | IIe-5 |
| I-1-g | IIe-11 |
| I-2-a | Cloquintocet-mexyl |
| I-2-a | Fenchlorazole-ethyl |
| I-2-a | Isoxadifen-ethyl |
| I-2-a | Mefenpyr-diethyl |
| I-2-a | Furilazole |
| I-2-a | Fenclorim |
| I-2-a | Cumyluron |
| I-2-a | Daimuron /Dymron |
| I-2-a | Dimepiperate |
| I-2-a | IIe-5 |
| I-2-a | IIe-11 |
| I-2-b | Cloquintocet-mexyl |
| I-2-b | Fenchlorazole-ethyl |
| I-2-b | Isoxadifen-ethyl |
| I-2-b | Mefenpyr-diethyl |
| I-2-b | Furilazole |
| I-2-b | Fenclorim |
| I-2-b | Cumyluron |
| I-2-b | Daimuron /Dymron |
| I-2-b | Dimepiperate |
| I-2-b | IIe-5 |
| I-2-b | IIe-11 |
| I-2-c | Cloquintocet-mexyl |
| I-2-c | Fenchlorazole-ethyl |
| I-2-c | Isoxadifen-ethyl |
| I-2-c | Mefenpyr-diethyl |
| I-2-c | Furilazole |
| I-2-c | Fenclorim |
| I-2-c | Cumyluron |
| 1-2-c | Daimuron /Dymron |
| I-2-c | Dimepiperate |
| I-2-c | IIe-5 |
| I-2-c | IIe-11 |
| I-2-d | Cloquintocet-mexyl |
| I-2-d | Fenchlorazole-ethyl |
| I-2-d | Isoxadifen-ethyl |
| I-2-d | Mefenpyr-diethyl |
| I-2-d | Furilazole |
| I-2-d | Fenclorim |
| I-2-d | Cumyluron |
| I-2-d | Daimuron /Dymron |
| I-2-d | Dimepiperate |
| I-2-d | IIe-5 |
| I-2-d | IIe-11 |
| I-2-e | Cloquintocet-mexyl |
| I-2-e | Fenchlorazole-ethyl |
| I-2-e | Isoxadifen-ethyl |
| I-2-e | Mefenpyr-diethyl |
| I-2-e | Furilazole |
| I-2-e | Fenclorim |
| I-2-e | Cumyluron |
| I-2-e | Daimuron /Dymron |
| I-2-e | Dimepiperate |
| I-2-e | IIe-5 |
| I-2-e | IIe-11 |
| I-2-f | Cloquintocet-mexyl |
| I-2-f | Fenchlorazole-ethyl |
| I-2-f | Isoxadifen-ethyl |
| I-2-f | Mefenpyr-diethyl |
| I-2-f | Furilazole |
| I-2-f | Fenclorim |
| I-2-f | Cumyluron |
| I-2-f | Daimuron /Dymron |
| I-2-f | Dimepiperate |
| I-2-f | IIe-5 |
| I-2-f | IIe-11 |
| I-2-g | Cloquintocet-mexyl |
| I-2-g | Fenchlorazole-ethyl |
| I-2-g | Isoxadifen-ethyl |
| I-2-g | Mefenpyr-diethyl |
| I-2-g | Furilazole |
| I-2-g | Fenclorim |
| I-2-g | Cumyluron |
| I-2-g | Daimuron /Dymron |
| I-2-g | Dimepiperate |
| I-2-g | IIe-5 |
| I-2-g | IIe-11 |

Es wurde nun überraschend gefunden, dass die oben definierten Wirkstoffkombinationen aus Verbindungen der allgemeinen Formel (I) und Safenem (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenem oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von halogenalkoxyspirocyclischen Tetram- und Tetronsäure-Derivaten auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte cyclische Ketoenole beschrieben (US 6 645 914, EP-A2 0 036 106, WO 07/068427). Eine entsprechende Wirkungssteigerung bei Insektiziden ist für bestimmte Cyclische Ketoenole durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Herbiziden aus der Klasse der halogenalkoxyspirocyclischen Tetram- und Tetronsäure-Derivate durch den Zusatz von Ammonium- oder Phosphoniumsalzen zur Anwendungslösung oder durch den Einbau dieser Salze in eine Formulierung enthaltend halogenalkoxyspirocyclische Tetram- und Tetronsäure-Derivate der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und / oder akarizid wirksame halogenalkoxyspirocyclischen Tetram- und Tetronsäure-Derivate der Formel (I) als Wirkstoff enthalten. Gegenstand der Erfindung sind ebenfalls Mittel, die herbizid und/oder akarizid und/oder insektizid wirksame halogenalkoxyspirocyclische Tetram- und Tetronsäure-Derivate der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig. Diese Eigenschaften können jedoch durch den Zusatz von Ammonium- oder Phosphoniumsalzen insgesamt oder partiell verbessert werden.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Wirkstoffe aus der Klasse der halogenalkoxyspirocyclischen Tetram- und Tetronsäure-Derivate der Formel (I) steigern, werden durch Formel (III') definiert 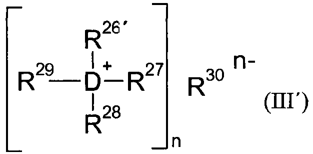 in welcher
- D: für Stickstoff oder Phosphor steht,
- D: bevorzugt für Stickstoff steht,
- E^{26'}, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R^{26'}, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R^{26'}, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R^{26'}, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht.
- R³⁰: ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die herbizid und/oder akarizid und/oder insektizid wirksame, halogenalkoxyspirocyclische Tetram- und Tetronsäure-Derivate der Formel (I) als Wirkstoff enthalten. Gegenstand der Erfindung sind ebenfalls Mittel, die herbizid und/oder akarizid und/oder insektizid wirksame halogenalkoxyspirocyclische Tetram- und Tetronsäure-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,
PO für 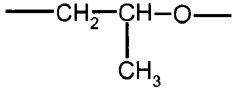 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 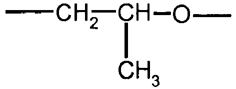 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R': die oben angegebenen Bedeutungen haben,
- EO: für CH₂-CH₂-O- steht,

- BO: für 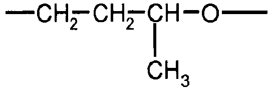 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R': die oben angegebenen Bedeutungen haben,

- BO: für 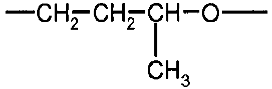 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 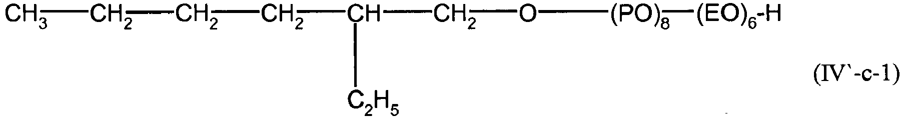 in welcher
- EO: für -CH₂-CH₂-O- steht,

- PO: für 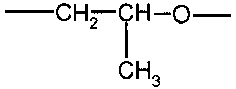 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: für 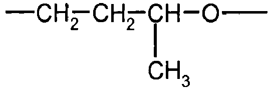 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxidpolypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-[(4-Chlor-2,6-dimethyl)-phenylacetyl]-1-amino-4-trifluorethoxy-cyclohexancarbonsäureethylester als Ausgangsstoff, so kann der Verlauf des erfindungsgemäßen Verfahrens durch folgendes Reaktionsschema wiedergegeben werden:

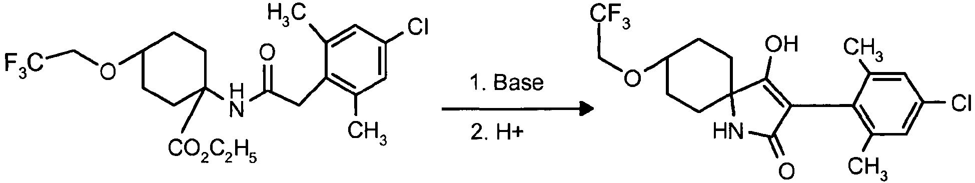

Verwendet man beispielsweise gemäß Verfahren (B) O-[(2-Chlor-6-methyl)-phenylacetyl]-1-hydroxy-4-trifluorethoxy-cyclohexancarbonsäureethylester, so kann der Verlauf des erfindungsgemäßen Verfahrens durch folgendes Reaktionsschema wiedergegeben werden:

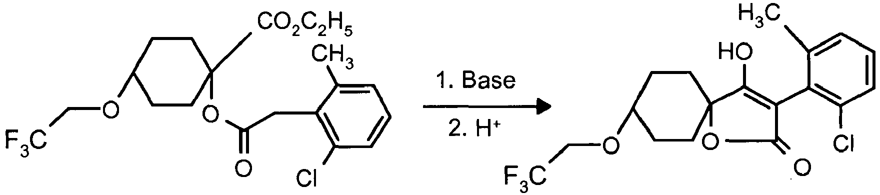

Verwendet man beispielsweise gemäß Verfahren (Cα) 8-Trifluorethoxy-3-[(4-chlor-2,6-dimethyl)-phenyl]-1-azaspiro[4,5]decan-2,4-dion und Pivaloylchlorid als Ausgangsstoffe, so kann der Verlauf des erfindungsgemäßen Verfahrens durch folgendes Reaktionsschema wiedergegeben werden: 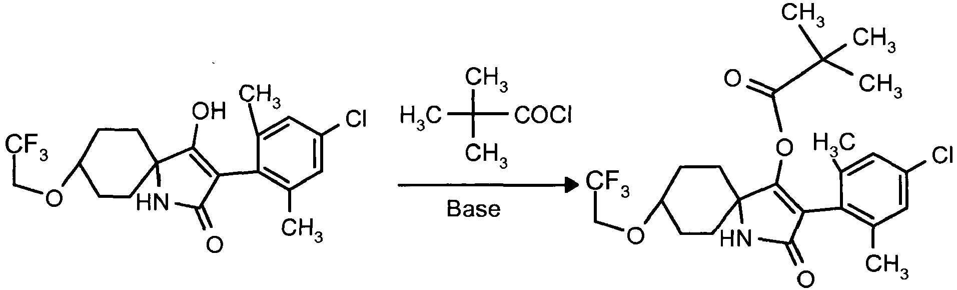 Verwendet man beispielsweise gemäß Verfahren (C) (Variante ß) 8-Trifluorethoxy-3-[(2,4-dichlor)-phenyl]-1-oxaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

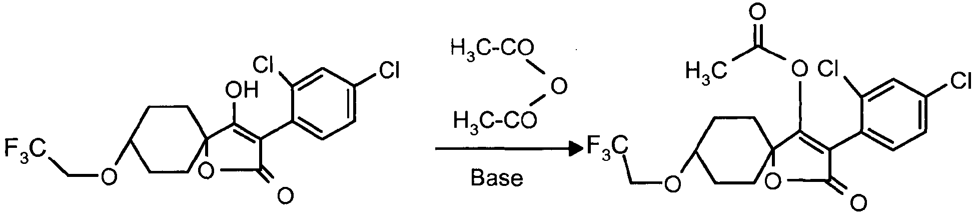

Verwendet man beispielsweise gemäß Verfahren (D) 8-Trifluorethoxy-3-[(2,4-dichlor-6-methyl)-phenyl]-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

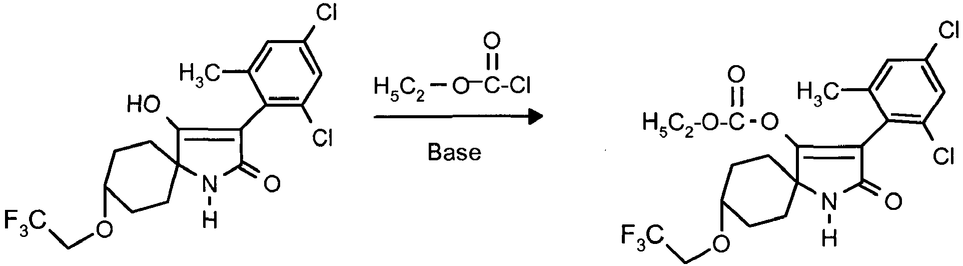

Verwendet man beispielsweise gemäß Verfahren (E) 8-Trifluorethoxy-3-[(2,4,6-trimethyl)-phenyl]-1-oxaspiro[4,5]decan-2,4-dion und Chlormonothioameisensäuremethylester als Ausgangsprodukte, so kann der Reaktionsverlauf folgendermaßen wiedergegeben werden:

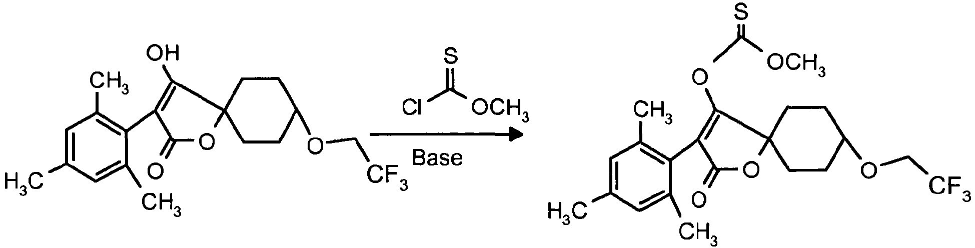

Verwendet man beispielsweise gemäß Verfahren (F) 8-Trifluorethoxy-3-[(2,4,6-trimethyl)-phenyl]-1-azaspiro[4,5]decan-2,4-dion und Methansulfonsäurechlorid als Ausgangsprodukt, so kann der Reaktionsverlauf durch folgendes Reaktionsschema wiedergegeben werden:

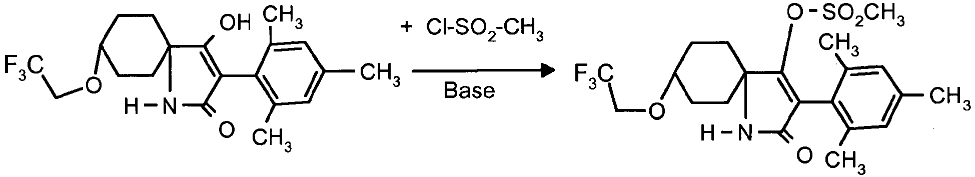

Verwendet man beispielsweise gemäß Verfahren (G) 8-Trifluorethoxy-3-[(2,4-dichlor-6-methyl)-phenyl]-1-oxaspiro[4,5]decan-2,4-dion und Methanthio-phosphonsäurechlorid-(2,2,2-trifluorethyl-ester) als Ausgangsprodukte, so kann der Reaktionsverlauf durch folgendes Reaktionsschema wiedergegeben werden:

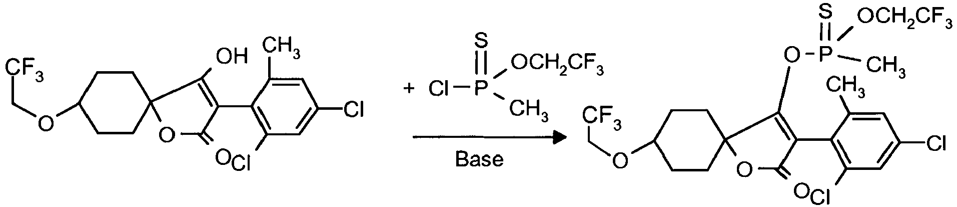

Verwendet man beispielsweise gemäß Verfahren (H) 8-Trifluorethoxy-3-[(2,3,4,6-tetramethylphe-nyl]-1-azapiro[4,5]decan-2,4-dion und NaOH als Komponenten, so kann der Verlauf des erfindungsgemäßen Verfahrens durch folgendes Reaktionsschema wiedergegeben werden:

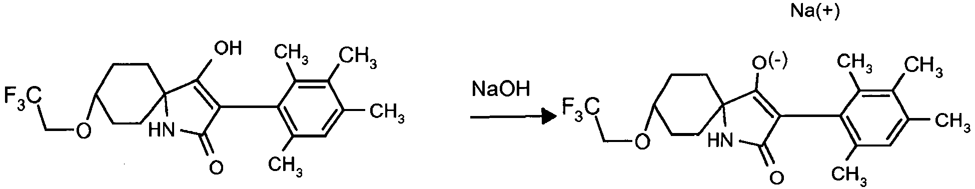

Verwendet man beispielsweise gemäß Verfahren (I) (Variante α) 8-Trifluorethoxy-3-[(2,4,5-trimeth-yl)-phenyl]-1-oxaspiro[4,5]decan-2,4-dion und Ethylisocyanat als Ausgangsprodukte, so kann der Reaktionsverlauf durch folgendes Reaktionsschema wiedergegeben werden:

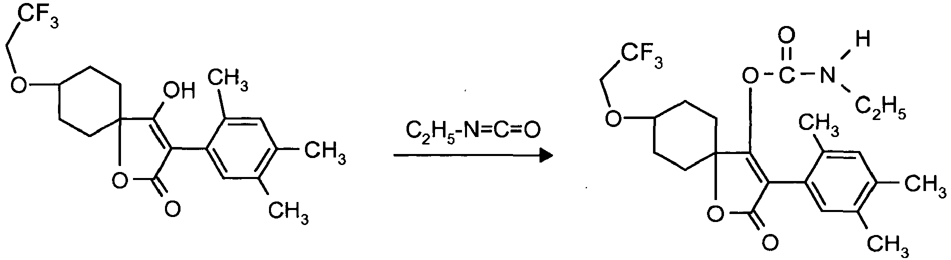

Verwendet man beispielsweise gemäß Verfahren (I) (Variante ß) 8-Trifluorethoxy-3-[(2-4,6-trimethyl)-phenyl]-1-azaspiro[4,5]decan-2,4-dion und Dimethylcarbamidsäurechlorid als Ausgangsprodukte, so kann der Reaktionsverlauf durch folgendes Schema wiedergegeben werden:

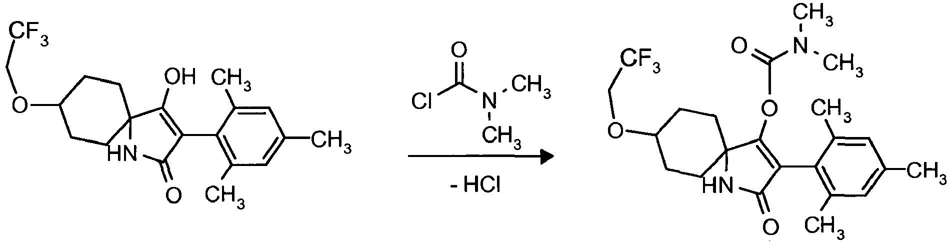

Verwendet man beispielsweise gemäß Verfahren (Jß) 8-Trifluorethoxy-3-[(4-brom-2,6-dimethylphenyl)]-1-azaspiro[4,5]decan-2,4-dion und 4-Chlorphenyl-boronsäure als Ausgangsstoffe, so kann der Reaktionsverlauf durch folgendes Schema wiedergegeben werden:

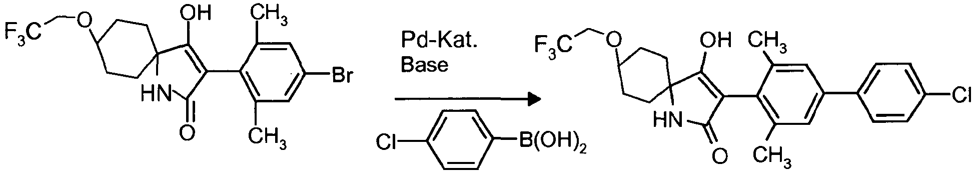

Die beim erfindungsgemäßen Verfahren (A) als Ausgangsstoffe benötigten Verbindungen der Formel (II) 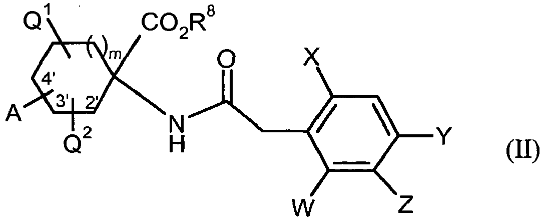 in welcher
- A, Q¹, Q², m, W, X, Y, Z und R⁸: die oben angegebenen Bedeutungen haben,
sind neu.

Man erhält die Acylaminosäureester der Formel (II) beispielsweise, wenn man Aminosäurederivate der Formel (XVI) 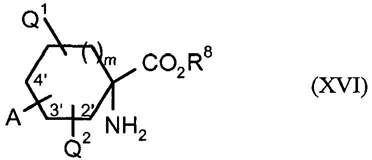 in welcher
- A, m, Q¹ und Q² und R⁸: die oben angegebene Bedeutung haben,
mit substituierten Phenylessigsäurederivaten der Formel (XVII) 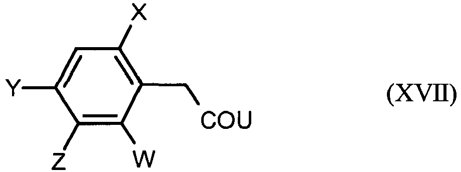 in welcher
- W, X, Y und Z: die oben angegebenen Bedeutungen haben und
- U: für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVIII) 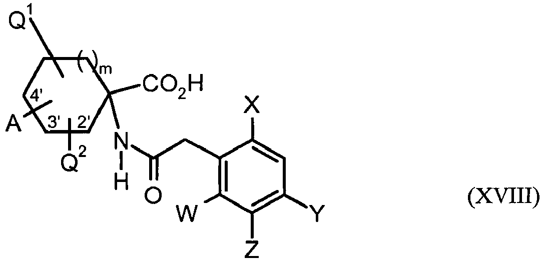 in welcher
- A, Q¹, Q², m, W, X, Y und Z: die oben angegebenen Bedeutungen haben,
verestert (Chem. Ind. (London) 1568 (1968)).

Die Verbindungen der Formel (XVIII) 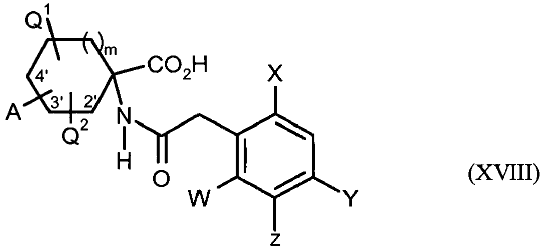 in welcher
- A, Q¹, Q², m, W, X, Y und Z: die oben angegebenen Bedeutungen haben,
sind neu.

Man erhält die Verbindungen der Formel (XVIII) beispielsweise, wenn man 1-Amino-cyclohexan-carbonsäuren der Formel (XIX) 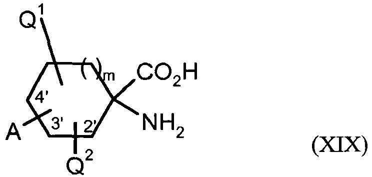 in welcher
- A, m, Q¹ und Q²: die oben angegebenen Bedeutungen haben
mit substituierten Phenylessigsäurederivaten der Formel (XVII) 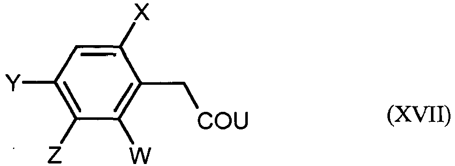 in welcher
- U, W, X, Y und Z: die oben angegebenen Bedeutungen haben und
z.B. nach Schotten-Baumann acyliert (Organikum, VEB Deutscher Verlag der Wissenschaften, Berlin 1977, S. 505).

Die Verbindungen der Formel (XVII) sind teilweise bekannt und/oder lassen sich nach den bekannten Verfahren in den eingangs zitierten Offenlegungsschriften herstellen.

Die Verbindungen der Formel (XVI) und (XIX) sind neu und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975).

Die neuen 1-Amino-cyclohexan-carbonsäuren (XIX) sind im Allgemeinen nach der Bucherer Bergs-Synthese oder nach der Strecker-Synthese erhältlich und fallen dabei jeweils in unterschiedlichen Isomerenformen an. Im Folgenden werden der Einfachheit halber die Isomeren als ß bezeichnet, in welchem der Rest A in der 4-Position und die Aminogruppe äquatorial/axial oder axial/äquatorial stehen. Im Folgenden werden der Einfachheit halber die Isomeren als α bezeichnet, in welchen die Aminogruppe und der Rest A in der 4-Position äquatorial/äquatorial oder axial/axial stehen. 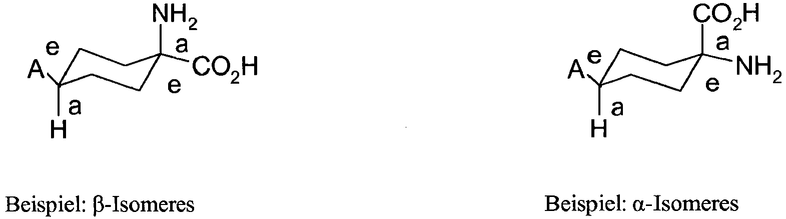 (L. Munday, J. Chem. Soc. 4372 (1961)).

Man erhält Verbindungen der Formel (XIX) beispielsweise durch Umsetzung von Verbindungen der Formel (XXIII) 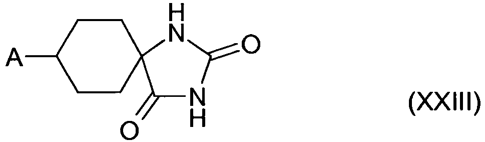 in welcher A die oben genannten Bedeutungen hat. Die Verbindungen der Formel (XXIII) sind teilweise neu und lassen sich nach bekannten Verfahren in den eingangs zitierten Offenlegungsschriften herstellen.

Weiterhin lassen sich die bei dem obigen Verfahren (A) verwendeten Ausgangsstoffe der Formel (II) 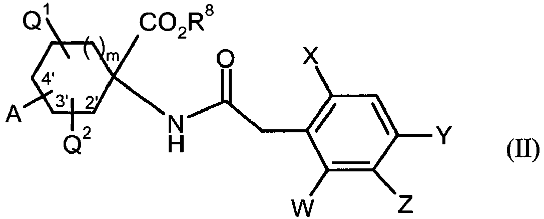 in welcher
- A, Q¹, Q², m, W, X, Y, Z und R⁸: die oben angegebenen Bedeutungen haben,
herstellen, wenn man 1-Amino-cyclohexan-carbonsäurenitrile der Formel (XX) 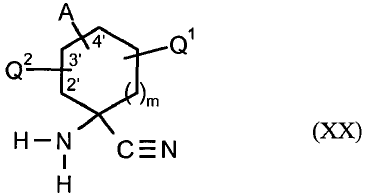 in welcher
- A, m, Q¹ und Q²: die oben angegebenen Bedeutungen haben,
mit substituierten Phenylessigsäurederivaten der Formel (XVII) 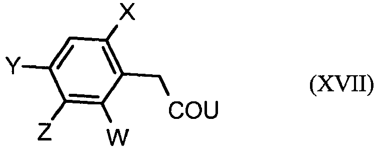 in welcher
- U, W, X, Y und Z: die oben angegebenen Bedeutungen haben,
zu Verbindungen der Formel (XXI) 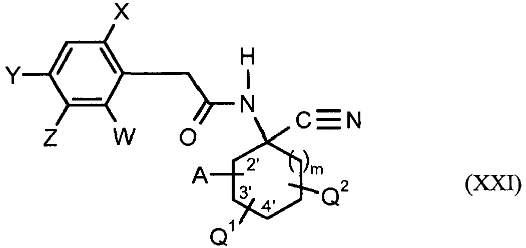 in welcher
- A, m, Q¹, Q², W, X, Y und Z: die oben angegebenen Bedeutungen haben,
umsetzt,
und diese anschließend einer sauren Alkoholyse unterwirft.

Die Verbindungen der Formel (XXI) sind ebenfalls neu. Die Verbindungen der Formel (XX) sind teilweise neu und lassen sich z.B. wie in EP-A-595 130 beschrieben herstellen.

Die bei dem erfindungsgemäßen Verfahren (B) als Ausgangstoffe benötigten Verbindungen der Formel (III) 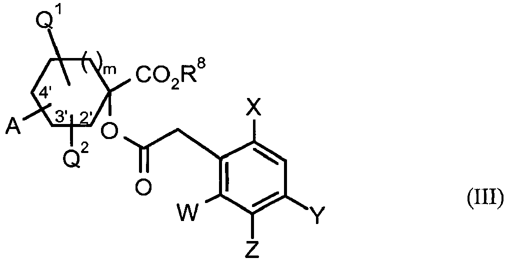 in welcher
- A, m, Q¹, Q², W, X, Y, Z und R⁸ die: oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man
1-Hydroxy-cyclohexan-carbonsäureester der Formel (XXII) 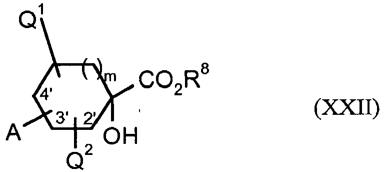 in welcher
- A, m, Q¹, Q² und R⁸: die oben angegebenen Bedeutungen haben,
mit substituierten Phenylessigsäurederivaten der Formel (XVII) 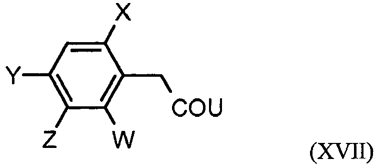 in welcher
- U, W, X, Y und Z: die oben angegebenen Bedeutungen haben,
acyliert (Chem. Reviews 52, 237-416 (1953)).

Die 1-Hydroxy-halogenalkoxy-cyclohexyl-carbonsäureester der Formel (XXII) sind neu. Man erhält sie beispielsweise, indem man substituierte 1-Hydroxy- halogenalkoxy -cyclohexan-carbonsäurenitrile in Gegenwart von Säuren, z.B. nach Pinner mit Alkoholen umsetzt. Das Cyanhydrin erhält man beispielsweise durch Umsetzung von substituierten Halogenalkoxycyclohexan-1-onen mit Blausäure (s. WO 99/16748).

Die zur Durchführung der erfindungsgemäßen Verfahren (C), (D), (E), (F), (G), (H), (I) und (J) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) und Boronsäuren der Formel (XV) sind allgemein bekannte Verbindungen der organischen bzw. anorganischen Chemie.

Die Verbindungen der Formeln (XVII), (I-1-a' - I-2-g') und (I-1-a" - I-2-g") sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, Q¹, Q², m, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass man Verbindungen der Formel (III), in welcher A, Q¹, Q², m, W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuss (bis zu 3 Mol) zu verwenden.

Das Verfahren (C_{α}) ist dadurch gekennzeichnet, dass man Verbindungen der Formeln (I-1-a) bis (I-2-a) jeweils mit Carbonsäurehalogeniden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (C_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (C_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{α}) werden die Ausgangsstoffe der Formeln (I-1-a) bis (I-2-a) und das Carbonsäurehalogenid der Formel (IV) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C_{ß}) ist dadurch gekennzeichnet, dass man Verbindungen der Formeln (I-1-a) bis (I-2-a) jeweils mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{ß}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C_{ß}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (C_{ß}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{ß}) werden die Ausgangsstoffe der Formeln (I-1-a) bis (I-2-a) und das Carbonsäureanhydrid der Formel (V) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formeln (I-1-a) bis (I-2-a) jeweils mit Chlorameisensäureestern oder Chlorameisensäurethiolestern der Formel (VI) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (D) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formeln (I-1-a) bis (I-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (I-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (1-2-a) dar, kann auf den weiteren Zusatz von Säurebindemitteln verzichtet werden.

Als Basen können beim Verfahren (E) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formeln (I-1-a) bis (I-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) bis (1-2-a) ca. 1 Mol Sulfonsäurechlorid der Formel (VIII) bei -20 bis 150°C, vorzugsweise bei 0 bis 70°C um.

Das Verfahren (F) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-a) bis (I-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formeln (I-1-e) bis (I-2-e) auf 1 Mol der Verbindungen (I-1-a) bis (I-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Das Verfahren (G) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (H) ist dadurch gekennzeichnet, dass man Verbindungen der Formeln (I-1-a) bis (I-2-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (I) ist dadurch gekennzeichnet, dass man Verbindungen der Formeln (I-1-a) bis (I-2-a) jeweils mit (Iα) Verbindungen der Formel (XII) gegebenenfalls in Gegenwart eines Verdünnungsmittels und gegebenenfalls in Gegenwart eines Katalysators oder (Iß) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Iα) setzt man pro Mol Ausgangsverbindung der Formeln (I-1-α) bis (I-2-a) ca. 1 Mol Isocyanat der Formel (XII) bei 0 bis 100°C, vorzugsweise bei 20 bis 50°C um.

Das Verfahren (Iα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Iß) setzt man pro Mol Ausgangsverbindung der Formeln (I-1-a) bis (I-2-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Zur Durchführung des erfindungsgemäßen Verfahrens (Jα) und (Jβ) sind Palladium(0)-Komplexe als Katalysator geeignet. Bevorzugt wird beispielsweise Tetrakis-(triphenylphosphin)palladium. Gegebenenfalls können auch Palladium(II)-Verbindungen eingesetzt werden, beispielsweise PdCl₂, Pd(OAc)₂. Bei der Verwendung von Palladium(II)-Verbindungen werden in der Regel Phosphine als Komplexbildner wie beispielsweise Tricyclohexylphosphin eingesetzt.

Als Säureakzeptoren zur Durchführung des erfindungsgemäßen Verfahrens (Jα) und (Jβ) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium-, Cäsium- oder Ammoniumcarbonat, Natriumhydrogen- oder Kaliumhydrogencarbonat, Alkalifluoride, wie beispielsweise Cäsiumfluorid, Alkaliphosphate wie z.B. Kalium-dihydrogenphosphat, Kaliumphosphat sowie tertiäre Amine, wie Trimethylamin, Triethylamin, Tributylamin, N,N-Dimethylanilin, N,N-Dimethylbenzylamin, Pyridin, N-Methylpiperidin, N-Methylmotpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (Jα) und (Jβ) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuren, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylglykolmonomethylether; Wasser.

Die Reaktionstemperatur kann bei dem erfindungsgemäßen Verfahren (Jα) und (Jβ) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (Jα) und (Jβ) werden die Boronsäuren der Formeln (XVα) und (XVβ), in welchen Y und Z die oben angegebene Bedeutung haben und Verbindungen der Formeln (I-1-a') bis (I-2-g'), in welchen A, D, G, Q¹, Q², m, W, X, Y, und Z' bzw. (I-1-a") bis (I-2-g"), in welchen A, D, G, Q¹, Q², m, W, X, Z und Y' die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1-a') bis (I-2-g') bzw. (I-1-a") bis (I-2-g") ein. Die Base setzt man im Allgemeinen in einem Überschuss ein. Die Aufarbeitung geschieht nach üblichen Methoden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp.
Aus der Ordnung der Chilopoda z.B. Geophilus spp., Scutigera spp.
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp.
Aus der Klasse der Helminthen z.B. Ancylostoma duodenale, Ancylostöma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
   Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.
Aus der Ordnung der Hymenoptera z.B. Diprion spp., Hoplocampa spp., Lasius spp., Monomorium pharaonis, Vespa spp.
Aus der Ordnung der Isopoda z.B. Armadillidium vulgare, Oniscus asellus, Porcellio scaber.
Aus der Ordnung der Isoptera z.B. Reticulitermes spp., Odontotermes spp.
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.
Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Die Bezeichnung "Wirkstoffe" schließt immer auch die hier genannten Wirkstoffkombinationen mit ein.

Die Herstellung und die Verwendung der erfindungsgemäßen Wirkstoffe geht aus den nachfolgenden Beispielen hervor.

### Herstellungsbeispiele

### Beispiel I-1-a-1

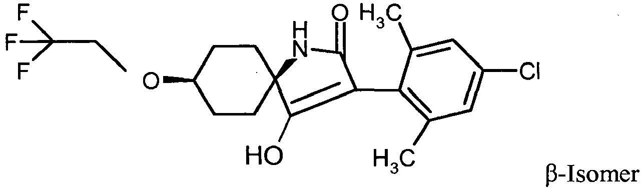

Es werden unter Argon 2.98 g Kalium-tert-butylat-95%ig- in 10 ml Dimethylacetamid vorgelegt. Bei 40°C tropft man 4.4 g der Verbindung gemäß Beispiel II-1 in 10 ml Dimethylacetamid zu. Man rührt 4 h bei 40°C. Nach Reaktionsende (DC-Kontrolle) rührt man in 400 ml Eiswasser ein; stellt mit konz. HCl auf pH2; saugt ab. Es erfolgt Reinigung über Säulenchromatographie an Kieselgel (Methylenchlorid/Essigsäureethylester 3:1)
Ausbeute: 1,4 g (34 % d. Theorie), Fp 262°C

In Analogie zu Beispiel (I-1-a-1) und gemäß den allgemeinen Angaben zur Herstellung erhält man folgende Verbindungen der Formel (I-1-a) mit Q¹ und Q² = H und m = 1

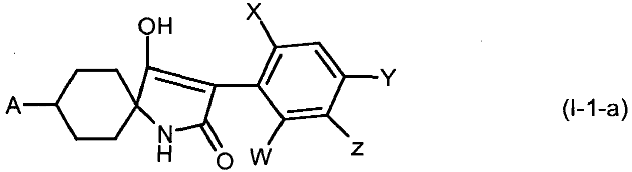

| Bsp.-Nr. | W | X | Y | Z | A | Fp°C | Isomer |
|---|---|---|---|---|---|---|---|
| I-1-a-2 | C₂H₅ | Br | CH₃ | H | -O-CH₂-CF₃ | 128 | α : β |
| | | | | | | | 2 : 7 |
| I-1-a-3 | CH₃ | CH₃ | H | 4-F-Ph | -O-CH₂-CF₃ | 280 | β |
| I-1-a-4 | C₂H₅ | OCH₃ | Cl | H | -O-CH₂-CF₃ | 189 | α : β |
| | | | | | | | 1 : 2 |
| I-1-a-5 | CH₃ | CH₃ | CH₃ | H | -O-CH₂-CF₃ | 203 | α : β |
| | | | | | | | 5 : 9 |
| I-1-a-6 | CH₃ | CH₃ | H | 4-F-Ph | -O-CH₂-CF₃ | 260 | α |
| I-1-a-7 | C₂H₅ | Br | CH₃ | H | -O-CH₂-CF₃ | 111 | α |
| I-1-a-8 | H | CH₃ | Cl | H | -O-CH₂-CF₃ | 210 | α : β |
| | | | | | | | ca. 1:9 |
| I-1-a-9 | CH₃ | CH₃ | Br | H | -O-CH₂-CF₃ | 269 | β |
| I-1-a-10 | H | CH₃ | H | H | -O-CH₂-CF₃ | 217 | α : β |
| | | | | | | | ca. 1:7.5 |
| I-1-a-11 | H | CH₃ | H | CH₃ | -O-CH₂-CF₃ | 212 | β |
| I-1-a-12 | C₂H₅ | C₂H₅ | CH₃ | H | -O-CH₂-CF₃ | 174 | β |
| I-1-a-13 | C₂H₅ | CH₃ | CH₃ | H | -O-CH₂-CF₃ | 176 | β |
| I-1-a-14 | C₂H₅ | O-C₂H₅ | Cl | H | -O-CH₂-CF₃ | 169 | β |
| I-1-a-15 | C₂H₅ | O-C₂H₅ | Cl | H | -O-CH₂-CF₃ | 106 | α |
| I-1-a-16 | CH₃ | CH₃ | CH₃ | CH₃ | -O-CH₂-CF₃ | 254 | β |
| I-1-a-17 | CH₃ | CH₃ | CH₃ | CH₃ | -O-CH₂-CF₃ | 243 | α |
| I-1-a-18 | H | Cl | H | 4-Cl-Ph | -O-CH₂-CF₃ | 247 | α |
| I-1-a-19 | H | Cl | H | 4-Cl-Ph | -O-CH₂-CF₃ | 226 | β |
| I-1-a-20 | H | Cl | H | 4-F-Ph | -O-CH₂-CF₃ | 247 | α |
| I-1-a-21 | H | Cl | H | 4-F-Ph | -O-CH₂-CF₃ | >300 | β |
| I-1-a-22 | CH₃ | OCH₃ | Cl | H | -O-CH₂-CF₃ | 167 | β |
| I-1-a-23 | CH₃ | OCH₃ | Cl | H | -O-CH₂-CF₃ | Wachs | α |
| I-1-a-24 | CH₃ | CH₃ | H | 4-Cl-Ph | -O-CH₂-CF₃ | 273 | α |
| I-1-a-25 | CH₃ | CH₃ | H | 4-Cl-Ph | -O-CH₂-CF₃ | 288 | β |
| I-1-a-26 | H | CH₃ | CH₃ | CH₃ | -O-CH₂-CF₃ | 126 | α |
| I-1-a-27 | H | CH₃ | CH₃ | CH₃ | -O-CH₂-CF₃ | 220 | β |
| I-1-a-28 | CH₃ | CH₃ | Cl | H | -O-CH₂-CF₂-CF₃ | * | α |
| I-1-a-29 | CH₃ | CH₃ | Cl | H | -O-CH₂-CF₂-CF₃ | 202 | β |
| I-1-a-30 | CH₃ | CH₃ | 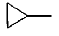 | H | -O-CH₂-CF₃ | 281 | β |
| I-1-a-31 | H | CH₃ | H | 4-F-Ph | -O-CH₂-CF₃ | 140 | α + β |
| | | | | | | | ca. 1 : 2 |
| I-1-a-32 | H | CH₃ | H | CH₃ | -O-CH₂-CF₂-CF₃ | 200 | β |

| | | | | | | | |
|---|---|---|---|---|---|---|---|
| • ¹H-NMR (400 MHz, d₆-DMSO): 8 = 1.21-1.24 (dm, 2H, CH₂), 2.08(s, 6H, Ar-CH₃), 3.75 (zm, 1H, 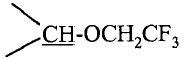 ) , 4.05-4.12 (dt, 2H, OCH₂CF₃), 7.08 (s, 2H, Ar-H) ppm. | | | | | | | |

### Beispiel I-1-b-1

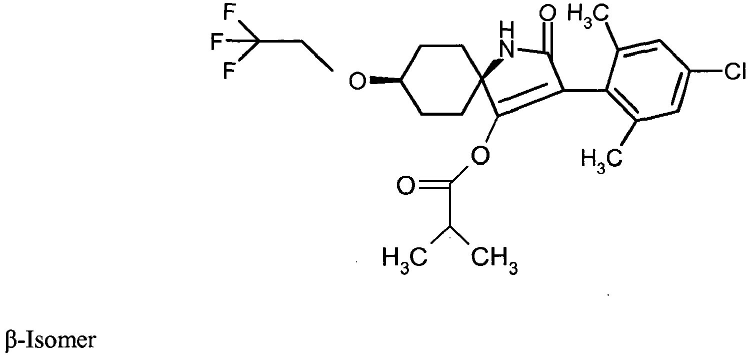

Es werden unter Argon 0,4 g der Verbindung gemäß Beispiel I-1-a-1 (0.001Mol) in 20 ml Essigsäureethylester (wasserfrei) und 0.1 g Triethylamin (0.14 ml) vorgelegt. Mit 10 mg Steglichbase wird katalysiert; bei 70°C werden 0.11 g Isobuttersäurechlorid (0.001 Mol) in 3 ml Essigsäureethylester (wasserfrei) zugetropft. Man rührt 1 h bei 70°C. Nach Einengen erfolgt säulenchromatographische Reinigung an Kieselgel (Dichlormethan: Essigsäureethylester = 10 : 1)
Ausbeute: 0.25 g (52 % d. Theorie), Fp 219°C

In Analogie zu Beispiel (I-1-b-1) und gemäß den allgemeinen Angaben zur Herstellung erhält man folgende Verbindungen der Formel (I-1-b) mit Q¹ und Q² = H und m = 1

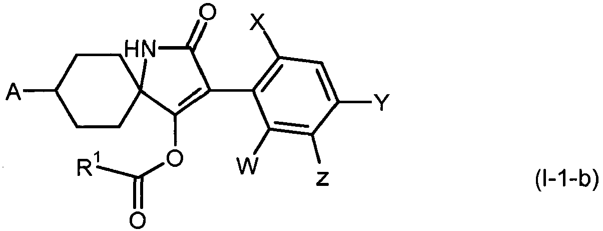

| Bsp.-Nr. | W | X | Y | Z | A | R¹ | Fp.°C | Isomer |
|---|---|---|---|---|---|---|---|---|
| I-1-b-2 | CH₃ | CH₃ | H | 4-F-Ph | O-CH₂-CF₃ | i-C₃H₇ | 210 | β |
| I-1-b-3 | C₂H₅ | OCH₃ | Cl | H | O-CH₂-CF₃ | CH₃ | 204-206 | β |

### Beispiel I-1-c-1

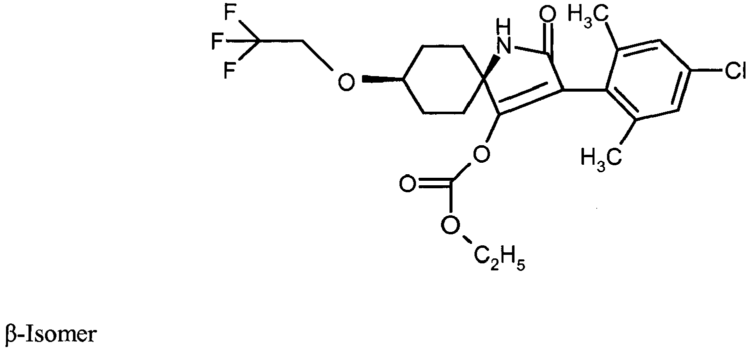

Es werden unter Argon 0,4 g der Verbindung gemäß Beispiel I-1-a-1 (0.001Mol) in 20 ml Methylenchlorid (wasserfrei) und 0.1 g Triethylamin (0.14 ml) vorgelegt. Mit 10 mg Steglichbase wird katalysiert; bei 20°C werden 0,1 ml Chlorameisensäureethylester (0.001 Mol) in 3 ml Methylenchlorid (wasserfrei) zugetropft. Man rührt 1 h bei 20°C. Nach Einengen erfolgt säulenchromatographische Reinigung an Kieselgel (Dichlormethan: Essigsäureethylester = 10 : 1
Ausbeute: 0,3 g (62,6 % d. Theorie), Fp 174°C

In Analogie zu Beispiel (I-1-c-1) und gemäß den allgemeinen Angaben zur Herstellung erhält man folgende Verbindungen der Formel (I-1-c) mit Q¹ und Q² = H und m = 1

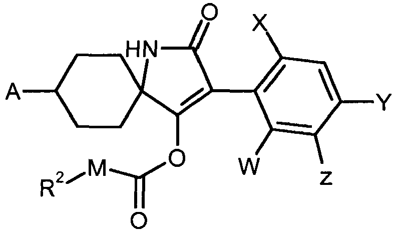

| Bsp.-Nr. | W | X | Y | Z | A | M | R² | Fp.°C | Isomer |
|---|---|---|---|---|---|---|---|---|---|
| I-1-c-2 | CH₃ | CH₃ | H | 4-F-Ph | -O-CH₂-CF₃ | O | CH₃ | 196 | β |
| I-1-c-3 | C₂H₅ | Br | CH₃ | H | -O-CH₂-CF₃ | O | C₂H₅ | 204-206 | β |

### Beispiel II-1

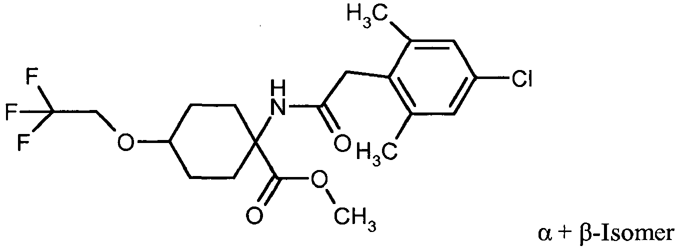

Es werden unter Argon 3,2 g der Verbindung gemäß Beispiel XVI-1 in 30 ml Essigsäureethylester vorgelegt. Man versetzt mit 11 ml 1N NaOH. Bei 0-5°C tropft man parallel 10 ml 1N NaOH und 2.17 g 4-Chlor-2,6-dimethyl-phenylessigsäurechlorid in 10 ml Essigsäureethylester zu. Man rührt 1 h nach. Die wässrige Phase wird abgetrennt und mit Methylenchlorid extrahiert. Die vereinigten organischen Phasen werden mit NaHCO₃-Lösung gewaschen, mit Magnesiumsulfat getrocknet und einrotiert. Die Reinigung erfolgt an Kieselgel mit Magnesiumsulfat als Laufmittel.
Ausbeute: 4.5 g (80 % d. Theorie), Fp 162°C

In Analogie zu Beispiel (II-1) und gemäß den allgemeinen Angaben zur Herstellung erhält man folgende Verbindungen der Formel (II) mit Q¹ und Q² = H und m = 1

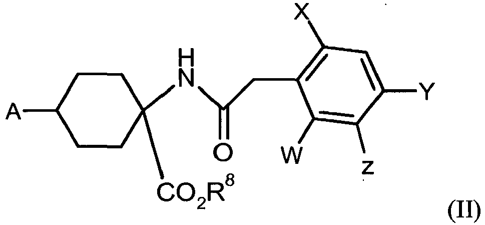

| Bsp.-Nr. | W | X | Y | Z | A | R⁸ | Fp° C | Isomer |
|---|---|---|---|---|---|---|---|---|
| II-2 | CH₃ | CH₃ | CH₃ | H | -O-CH₂-CF₃ | CH₃ | 160 | α + β Gemisch |
| II-3 | CH₃ | CH₃ | H | 4-F-Ph | -O-CH₂-CF₃ | CH₃ | 139 | α + β Gemisch |
| II-4 | C₂H₅ | OCH₃ | Cl | H | -O-CH₂-CF₃ | CH₃ | 136 | α + β Gemisch |
| II-5 | C₂H₅ | Br | 4-CH₃ | H | -O-CH₂-CF₃ | CH₃ | 138 | α + β Gemisch |
| II-6 | H | CH₃ | H | CH₃ | -O-CH₂-CF₃ | CH₃ | 151 | α + β Gemisch |
| II-7 | H | CH₃ | Cl | H | -O-CH₂-CF₃ | CH₃ | 155 | α + β Gemisch |
| II-8 | H | CH₃ | H | H | -O-CH₂-CF₃ | CH₃ | 130 | α + β Gemisch |
| II-9 | CH₃ | CH₃ | Br | H | -O-CH₂-CF₃ | CH₃ | 174 | α + β Gemisch |
| II-10 | CH₃ | CH₃ | CH₃ | H | -O-CH₂-CF₂-CF₃ | CH₃ | 143 | α + β Gemisch |
| II-11 | CH₃ | C₂H₅ | CH₃ | H | -O-CH₂-CF₃ | CH₃ | 130 | α + β Gemisch |
| II-12 | CH₃ | OCH₃ | Cl | H | -O-CH₂-CF₃ | CH₃ | 160 | α + β Gemisch |
| II-13 | C₂H₅ | C₂H₅ | CH₃ | H | -O-CH₂-CF₃ | CH₃ | 143 | α + β Gemisch |
| II-14 | CH₃ | CH₃ | CH₃ | CH₃ | -O-CH₂-CF₃ | CH₃ | 165 | α + β Gemisch |
| II-15 | C₂H₅ | OC₂H₅ | Cl | H | -O-CH₂-CF₃ | CH₃ | 152 | α + β Gemisch |
| II-16 | CH₃ | CH₃ | 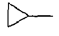 | H | -O-CH₂-CF₃ | CH₃ | 135 | α + β Gemisch |
| II-17 | H | Cl | H | 4-F-Ph | -O-CH₂-CF₃ | CH₃ | 131 | α + β Gemisch |
| II-18 | H | CH₃ | H | 4-Cl-Ph | -O-CH₂-CF₃ | CH₃ | 152 | α + β Gemisch |
| II-19 | CH₃ | CH₃ | H | 4-Cl-Ph | -O-CH₂-CF₃ | CH₃ | 188 | α + β Gemisch |
| II-20 | H | Cl | H | 4-Cl-Ph | -O-CH₂-CF₃ | CH₃ | 160 | α + β Gemisch |
| II-21 | H | CH₃ | CH₃ | CH₃ | -O-CH₂-CF₃ | CH₃ | 153 | α + β Gemisch |
| II-22 | CH₃ | CH₃ | Cl | H | -O-CH₂-CF₂-CF₃ | CH₃ | Öl | α + β Gemisch |
| II-23 | H | CH₃ | H | CH₃ | -O-CH₂-CF₂-CF₃ | CH₃ | Öl | α + β Gemisch |
| II-24 | H | CH₃ | H | 4-F-Ph | -O-CH₂-CF₃ | CH₃ | 116 | α + β Gemisch |
| II-25 | C₂H₅ | Br | CH₃ | H | -O-CH₂-CF₂-CF₃ | CH₃ | 116 | α + β Gemisch |

### Beispiel XVI-1

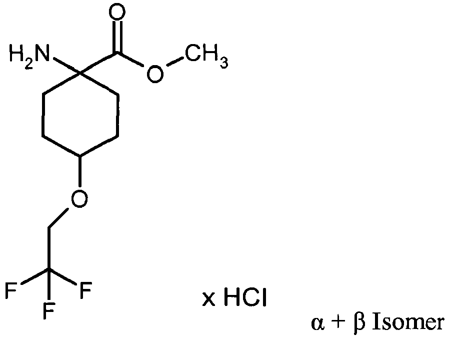

Es werden unter Argon 43 g der Verbindung gemäß Beispiel XIX-1 in 500 ml Methanol vorgelegt und bei 0-5°C 15 ml (0.205 Mol) Thionylchlorid langsam zugetropft. Es wird 30 Min. bei 0°C gerührt, dann 24 h bei 40°C. Die Lösung wird auf 5°C abgekühlt. Der Niederschlag wird abgesaugt und das Lösungsmittel einrotiert. Der Rückstand wird mit Methyl-tert.-butyl-Ether verrieben und der Niederschlag abgesaugt. Es wird aus Methylenchlorid / n-Hexan gefällt.
Ausbeute ausgehend von 4-Trifluorethoxycyclohexanon: 37 g (= 95 % d. Theorie), Fp. 176°C ca. 1:2 α + β Isomer

In Analogie zu Beispiel XVI-1 erhält man folgende Beispiele der Formel (XVI) mit Q¹ und Q² = H und m = 1

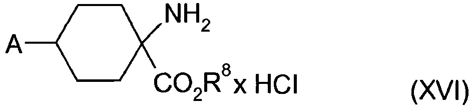

| Bsp.-Nr. | A | R⁸ | ¹H-NMR (ppm) (400 MHz, d₆-DMSO) | Isomer |
|---|---|---|---|---|
| XVI-2 | O-CH₂-CF₂-CF₃ | CH₃ | 3.74, 3.75 (s, 3H, OCH₃) | α + β |
| | | | 4.08, 4.15 (tm, 2H, O-CH₂-CF₂) | |
| XVI-3 | O-CH₂-CF₂-CHF₂ | CH₃ | 3.75, 3.76 (s, 3H, OCH₃) | α + β |
| | | | 3.87-3.95 (tm, 2H, O-CH₂-CF₂) | |
| | | | 6.27-6.73 (qm, 1, CF₂-CHF₂) | |

### Beispiel XIX-1

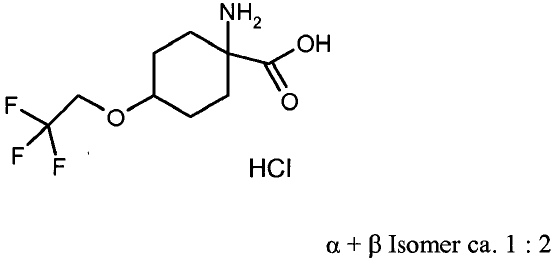

Unter Argon werden 41.5 g der Verbindung gemäß Beispiel XXIII-1 in 250 ml 30%ige KOH suspendiert. Es wird unter Rückfluss (Stickstoff) 24 h gerührt. Es wird auf ca 25 % des Volumens einrotiert; bei 0 - 10 °C mit HCI-konz. auf pH 5 gestellt, das Wasser abrotiert und der Niederschlag getrocknet. Das Rohprodukt wird ohne weitere Reinigung umgesetzt.

In Analogie zu Beispiel XIX-1 erhält man folgende Verbindungen der Formel (XIX) mit Q¹ und Q² = Hundm = 1

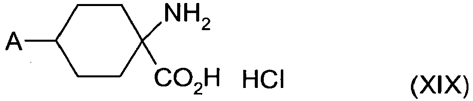

| Bsp.-Nr. | A |
|---|---|
| XIX-2 | O-CH₂-CF₂-CF₃ |
| XIX-3 | O-CH₂-CF₂-CHF₂ |

### Beispiel XXIII-1

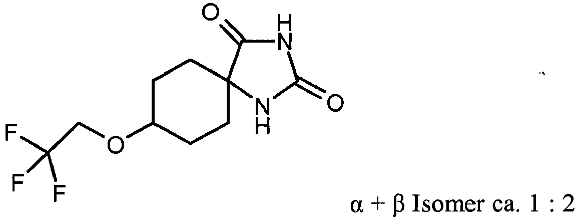

Unter Argon werden 270 ml Wasser, 64 g (0.666 Mol) Ammoniumcarbonat und 7.15 g (0.146 Mol) Natriumcyanid vorgelegt. Bei 20°C tropft man 26 g (0.1325 Mol) 4-Trifluorethoxy-cyclohexanon zu. Die Reaktionsmischung wird 24 h bei 55°C bis 60°C, dann 2 h bei 0°C bis 5°C gerührt. Der Feststoff wird abgesaugt, mit der Mutterlauge nachgewaschen und getrocknet. Das Rohprodukt wird ohne weitere Reinigung umgesetzt.

In Analogie zu Beispiel (XXIII-1) erhält man folgende Verbindungen der Formel (XXIII) mit Q¹ und Q² = Hundm = 1

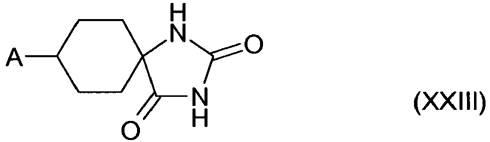

| Bsp.-Nr. | A | ¹H-NMR (ppm) | Isomer |
|---|---|---|---|
| | | (400 MHz, d₆-DMSO) | |
| XXIII-2 | O-CH₂-CF₂-CF₃ | 3.49-3.56; 3.63-3.68 (2m, 1H, 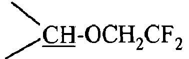 ) | α + β |
| | | 4.03-4.14 (qm, 2H, OCH₂-CF₂) | |
| XXIII-3 | O-CH₂-CF₂-CHF₂ | 3.45-3.51; 3.63 (2m, 1H, 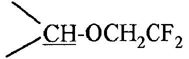 ) | α + β |
| | | | 1 : 2 |
| | | 3.85-3.96 (qm, 2H, O-CH₂-CF₂) | |
| | | 6.24-6.54 (tm, 1H, CF₂-CHF₂) | |
| | | 8.12, 8.17 (2sb, 1H, NH) | |

### Herstellung von 4-Trifluorethoxy-cyclohexanon

### Stufe 1:

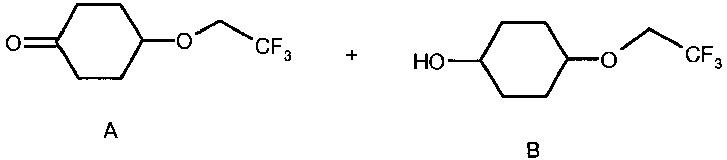

Unter Stickstoff wird 53 g (0.276 mol) 4-Trifluorethoxy-phenol in 420 ml Methylcyclohexan vorgelegt und mit 0.3 g (0.8 mmol) Borax und dem Katalysator (5 % Pd/C , 6 g) versetzt. Das Reaktionsgemisch wird mit 50 Bar Wasserstoff beaufschlagt und auf 160°C erwärmt. Dann wird der Wasserstoffdruck auf 100 Bar erhöht und weiter hydriert bis das Edukt nahezu vollständig umgesetzt ist. Der Autoklav wird nach Entleerung sorgfältig mit Dichlormethan nachgespült, die vereinigten organischen Phasen werden über Celite 545 filtriert und mit 500 ml Dichlormethan nachgewaschen. Das Filtrat wird eingelegt, bei max. 40°C Wasserbad und 78 mbar.

Ausbeute: Man erhält 51 g einer hellrosa Flüssigkeit, enthaltend ca. 31 % 4-Trifluorethoxy-cyclohexanon, ca. 51 % 4-Trifluorethoxy-cyclohexanol und ca. 14 % Methylcylohexan. Das Rohprodukt wird ohne weitere Aufreinigung mit Pyridiniumdichromat oxidiert.

### Stufe 2:

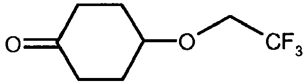

100 g Molekularsieb und 105,25 g ( 0.28 mol) fein gepulvertes Pyridiniumdichromat werden in 450 ml Dichlormethan suspendiert und 51 g des Gemisches aus Stufe 1 in ca. 50 ml Dichlormethan zugetropft. Die Reaktionsmischung wird für 18 h bei Raumtemperatur unter gaschromatographischer Kontrolle gerührt. Nach Reaktionsende werden (550 ml) Diethylether zugesetzt und über Celite filtriert. Es erfolgt ein Nachwaschen mit Diethylether. Das Filtrat wird mit 1N HCl (3 x 250 ml), Wasser (200 ml) und gesättigter NaCl-Lösung (200 ml) gewaschen und getrocknet. Das Lösungsmittel wird vorsichtig entfernt bei 35°C und 60 mbar. Der Rückstand wird im Vakuum destilliert.
Ausbeute: 24 g (Kp₁₇ 102-104°C)

### Beispiel (I-2-a-1)

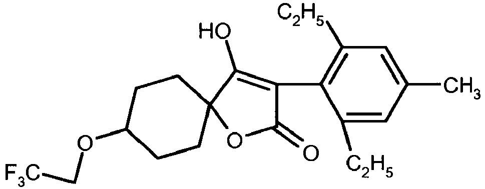

1.081 g (4mmol) 4-Trifluorethoxy-1-hydroxy-cyclohexan-carbonsäureethylester (XXII-1) und 0.899 g (4mmol) 2,6-Diethyl-4-methyl-phenylessigsäurechlorid werden 8 h auf 120°C erhitzt, abgekühlt, zwischen Methyl-tert.-butylether (MTBE) und 5 %-iger Natronlauge verteilt, getrennt, getrocknet und einrotiert. Man erhält 1.6 g Produkt, das in 30 ml N,N-Dimethylformamid vorgelegt, mit 0,59 g Kalium-tert.-butylat versetzt und über Nacht bei Raumtemperatur gerührt wird. Die Reaktionsmischung wird in Wasser eingerührt, mit MTBE extrahiert, die wässrige Phase mit Salzsäure angesäuert, mit Methylenchlorid extrahiert, getrocknet und einrotiert.
Ausbeute: 1.4 g (= 92 % d. Theorie) Isomerengemisch α + β ca. 1 : 1, log P α 3.78; β 3.64

In Analogie zu Beispiel (I-1-a-2) und gemäß den allgemeinen Angaben zur Herstellung erhält man folgende Verbindungen der Formel (I-2-a) mit Q¹ und Q² = H und m = 1:

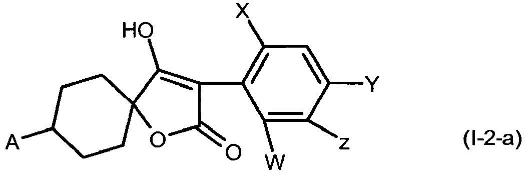

| Bsp.-Nr. | W | X | Y | Z | A | Log P | Isomer |
|---|---|---|---|---|---|---|---|
| I-2-a-2 | H | CH₃ | H | CH₃ | -O-CH₂-CF₃ | α 3.10 | α + β |
| | | | | | | β 2.95 | ca. 1 : 1 |
| I-2-a-3 | CH₃ | CH₃ | CH₃ | H | -O-CH₂-CF₃ | α 3.31 | α + β |
| | | | | | | β 3.18 | ca. 1 : 1 |
| I-2-a-4 | H | CH₃ | CH₃ | CH₃ | -O-CH₂-CF₃ | α 3.34 | α + β |
| | | | | | | β 3.20 | ca. 1 : 1 |
| I-2-a-5 | CH₃ | C₂H₅ | CH₃ | H | -O-CH₂-CF₃ | α 3.54 | α + β |
| | | | | | | β 3.41 | ca. 1 : 1 |
| I-2-a-6 | CH₃ | CH₃ | H | 4-Cl-Ph | -O-CH₂-CF₃ | α 4.29 | α + β |
| | | | | | | β 4.13 | ca. 1 : 1 |
| I-2-a-7 | H | CH₃ | H | 4-Cl-Ph | -O-CH₂-CF₃ | α 4.09 | α + β |
| | | | | | | β 3.93 | ca. 1 : 1 |
| I-2-a-8 | CH₃ | CH₃ | Cl | H | -O-CH₂-CF₃ | α 3.39 | α + β |
| | | | | | | β 3.25 | ca. 1 : 1 |
| I-2-a-9 | CH₃ | CH₃ | CH₃ | H | -O-CH₂-CF₃ | 3.29 | α * |
| I-2-a-10 | CH₃ | CH₃ | CH₃ | H | -O-CH₂-CF₃ | 3.16 | β * |
| I-2-a-1 | CH₃ | OCH₃ | CH₃ | H | -O-CH₂-CF₃ | α 3.07 | α + β |
| | | | | | | β 2.94 | ca. 1 : 1 |
| I-2-a-12 | CH₃ | C₂H₅ | 4-Cl-Ph | H | -O-CH₂-CF₃ | α 4.51 | α + β |
| | | | | | | β 4.36 | ca. 1 : 1 |

| | | | | | | | |
|---|---|---|---|---|---|---|---|
| * isoliert über präparative HPLC | | | | | | | |

### Beispiel (I-2-b-1)

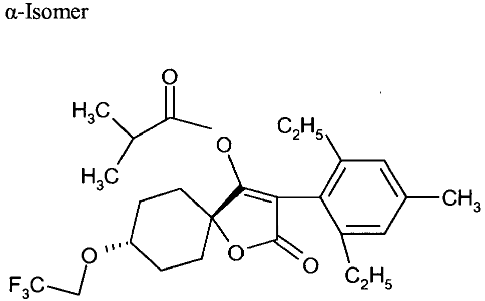

### Beispiel (I-2-b-2)

### β-Isomer

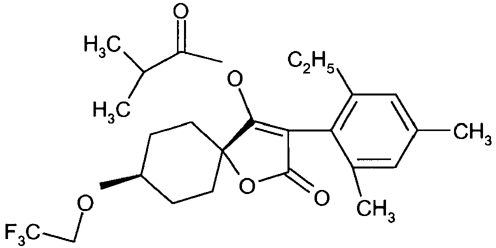

0.2 g (0.485 mmol) der Verbindung gemäß Beispiel (I-2-a-1) und 0.06 g (0.582 mmol) Triethylamin werden in 10 ml Methylenchlorid und bei Raumtemperatur mit 0.062 g (0.582 mmol) Isobuttersäurechlorid in Methylchlorid versetzt und über Nacht bei Raumtemperatur gerührt. Die Reaktionsmischung wird eingeengt und der Rückstand auf einer präparativen HPLC an RP18 Kieselgel mit einem Gradienten Autonitril/Wasser (0.05 % Ameisensäure) 50:50 → 100 : 0 in 20 Min. aufgetrennt.

### Ausbeute:

59 mg = 24 % d. Theorie (I-2-b-1), log P 5.69 und 72 mg = 30 % d. Theorie (I-2-b-2), log P 5.40

In Analogie zu den Beispielen (I-2-b-1) und (I-2-b-2) und gemäß den allgemeinen Angaben zur Herstellung erhält man folgende Verbindungen der Formel (I-2-b) mit Q¹ und Q² = H und m = 1:

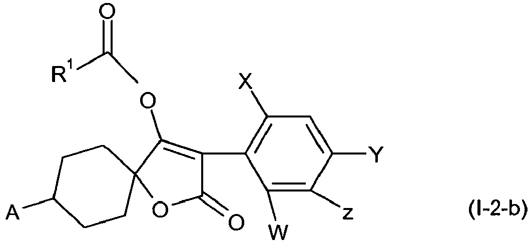

| Bsp.-Nr. | W | X | Y | Z | A | R¹ | logP | Isomer |
|---|---|---|---|---|---|---|---|---|
| I-2-b-3 | CH₃ | C₂H₅ | CH₃ | H | -O-CH₂-CF₃ | i-C₃H₇ | 5.44 | α |
| I-2-b-4 | CH₃ | C₂H₅ | CH₃ | H | -O-CH₂-CF₃ | i-C₃H₇ | 5.15 | β |
| I-2-b-5 | H | CH₃ | H | CH₃ | -O-CH₂-CF₃ | i-C₃H₇ | 4.88 | α |
| I-2-b-6 | H | CH₃ | H | CH₃ | -O-CH₂-CF₃ | i-C₃H₇ | 4.62 | β |
| I-2-b-7 | H | CH₃ | CH₃ | CH₃ | -O-CH₂-CF₃ | i-C₃H₇ | 5.14 | α |
| I-2-b-8 | H | CH₃ | CH₃ | CH₃ | -O-CH₂-CF₃ | i-C₃H₇ | 4.87 | β |
| I-2-b-9 | CH₃ | CH₃ | CH₃ | H | -O-CH₂-CF₃ | i-C₃H₇ | 5.14 | α |
| I-2-b-10 | CH₃ | CH₃ | CH₃ | H | -O-CH₂-CF₃ | i-C₃H₇ | 4.88 | β |
| I-2-b-11 | CH₃ | CH₃ | H | 4-Cl-Ph | -O-CH₂-CF₃ | i-C₃H₇ | 6.11 | α |
| I-2-b-12 | CH₃ | CH₃ | H | 4-Cl-Ph | -O-CH₂-CF₃ | i-C₃H₇ | 5.83 | β |
| I-2-b-13 | H | CH₃ | H | 4-Cl-Ph | -O-CH₂-CF₃ | i-C₃H₇ | 5.84 | α |
| I-2-b-14 | H | CH₃ | H | 4-Cl-Ph | -O-CH₂-CF₃ | i-C₃H₇ | 5.55 | β |
| I-2-b-15 | CH₃ | CH₃ | Cl | H | -O-CH₂-CF₃ | i-C₃H₇ | 5.25 | α |
| I-2-b-16 | CH₃ | CH₃ | Cl | H | -O-CH₂-CF₃ | i-C₃H₇ | 4.97 | β |
| I-2-b-17 | CH₃ | C₂H₅ | 4-Cl-Ph | H | -O-CH₂-CF₃ | i-C₃H₇ | 6.33 | α |
| I-2-b-18 | CH₃ | C₂H₅ | 4-Cl-Ph | H | -O-CH₂-CF₃ | i-C₃H₇ | 6.02 | β |
| I-2-b-19 | CH₃ | OCH₃ | CH₃ | H | -O-CH₂-CF₃ | i-C₃H₇ | 4.72 | α |
| I-2-b-20 | CH₃ | OCH₃ | CH₃ | H | -O-CH₂-CF₃ | i-C₃H₇ | 4.48 | β |

| | | | | | | | | |
|---|---|---|---|---|---|---|---|---|
| Ph = Phenyl | | | | | | | | |

### Bestimmung der LogP-Werte (LC/MS, HCOOH Methode):

Die Bestimmung der in den Tabellen angegebenen logP-Werte erfolgt gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluent A: Acetonitril + 1 ml Ameisensäure/Liter. Eluent B: Wasser + 0,9 ml Ameisensäure/Liter. Gradient: von 10 % Eluent A / 90 % Eluent B bis 95 % Eluent A / 5 % Eluent B in 4,25 min. Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interplation zwischen zwei aufeinander folgenden Alkanonen). Die lambda-max-Werte werden an Hand der UV-Spektren von 200 nm bis 400 nm in den Maxima der chromatographischen Signale ermittelt.

### Vorstufe zu Beispiel (XXII-1)

### 1-Hydroxy-4-trifluoroethoxy-cyclohexancarbonitril

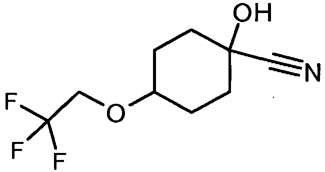

13,74 g Natriumcyanid werden in 200 ml Wasser gelöst. Dann werden in 30 min. bei 20°-28°C unter leichter Kühlung 50 g 4-Trifluoroethoxy-cyclohexanon zugetropft. Es wird 5 Minuten bei 25°C gerührt, dann werden 31,48 g Natriumdisulfit, gelöst in 100 ml Wasser, in 30 min. bei 25°-30°C unter Kühlung zugetropft. Es wird bei Raumtemperatur unter dünnschichtchromatographischer Kontrolle gerührt. Die wässrige Phase wird 3 x mit je 90 ml Toluol extrahiert. Die organischen Phasen werden vereint und im Vakuum eingeengt.
Ausbeute: 32, 52 g

### Beispiel (XXII-1)

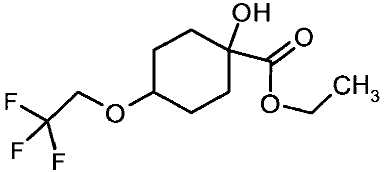

24 g 1-Hydroxy-4-trifluoroethoxy-cyclohexancarbonitril werden in 150 ml Ethanol gelöst. Bei -20°C wird HCl-Gas eingeleitet. Kältebad langsam antauen lassen (Ende bei -5°C), Dauer der HCl-Einleitung ca. 2 h. Es wird über Nacht ohne Kühlung gerührt. Ethanol wird bei 45°C abdestilliert. Der Rückstand wird mit 150 ml Eiswasser versetzt und bei Raumtemperatur 3 Stunden gerührt. Das Reaktionsgemisch wird 3 x mit je 150 ml Methylenchlorid extrahiert. Die vereinten Methylenchlorid-Phasen mit 200 ml gesättigter Natriumhydrogencarbonat-Lösung. gewaschen und eingeengt. Destillation erfolgt im Hochvakuum mit Kolonne (geschätzter Siedepunkt bei 8·10⁻² mbar: ~85°C).

Ausbeute: 25,6 g

### Anwendungsbeispiele

### Beispiel Nr. 1

### Phaedon-Test (Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. I-1-a-1, I-1-b-1, I-2-a-4, I-2-b-6

Bei diesem Test zeigen z. B. die folgenden Verbindungen der Herstellungsbeispiele eine Wirkung von ≥ 80 % bei einer Aufwandmenge von 500 g/ha:
Bsp. Nr. I-1-a-2, I-1-a-3, I-1-a-5, I-1-a-6, I-1-a-7, I-1-a-8, I-1-a-9, I-1-a-12, I-1-a-13, I-1-a-15, I-1-a-16, I-1-a-17, I-1-a-18, I-1-a-19, I-1-a-20, I-1-a-21, I-1-a-22, I-1-a-23, I-1-a-24, I-1-a-25, 1-1-a-26, I-1-a-27, I-1-a-2 8, I-1-a-29, I-1-a-3 0, I-1-b-2, I-1-b-3, I-1-c-1, I-1-c-2, I-1-c-3, 1-2-a-1, I-2-a-2, I-2-a-3, I-2-a-5, I-2-a-6, I-2-a-8, I-2-a-9, I-2-a-10, I-2-a-11, I-2-a-12, I-2-a-7, I-2-a-8, I-2-b-3, 1-2-b-9, I-2-b-10, I-2-b-11, I-2-b-12, I-2-b-13, I-2-b-14, I-2-b-15, I-2-b-5, I-2-b-8, I-2-b-16

### Beispiel Nr. 2

### Myzus-Test (MYZUPE Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-1-a-1, I-1-a-2, I-1-a-3, I-1-a-5, I-1-a-6, I-1-a-7, I-1-a-8, I-1-a-9, I-1-a-10, I-1-a-11, I-1-a-23, I-1-a-24, I-1-a-25, I-1-a-26, I-1-a-27, I-1-a-28, I-1-a-29, I-1-a-30, I-1-b-2, I-1-b-3, I-1-c-1, I-1-c-2, I-1-c-3, I-2-a-1, I-2-a-2, I-2-a-3, I-2-a-4, I-2-a-5, I-2-a-6, I-2-a-8, I-2-a-9, I-2-a-10, I-2-a-11, I-2-a-12, I-2-b-4, I-2-b-5, I-2-b-8, I-2-b-10, I-2-b-11, I-2-b-12, I-2-b-14, I-2-b-15, I-2-b-16

Bei diesem Test zeigen z. B. die folgenden Verbindungen der Herstellungsbeispiele eine Wirkung von ≥ 80 % bei einer Aufwandmenge von 100 g/ha:
Bsp. Nr. I-1-b-1, I-2-b-6, I-1-a-17

### Beispiel Nr. 3

### Tetranychus-Test, OP-resistent (TETRUR Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris*), die von allen Stadien der Gemeinen Spinnmilbe *(Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha: siehe Tabelle
Bsp. Nr. I-1-a-1, I-1-a-2, I-1-a-3, I-1-a-4, I-1-a-5, I-1-a-6, I-1-a-7, I-I-a-8, I-1-a-9, I-1-a-11, I-1-a-12, I-1-a-16, I-1-a-20, I-1-a-22, I-1-a-23, I-1-a-24, I-1-a-25, I-1-a-29, I-1-a-30, I-1-b-2, I-1-b-3, I-1-c-1, I-1-c-2, I-1-c-3, I-2-a-6, I-2-a-7, I-2-a-8, I-2-a-9, I-2-a-10, I-2-a-11, I-2-a-12, I-2-b-3, I-2-b-4, I-2-b-7, I-2-b-8, I-2-b-10, I-2-b-11, I-2-b-13, I-2-b-14, I-2-b-15,

### Beispiel Nr. 4

### Spodoptera frugiperda-Test ( SPODFR Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-1-a-3, I-1-a-6, I-1-a-9, I-1-a-11, I-1-a-19, I-1-a-24, I-1-a-25, I-1-a-27, I-1-a-29, I-1-b-1, I-1-b-2, I-2-a-3, I-2-a-6, I-2-a-10, I-2-b-10, I-2-b-11, I-2-b-12

### Beispiel Nr. 5

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier:
Bsp. Nr. I-1-a-1, I-1-a-2, I-1-a-3, I-1-a-5, I-1-a-8, I-1-a-9, I-1-b-1, I-1-b-2, I-1-c-1, I-1-c-2

### Beispiel Nr. 6

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-a-1, I-1-a-3, I-1-a-5, I-1-a-6, I-1-a-8, I-1-a-9, I-1-b-1, I-1-b-2, I-1-c-1, I-1-c-2

### Beispiel Nr. 7

### Meloidogyne-Test (MELGIN Spritzbehandlung)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm:
Bsp. Nr. I-1-a-3, I-1-a-7

### Beispiel Nr. 8: Wirkungssteigerung durch Ammonium- / Phosphoniumsalze

### Myzus persicae -Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen werden diese in einer Konzentration von 1000 ppm der Spritzbrühe hinzugegeben.

Paprikapflanzen (*Capsicum annuum*)*,* die stark von der Grünen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Wirkstoff ppm | Abtötungsgrad / % nach 6 Tagen | |
|---|---|---|---|
| | | | + AS (1000 ppm) |
| I-1-a-5 | 4 | 5 | 99 |

### Beispiel Nr. 9

### Aphis gossypii -Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium- oder Phosphoniumsalzen werden diese in einerKonzentration von 1000 ppm der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Wirkstoff ppm | Abtötungsgrad / % nach 6 Tagen | |
|---|---|---|---|
| | | | + AS (1000 ppm) |
| I-1-a-1 | 4 | 25 | 95 |

### Beispiel Nr. 10: Wirkungssteigerung durch Ammonium- / Phosphoniumsalze in Kombination mit Penetrationsförderern

### Myzus persicae -Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen und Penetrationsförderer (Rapsölmethylester 500 EW) werden diese jeweils in einerKonzen-tration von 1000 ppm der Spritzbrühe hinzugegeben.

Paprikapflanzen *(Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Konzentrat ion / ppm | Abtötungsgrad / % nach 6 Tagen | | | |
|---|---|---|---|---|---|
| | | | + AS (1000 ppm) | + RME(1000 ppm) | + RME + AS (je 1000 ppm) |
| I-1-a-1 | 4 | 95 | 90 | 99 | 100 |
| I-1-a-1 | 0.8 | 0 | 0 | 0 | 95 |

### Beispiel Nr. 11

### Aphis gossypii -Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium- oder Phosphoniumsalzen und Penetrationsförderern (Rapsölmethylestern 500 EW) werden diese jeweils in einerKonzentration von 1000 ppm der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Konzentration / ppm | Abtötungsgrad / % | | | |
|---|---|---|---|---|---|
| | | nach 6 Tagen | | | |
| | | | + AS (1000 ppm) | + RME (1000 ppm) | + RME + AS (je 1000 ppm) |
| I-1-a-3 | 20 | 25 | 5 | 10 | 90 |
| I-1-a-3 | 4 | 0 | 0 | 5 | 25 |

### Beispiel Nr. 12

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel Nr. 13

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| | |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel Nr. 14

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Lolium multiflorum und Setaria viridis eine Wirkung von ≥ 80 %: I-1-a-1, I-1-a-2, I-1-a-4, I-1-a-5, I-1-a-7, I-1-a-8, I-1-a-9, I-1-a-12, I-1-a-13, I-1-a-14, I-1-b-1, I-I-b-3, I-1-c-1, I-1-c-2, I-1-c-3.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Echinocloa crus-galli, Lolium multiflorum und Setaria viridis eine Wirkung von ≥ 80%: I-1-a-2, I-1-a-4, I-1-a-5, I-1-a-7, I-1-a-9, I-1-a-12, I-1-a-13, I-1-a-14, I-1-b-3.

### Verwendung von Safenern:

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

**Tabelle**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| Bsp. (I-1-a-7) | 100 | 60 | 40 |
| | 50 | 60 | 30 |
| | 25 | 50 | 20 |
| | 12,5 | 40 | 10 |
| Bsp. (I-1-a-7) + Mefenpyr | 100 + 50 | 40 | 20 |
| | 50 + 50 | 40 | 20 |
| | 25 + 50 | 30 | 10 |
| | 12,5 + 50 | 25 | 0 |

Anwendung des Safeners 1 Tag vor dem Herbizid

## Patentansprüche

1. Verbindungen der Formel (I) 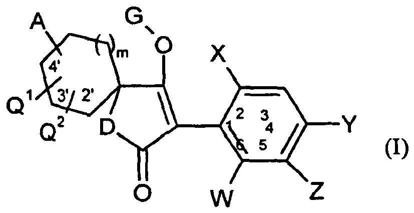 in welcher
W für Wasserstoff, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, Halogen, C₁-C₆-Alkoxy, C₁-C₄-Halogenalkyl, C₁-C₄-Halogenalkoxy oder Cyano steht,
X für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro oder Cyano steht,
Y und Z unabhängig voneinander für Wasserstoff, Halogen, C₁-C₆- Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenalkyl, C₁-C₆-Halogenalkoxyl, Cyano, C₂-C₆-Alkenyl, C₂-C₆-Alkinyl oder für einen der (Het)-arylreste stehen 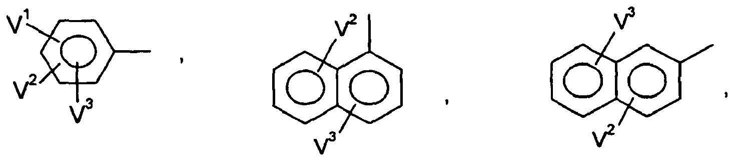 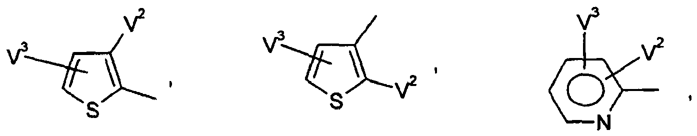 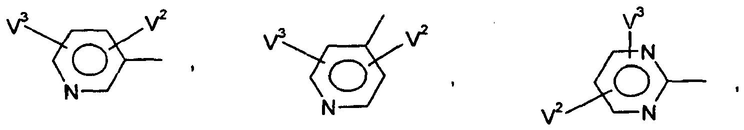 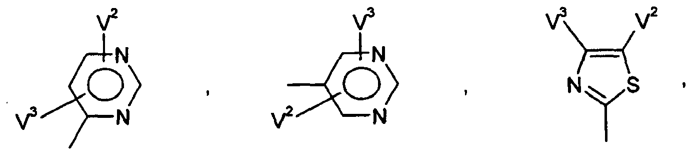 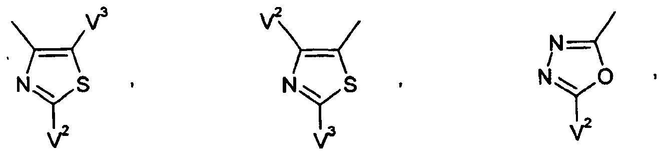 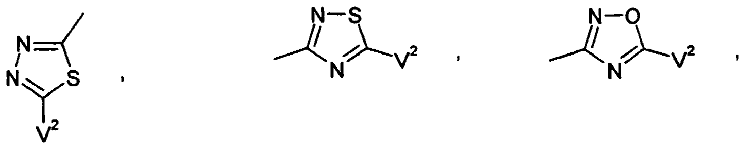 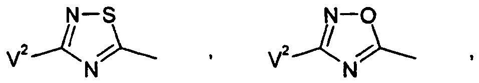 wobei im Falle von (Het)-aryl nur einer der Reste Y oder Z für (Het)-aryl stehen darf,
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
A für einfach bis siebenfach durch Fluor, Chlor, Brom und/oder Jod substituiertes C₁-C₄-Alkoxy oder für einfach bis fünffach durch Fluor, Chlor und/oder Brom substituiertes C₃-C₆-Cycloalkyl-C₁-C₂-alkoxy steht, welches gegebenenfalls durch C₁-C₂-Alkyl, C₁-C₂-Halogenalkyl oder C₁-C₂-Alkoxy substituiert sein kann,
D für NH (1) oder Sauerstoff (2) steht,
Q¹ und Q² unabhängig voneinander für Wasserstoff, C₁-C₆-Alkyl, C₁-C₂-Halogenalkyl oder C₁-C₄-Alkoxy stehen,
m für die Zahl 0 oder 1 steht,
G für Wasserstoff (a) oder für eine der Gruppen 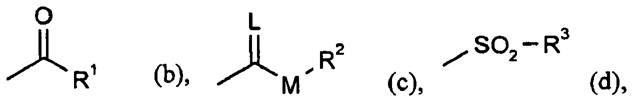 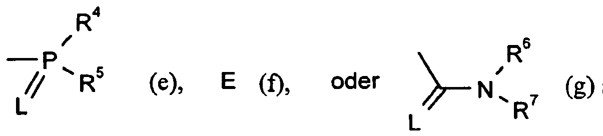 steht, in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht mit ein oder zwei Heteroatomen aus der Reihe Sauerstoff, Schwefel und Stickstoff,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylarnino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-All.yl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Al-kylenrest stehen, in welchem gegebenenfalls eine Methylengruppe durch Sauerstoff oder Schwefel ersetzt ist.

2. Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy steht,
X für Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano steht,
Y und Z unabhängig voneinander für Wasserstoff, Fluor, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste stehen, 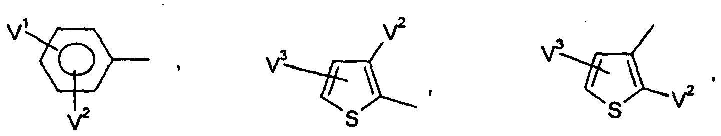 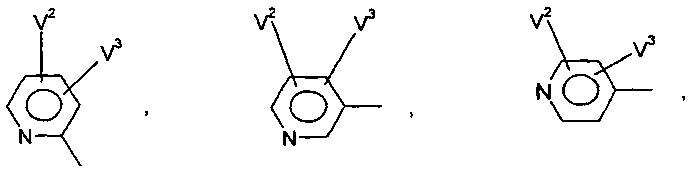 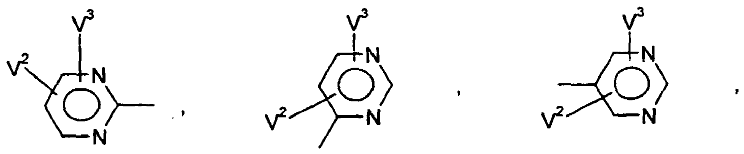 wobei im Falle von (Het)-aryl nur einer der Reste Y oder Z für (Het)-aryl stehen darf,
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl steht,
V² und V³ unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy stehen,
A für einfach bis fünffach durch Fluor, Chlor und/oder Brom substituiertes C₁-C₄-Alkoxy oder für einfach bis dreifach durch Fluor und/oder Chlor substituiertes C₃-C₆-Cycloalkyl-C₁-C₂-alkoxy stehen,
D für NH (1) oder Sauerstoff (2) steht,
Q¹ und Q² unabhängig voneinander für Wasserstoff, Methyl, Ethyl, Trifluormethyl, Methoxy oder Ethoxy stehen,
m für die Zahl 0 oder 1 steht,
G für Wasserstoff (a) oder für eine der Gruppen 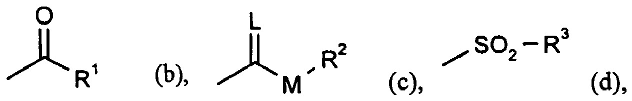 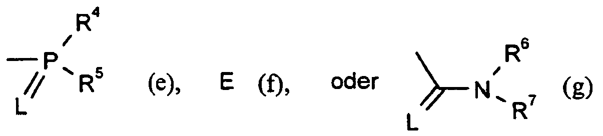 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen,
R⁶ und R⁷ unabhängig voneinander für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Chlor, Brom, Methyl, Ethyl, Vinyl, Ethinyl, Propinyl, Cyclopropyl, Methoxy, Ethoxy oder Trifluormethyl steht,
X für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano steht,
Y und Z unabhängig voneinander für Wasserstoff, Fluor, Chlor, Brom, Methyl, Ethyl, Vinyl, Ethinyl, Propinyl, Cyclopropyl, Methoxy, Trifluormethyl, Trifluormethoxy, Cyano oder einen Phenylrest stehen, 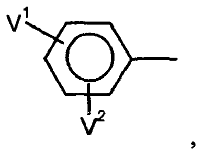 wobei im Falle von Phenyl nur einer der Reste Y oder Z für Phenyl stehen darf,
V¹ für Wasserstoff, Fluor, Chlor, Brom, Methyl, Ethyl, n-Propyl, iso-Propyl, tert.-Butyl, Methoxy, Ethoxy, n-Propoxy, iso-Propoxy, Trifluormethyl oder Trifluormethoxy steht,
V² für Wasserstoff, Fluor, Chlor, Methyl, Ethyl, n-Propyl, iso-Propyl, Methoxy, Ethoxy oder Trifluormethyl steht,
A für jeweils einfach bis dreifach durch Fluor und/oder Chlor substituiertes Methoxy, Ethoxy, Propoxy, Butoxy oder Isobutoxy oder für jeweils einfach bis dreifach durch Fluor und/oder Chlor substituiertes Cyclopropylmethoxy oder Cyclopropylethoxy steht,
D für NH (1) oder Sauerstoff (2) steht,
Q¹ und Q² für Wasserstoff stehen,
m für die Zahl 1 steht,
G für Wasserstoff (a) oder für eine der Gruppen 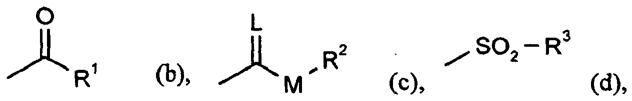 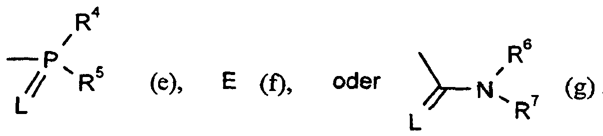 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₀-Aikyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl steht,
R² für jeweils gegebenenfalls einfach bis dreifach durch Fluor oder Chlor substituiertes C₁-C₁₀-Alkyl, C₂-C₁₀-Alkenyl oderC₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht,
R³ für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht,
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls einfach bis zweifach durch Fluor, Chlor, Brom, Methyl, Methoxy oder Trifluormethyl substituiertes Phenyl, oder zusammen für einen C₅-C₆-Alkylenrest stehtn, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Chlor, Brom, Methyl, Ethyl oder Methoxy steht,
X für Chlor, Brom, Methyl, Ethyl, Methoxy oder Ethoxy steht,
Y und Z unabhängig voneinander für Wasserstoff, Chlor, Brom, Methyl, Methoxy, Cyclopropy oder für die Reste 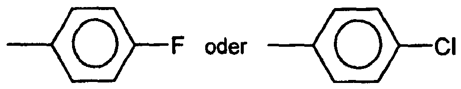 stehen,
wobei in diesem Falle nur einer der Reste Y oder Z für einen substituierten Phenylrest stehen darf,
A für jeweils einfach bis dreifach durch Fluor und/oder Chlor substituiertes Methoxy, Ethoxy oder Propoxy steht,
D für NH (1) oder Sauerstoff (2) steht,
Q¹ und Q² für Wasserstoff stehen,
m für die Zahl 1 steht,
G für Wasserstoff (a) oder für eine der Gruppen 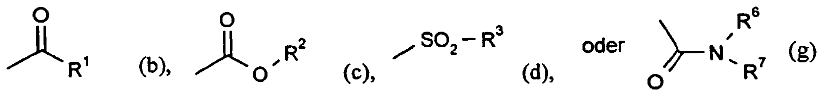 steht,
R¹ für C₁-C₁₀-Alkyl, C₁-C₄-Alkoxy-C₁-C₂-alkyl, C₃-C₆-Cycloalkyl,
für gegebenenfalls einfach durch Chlor substituiertes Phenyl oder für Thienyl steht,
R² für C₁-C₁₀-Alkyl, C₂-C₁₀-Alkenyl, oder für Benzyl steht,
R³ für Methyl steht,
R⁶ und R⁷ zusammen für einen C₅-C₆-Alkylenrest stehen, in welchem gegebenenfalls eine Methylengruppe durch Sauerstoff oder Schwefel ersetzt ist.

5. Verfahren zur Herstellung von Verbindungen der Formel (I) gemäß Anspruch 1, **dadurch gekennzeichnet, dass** man zum Erhalt von
(A) Verbindungen der Formel (I-1-a) 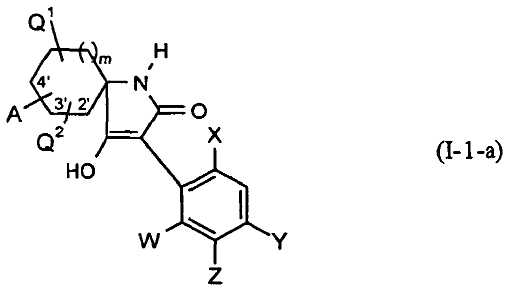 in welcher
A, Q¹, Q², m, W, X, Y und Z die oben angegebenen Bedeutungen haben,
Verbindungen der Formel (II) 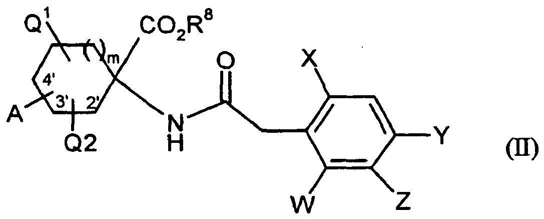 in welcher
A, Q¹, Q², m, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 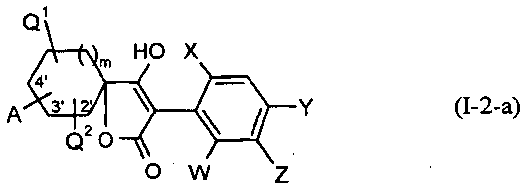 in welcher
A, Q¹, Q², m, W, X, Y und Z die oben angegebenen Bedeutungen haben,
Verbindungen der Formel (III) 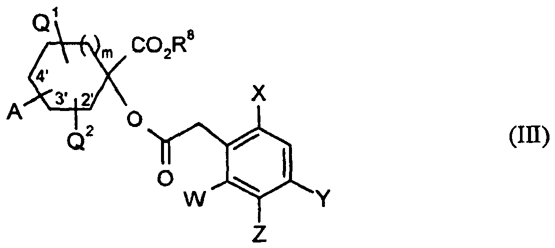 in welcher A, Q¹, Q², m, W, X, Y, Z und R⁸ die oben angegebenen Bedeutungen haben,
in Gegenwart eines Verdünnungsmittels und in Gegenwart einer Base intramolekular kondensiert,
(C) Verbindungen der oben gezeigten Formeln (I-1-b) bis (I-2-b), in welchen R¹, A, Q¹, Q², m, W, X, Y und Z die oben angegebenen Bedeutungen haben, Verbindungen der oben gezeigten Formeln (I-1-a) bis (I-2-a), in welchen A, Q¹, Q², m, W, X, Y und Z die oben angegebenen Bedeutungen haben, jeweils
α) mit Verbindungen der Formel (IV) 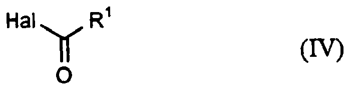 in welcher
R¹ die oben angegebene Bedeutung hat und
Hal für Halogen steht
oder
β) mit Carbonsäureanhydriden der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der oben gezeigten Formeln (I-1-c) bis (I-2-c), in welchen R², A, Q¹, Q², m, W, M, X, Y und Z die oben angegebenen Bedeutungen haben und L für Sauerstoff steht, Verbindungen der oben gezeigten Formeln (I-1-a) bis (I-2-a), in welchen A, Q¹, Q², m, W, X, Y und Z die oben angegebenen Bedeutungen haben, jeweils
mit Chlorameisensäureestern oder Chlorameisensäurethioestern der Formel (VI)
R²-M-CO-Cl (VI)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formeln (I-1-c) bis (I-2-c), in welchen R², A, Q¹ Q², m, W, M, X, Y und Z die oben angegebenen Bedeutungen haben und L für Schwefel steht, Verbindungen der oben gezeigten Formeln (I-1-a) bis (I-2-a), in welchen A, Q¹, Q², m, W, X, Y und Z die oben angegebenen Bedeutungen haben, jeweils
mit Chlormonothioameisensäureestern oder Chlordithioameisensäureestern der Formel (VII) 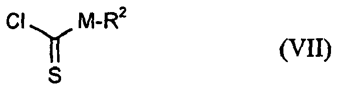 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(F) Verbindungen der oben gezeigten Formeln (I-1-d) bis (I-2-d), in welchen R³, A, W, Q¹, Q², m, X, Y und Z die oben angegebenen Bedeutungen haben, Verbindungen der oben gezeigten Formeln (I-1-a) bis (I-2-a), in welchen A, B, Q¹, Q², m, W, X, Y und Z die oben angegebenen Bedeutungen haben, jeweils
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formeln (I-1-e) bis (I-2-e), in welchen L, R⁴, R⁵, A, Q¹, Q², m, W, X, Y und Z die oben angegebenen Bedeutungen haben, Verbindungen der oben gezeigten Formeln (I-1-a) bis (I-2-a), in welchen A, Q¹, Q², m, W, X, Y und Z die oben angegebenen Bedeutungen haben, jeweils
mit Phosphorverbindungen der Formel (IX) 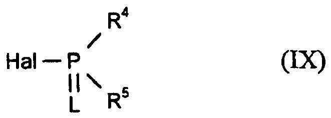 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formeln (I-1-f) bis (I-2-f), in welchen E, A, Q¹, Q², m, W, X, Y und Z die oben angegebenen Bedeutungen haben, Verbindungen der Formeln (I-1-a) bis (I-2-a), in welchen A, Q¹, Q², m, W, X, Y und Z die oben angegebenen Bedeutungen haben, jeweils
mit Metallverbindungen oder Aminen der Formeln (X) oder (XI)
Me(OR¹⁰)ₜ (X) 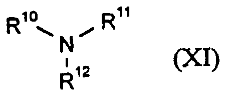
in welchen
Me für ein ein- oder zweiwertiges Metall,
t für die Zahl 1 oder 2 und
R¹⁰, R¹¹, R¹² unabhängig voneinander für Wasserstoff oder Alkyl stehen, gegebenenfalls in Gegenwart eines Verdünnungsmittels umsetzt,
(I) Verbindungen der oben gezeigten Formeln (I-1-g) bis (I-2-g), in welchen L, R⁶, R⁷, A, Q¹, Q², m, W, X, Y und Z die oben angegebenen Bedeutungen haben, Verbindungen der oben gezeigten Formeln (I-1-a) bis (I-2-a), in welchen A, Q¹, Q², m, W, X, Y und Z die oben angegebenen Bedeutungen haben, jeweils
α) mit Isocyanaten oder Isothiocyanaten der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XIII) 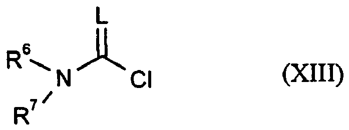 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt,
(Jα) Verbindungen der oben gezeigten Formeln (I-1a) bis (I-1-g), in welchen A, D, G, Q¹, Q², m, W, X, Y und Z die oben angegebene Bedeutung haben, Verbindungen der Formeln (I-1-a') bis (I-2-g'), in welchen A, D, G, Q¹, Q², m, W, X und Y die oben genannte Bedeutung haben und Z' für Brom oder Iod steht 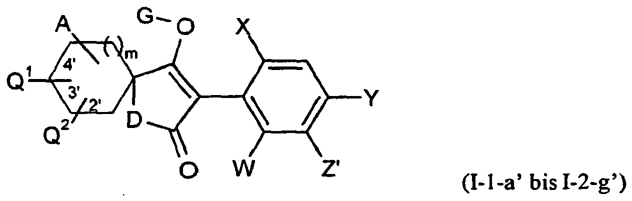 und
(Jβ) Verbindungen der oben gezeigten Formeln (I-1-a) bis (I-2-g), in welchen A, D, G, Q¹, Q², m, W, X, Y und Z die oben angegebene Bedeutung haben, Verbindungen der Formeln (I-1-a") bis (I-2-g"), in welchen A, D, G, Q¹, Q², m, W, X und Z die oben genannte Bedeutung haben und Y' für Brom oder Iod steht 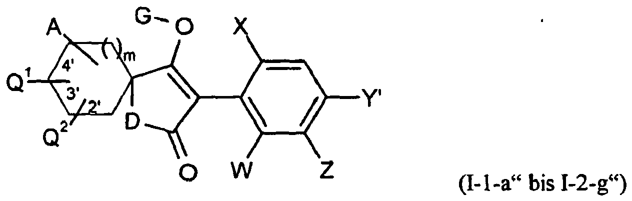 mit kupplungsfähigen (Het)-arylderivaten, der Formeln (XVα) und (XVβ) 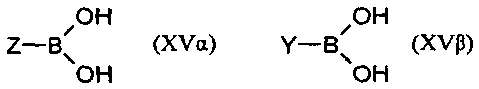 oder deren Ester in Gegenwart eines Lösungsmittels, in Gegenwart eines Katalysators und in Gegenwart einer Base kuppelt.

6. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

7. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt, ausgenommen Verfahren zur chirurgischen oder therapeutischen Behandlung des menschlichen oder tierischen Körpers.

8. Verbindungen der Formel (I) gemäß Anspruch 1 zur Bekämpfung von tierischen Schädlingen und/oder unerwünschtem Pflanzenbewuchs.

9. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

10. Verwendung von Verbindungen der Formel (I) gemäß Anspruch I zur Herstellung von Mitteln zur Bekämpfung von Schädlingen und/oder unerwünschtem Pflanzenwuchs.

11. Mittel enthaltend einen wirksamen Gehalt an einer Wirkstoffkombination umfassend als Komponenten
(a') mindestens eine Verbindung der Formel (I), in welcher A, D, G, Q¹, Q², m, W, X, Y und Z die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexa-hydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Ver-bindungen in EP-A-86750, EP-A-94349, EP-A-191736, EP-A-492366), 3-(2-Chlorbenzyl)-1-(1-methyl-1-phenyl-ethyl)-harnstoff (Cumyluron), α-(Cyanomethoximino)-phenylacetonitril (Cyometrinil), 2,4-Dichlor-phenoxyessig säure (2,4-D), 4-(2,4-Dichlor-phenoxy)-buttersäure (2,4-DB), 1-(1-Methyl-1-phenyl-ethyl)-3-(4-methyl-phenyl)-harn-stoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxyra-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazol-carboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyl-oxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(l,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxy-essigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoyl-sulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen der allgemeinen Formel (IIa) 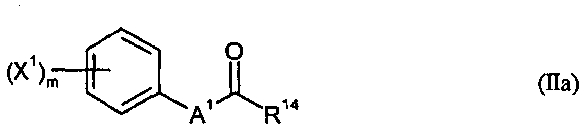 oder der allgemeinen Formel (IIb) 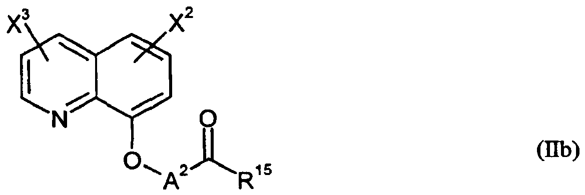 oder der Formel (IIc) 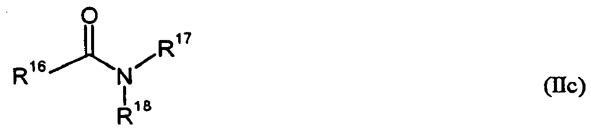 wobei
m für eine Zahl 0, 1, 2, 3, 4 oder 5 steht,
A¹ für eine der nachstehend skizzierten divalenten heterocyclischen Gruppierungen steht, 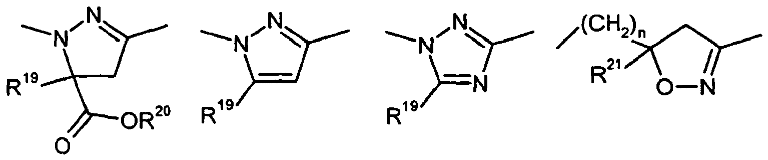
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkylthio, C₁-C₆-Alkenyloxy, C₁-C₆-Alkenyloxy-C₁-C₆-alkoxy, C₁-C₆-Alkylamino oder Di-(C₁-C₄-alkyl)-amino steht,
R¹⁶ für gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Di-oxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Di-oxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 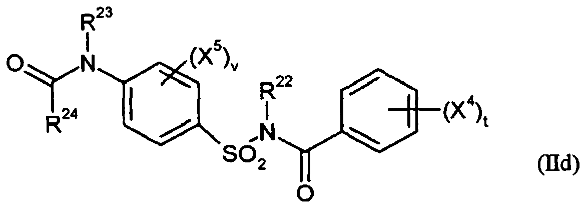 oder der allgemeinen Formel (IIe) 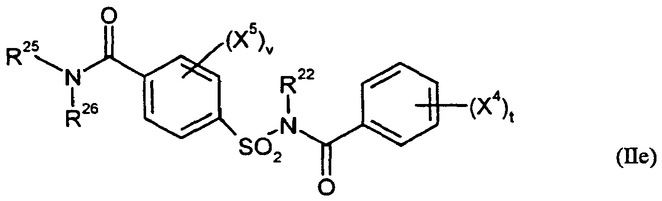 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkyl-thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxa-alkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

12. Mittel nach Anspruch 11, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 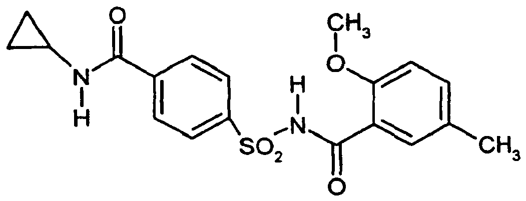 und

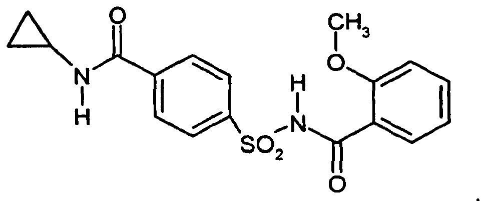

13. Mittel gemäß einem der Ansprüche 11 oder 12, bei denen die Kulturpflanzen-Verträglichkeit verbessernde Verbindung Cloquintocet-mexyl ist.

14. Mittel gemäß einem der Ansprüche 11 oder 12, bei denen die Kulturpflanzen-Verträglichkeit verbessernde Verbindung Mefenpyr-diethyl ist.

15. Verfahren zum Bekämpfen von unerwünschtem Pflanzenwuchs, **dadurch gekennzeichnet, dass** man ein Mittel gemäß Anspruch 11 auf die Pflanzen oder ihre Umgebung einwirken lässt.

16. Verwendung eines Mittels gemäß Anspruch 11 zum Bekämpfen von unerwünschten Pflanzenwuchs.

17. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 11 in zeitlich naher Abfolge getrennt auf die Pflanzen oder ihre Umgebung einwirken lässt.

18. Zusammensetzung umfassend
- mindestens eine Verbindung der Formel (I) gemäß Anspruch 1 oder ein Mittel gemäß Anspruch 11 und
- mindestens ein Salz der Formel (III') 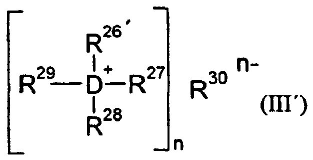 in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

19. Zusammensetzung gemäß Anspruch 18, **dadurch gekennzeichnet, dass** sie mindestens einen Penetrationsförderer enthält.

20. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 11, **dadurch gekennzeichnet, dass** das anwendungsfertige Mittel (Spritzbrühe) unter Einsatz eines Salzes der Formel (III') gemäß Anspruch 18 zubereitet wird.

21. Verfahren gemäß Anspruch 20, **dadurch gekennzeichnet, dass** die Spritzbrühe unter Einsatz eines Penetrationsförderers zubereitet wird.

22. Verbindungen der Formel (II) 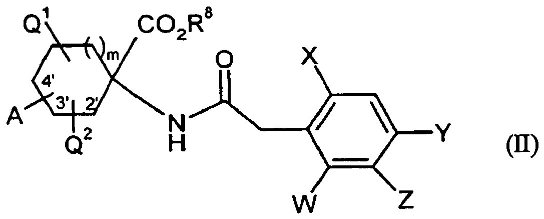 in welcher
A, m, Q¹, Q², W, X, Y und Z die oben angegebene Bedeutung haben.

23. Verbindungen der Formel (III) 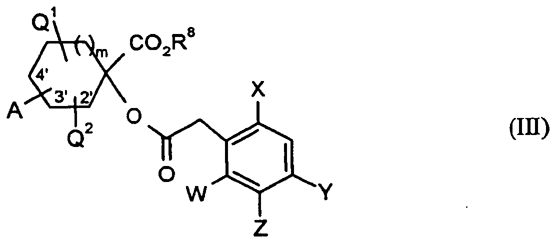 in welcher
A, m, Q¹, Q², W, X, Y, Z und R⁸ die oben angegebene Bedeutung haben.

24. Verbindungen der Formel (XVI) 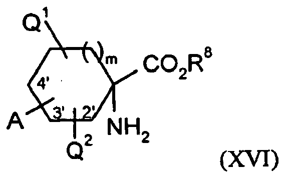 in welcher
A, m, Q¹, Q², und R⁸ die oben angegebene Bedeutung haben.

25. Verbindungen der Formel (XVIII) 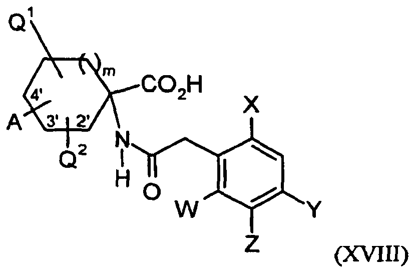 in welcher
A, Q¹, Q², m, W, X, Y, und Z die oben angegebene Bedeutung haben.

26. Verbindungen der Formel (XIX) 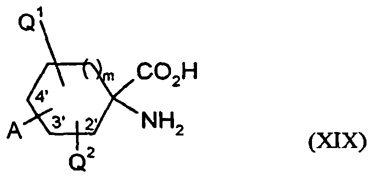 in welcher
A, m, Q¹ und Q² die oben angegebene Bedeutung haben.

27. Verbindungen der Formel (XXI) 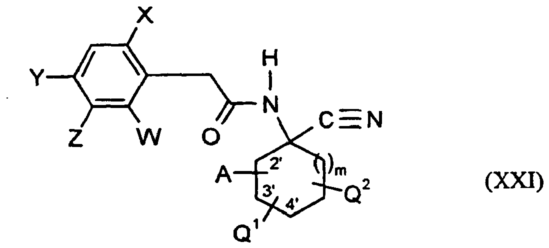 in welcher
A, m, Q¹, Q², W, X, Y, und Z die oben angegebene Bedeutung haben.

28. Verbindungen der Formel (XXII) 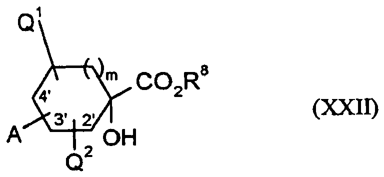 in welcher
A, m, Q¹, Q² und R⁸ die oben angegebene Bedeutung haben.

29. Verbindungen der Formel (XXIII-1)

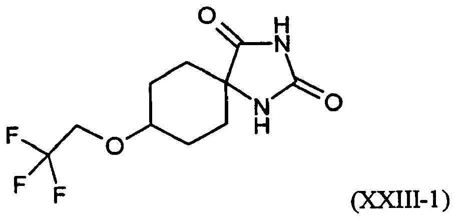

## Claims

1. Compounds of the formula (I) 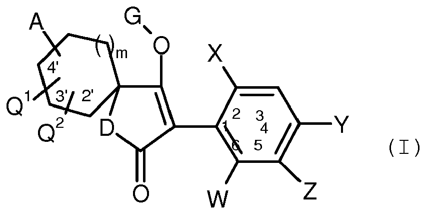 in which
W represents hydrogen, C₁-C₆-alkyl, C₂-C₆-alkenyl, C₂-C₆-alkynyl, represents C₃-C₆-cycloalkyl which is optionally mono- or disubstituted by C₁-C₂-alkyl, C₁-C₂-alkoxy, fluorine, chlorine, trifluoromethyl or C₃-C₆-cycloalkyl, represents halogen, C₁-C₆-alkoxy, C₁-C₄-haloalkyl, C₁-C₄-haloalkoxy or cyano,
X represents halogen, C₁-C₆-alkyl, C₂-C₆-alkenyl, C₂-C₆-alkynyl, represents C₃-C₆-cycloalkyl which is optionally mono- or disubstituted by C₁-C₂-alkyl, C₁-C₂-alkoxy, fluorine, chlorine, trifluoromethyl or C₃-C₆-cycloalkyl, represents C₁-C₆-haloalkyl, C₁-C₆-alkoxy, C₃-C₆-alkenyloxy, C₁-C₆-alkylthio, C₁-C₆-alkylsulfinyl, C₁-C₆-alkylsulfonyl, C₁-C₆-haloalkoxy, C₃-C₆-haloalkenyloxy, nitro or cyano,
Y and Z independently of one another represent hydrogen, halogen, C₁-C₆-alkyl, C₂-C₆-alkenyl, C₂-C₆-alkynyl, represent C₃-C₆-cycloalkyl which is optionally mono- or disubstituted by C₁-C₂-alkyl, C₁-C₂-alkoxy, fluorine, chlorine, trifluoromethyl or C₃-C₆-cycloalkyl, represent C₁-C₆-alkoxy, C₁-C₆-haloalkyl, C₁-C₆-haloalkoxy, cyano, C₂-C₆-alkenyl, C₂-C₆-alkynyl or one of the (het)aryl radicals 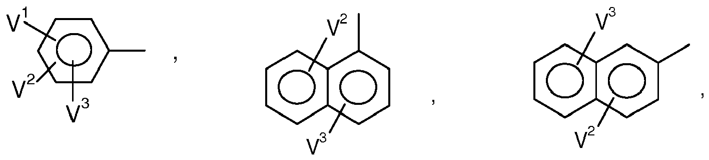 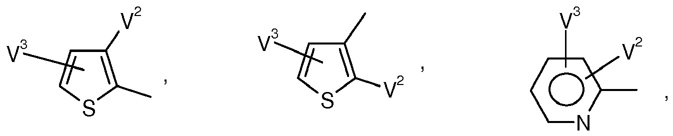 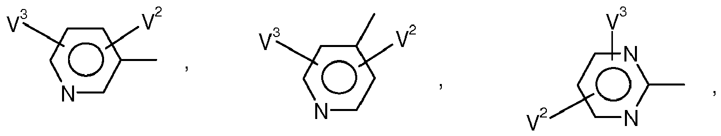 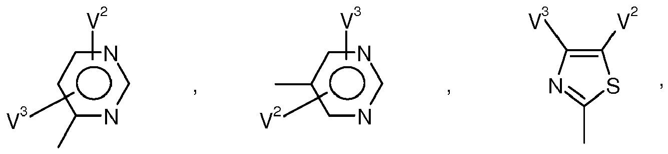 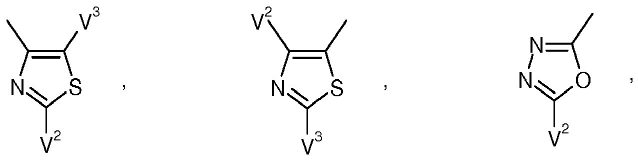 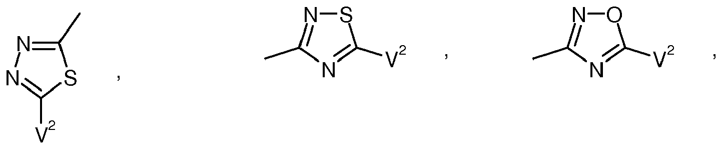 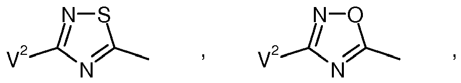 where in the case of (het)aryl only one of the radicals Y or Z may represent (het)aryl,
V¹ represents hydrogen, halogen, C₁-C₁₂-alkyl, C₁-C₆-alkoxy, C₁-C₆-alkylthio, C₁-C₆-alkylsulfinyl, C₁-C₆-alkylsulfonyl, C₁-C₄-haloalkyl, C₁-C₄-haloalkoxy, nitro, cyano, or represents phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally monosubstituted or polysubstituted by halogen, C₁-C₆-alkyl, C₁-C₆-alkoxy, C₁-C₄-haloalkyl, C₁-C₄-haloalkoxy, nitro or cyano,
V² and V³ independently of one another represent hydrogen, halogen, C₁-C₆-alkyl, C₁-C₆-alkoxy, C₁-C₄-haloalkyl or C₁-C₄-haloalkoxy,
A represents C₁-C₄-alkoxy which is mono- to heptasubstituted by fluorine, chlorine, bromine and/or iodine or represents C₃-C₆-cycloalkyl-C₁-C₂-alkoxy which is mono- to pentasubstituted by fluorine, chlorine and/or bromine and which may optionally be substituted by C₁-C₂-alkyl, C₁-C₂-haloalkyl or C₁-C₂-alkoxy,
D represents NH (1) or oxygen (2),
Q¹ and Q² independently of one another represent hydrogen, C₁-C₆-alkyl, C₁-C₂-haloalkyl or C₁-C₄-alkoxy,
m represents the number 0 or 1,
G represents hydrogen (a) or one of the groups 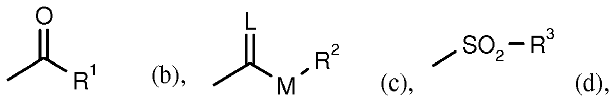 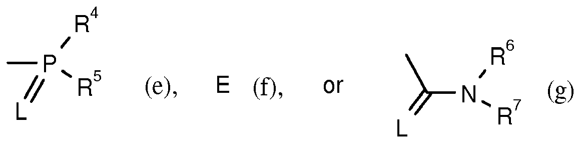 in which
E represents a metal ion or an ammonium ion,
L represents oxygen or sulfur and
M represents oxygen or sulfur,
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or two not directly adjacent methylene groups are replaced by oxygen and/or sulfur,
represents phenyl which is optionally substituted by halogen, cyano, nitro, C₁-C₆-alkyl, C₁-C₆-alkoxy, C₁-C₆-haloalkyl, C₁-C₆-haloalkoxy, C₁-C₆-alkylthio or C₁-C₆-alkylsulfonyl,
represents phenyl-C₁-C₆-alkyl which is optionally substituted by halogen, nitro, cyano, C₁-C₆-alkyl, C₁-C₆-alkoxy, C₁-C₆-haloalkyl or C₁-C₆-haloalkoxy,
represents optionally halogen- or C₁-C₆-alkyl-substituted 5- or 6-membered hetaryl having one or two heteroatoms from the group consisting of oxygen, sulfur and nitrogen,
represents phenoxy-C₁-C₆-alkyl which is optionally substituted by halogen or C₁-C₆-alkyl,
represents optionally halogen-, amino- or C₁-C₆-alkyl-substituted 5- or 6-membered hetaryloxy-C₁-C₆-alkyl having one or two heteroatoms from the group consisting of oxygen, sulfur and nitrogen,
R² represents in each case optionally halogen- or cyano-substituted C₁-C₂₀-alkyl, C₂-C₂₀-alkenyl, C₁-C₈-alkoxy-C₂-C₈-alkyl or poly-C₁-C₈-alkoxy-C₂-C₈-alkyl,
or represents C₃-C₈-cycloalkyl which is optionally substituted by halogen, C₁-C₆-alkyl or C₁-C₆-alkoxy,
or represents phenyl or benzyl, each of which is optionally substituted by halogen, cyano, nitro, C₁-C₆-alkyl, C₁-C₆-alkoxy, C₁-C₆-haloalkyl or C₁-C₆-halogenalkoxy,
R³ represents optionally halogen-substituted C₁-C₈-alkyl or in each case optionally halogen-, C₁-C₆-alkyl-, C₁-C₆-alkoxy-, C₁-C₄-haloalkyl-, C₁-C₄-haloalkoxy-, cyano- or nitro-substituted phenyl or benzyl,
R⁴ and R⁵ independently of one another represent in each case optionally halogen-substituted C₁-C₈-alkyl, C₁-C₈-alkoxy, C₁-C₈-alkylamino, di(C₁-C₈-alkyl) amino, C₁-C₈-alkylthio or C₃-C₈-alkenylthio or represent in each case optionally halogen-, nitro- cyano-, C₁-C₄-alkoxy-, C₁-C₄-haloalkoxy-, C₁-C₄-alkylthio-, C₁-C₄-haloalkylthio-, C₁-C₄-alkyl- or C₁-C₄-haloalkyl-substituted phenyl, phenoxy or phenylthio,
R⁶ and R⁷ independently of one another represent hydrogen, represent in each case optionally halogen- or cyano-substituted C₁-C₈-alkyl, C₃-C₈-cycloalkyl, C₁-C₈-alkoxy, C₃-C₈-alkenyl or C₁-C₈-alkoxy-C₂-C₈-alkyl, represent in each case optionally halogen-, C₁-C₈-alkyl-, C₁-C₈-haloalkyl- or C₁-C₈-alkoxy-substituted phenyl or benzyl or together represent an optionally C₁-C₆-alkylsubstituted C₃-C₆-alkylene radical in which optionally one methylene group is replaced by oxygen or sulfur.

2. Compounds of the formula (I) according to Claim 1 in which
W represents hydrogen, chlorine, bromine, C₁-C₄-alkyl, C₂-C₄-alkenyl, C₂-C₄-alkynyl, represents C₃-C₆-cycloalkyl which is optionally monosubstituted by methyl, ethyl, methoxy, fluorine, chlorine, trifluoromethyl or cyclopropyl, represents C₁-C₄-alkoxy, C₁-C₂-haloalkyl or C₁-C₂-haloalkoxy,
X represents chlorine, bromine, C₁-C₄-alkyl, C₂-C₄-alkenyl, C₂-C₄-alkynyl, represents C₃-C₆-cycloalkyl which is optionally monosubstituted by methyl, ethyl, methoxy, fluorine, chlorine, trifluoromethyl or cyclopropyl, represents C₁-C₄-alkoxy, C₁-C₄-haloalkyl, C₁-C₄-haloalkoxy or cyano,
Y and Z independently of one another represent hydrogen, fluorine, chlorine, bromine, C₁-C₄-alkyl, C₂-C₄-alkenyl, C₂-C₄-alkynyl, represent C₃-C₆-cycloalkyl which is optionally monosubstituted by methyl, ethyl, methoxy, fluorine, chlorine, trifluoromethyl or cyclopropyl, represents C₁-C₆-alkoxy, C₁-C₄-haloalkyl, C₁-C₄-haloalkoxy, cyano, C₂-C₄-alkenyl, C₂-C₄-alkynyl or one of the (het)aryl radicals, 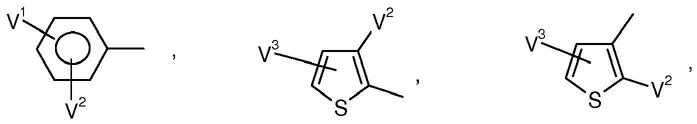 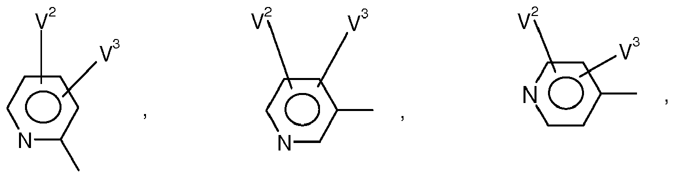 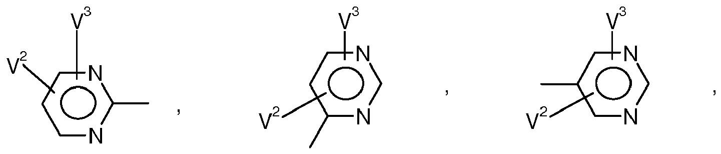 where in the case of (het)aryl only one of the radicals Y or Z may represent (het)aryl,
V¹ represents hydrogen, fluorine, chlorine, bromine, C₁-C₆-alkyl, C₁-C₄-alkoxy, C₁-C₂-haloalkyl, C₁-C₂-haloalkoxy, nitro, cyano or represents phenyl which is optionally mono- or disubstituted by fluorine, chlorine, bromine, C₁-C₄-alkyl, C₁-C₄-alkoxy, C₁-C₂-haloalkyl, C₁-C₂-haloalkoxy, nitro or cyano,
V² and V² independently of one another represent hydrogen, fluorine, chlorine, bromine, C₁-C₄-alkyl, C₁-C₄-alkoxy, C₁-C₂-haloalkyl or C₁-C₂-haloalkoxy,
A represents C₁-C₄-alkoxy which is mono- to pentasubstituted by fluorine, chlorine and/or bromine or represents C₃-C₆-cycloalkyl-C₁-C₂-alkoxy which is mono- to trisubstituted by fluorine and/or chlorine,
D represents NH (1) or oxygen (2),
Q¹ and Q² independently of one another represent hydrogen, methyl, ethyl, trifluoromethyl, methoxy or ethoxy,
m represents the number 0 or 1,
G represents hydrogen (a) or one of the groups 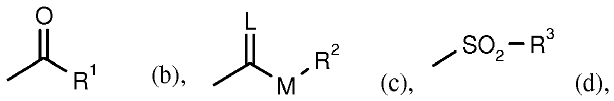 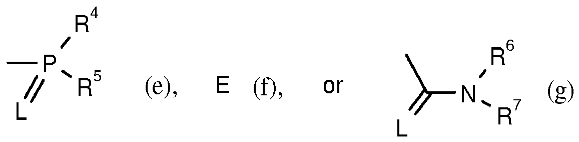 in which
E represents a metal ion or an ammonium ion,
L represents oxygen or sulfur and
M represents oxygen or sulfur,
R¹ represents C₁-C₁₆-alkyl, C₂-C₁₆-alkenyl, C₁-C₆-alkoxy-C₁-C₄-alkyl, C₁-C₆-alkylthio-C₁-C₄-alkyl or poly-C₁-C₁-alkoxy-C₁-C₄-alkyl, each of which is optionally mono- to trisubstituted by fluorine or chlorine, or represents C₃-C₇-cycloalkyl which is optionally mono- or disubstituted by fluorine, chlorine, C₁-C₅-alkyl or C₁-C₅-alkoxy and in which optionally one or two not directly adjacent methylene groups are replaced by oxygen and/or sulfur,
represents phenyl which is optionally mono- to trisubstituted by fluorine, chlorine, bromine, cyano, nitro, C₁-C₄-alkyl, C₁-C₄-alkoxy, C₁-C₃-haloalkyl, C₁-C₃-haloalkoxy, C₁-C₄-alkylthio or C₁-C₄-alkylsulfonyl,
represents phenyl-C₁-C₄-alkyl which is optionally mono- or disubstituted by fluorine, chlorine, bromine, C₁-C₄-alkyl, C₁-C₄-alkoxy, C₁-C₃-haloalkyl or C₁-C₃-haloalkoxy,
represents pyrazolyl, thiazolyl, pyridyl, pyrimidyl, furanyl or thienyl, each of which is optionally mono- or disubstituted by fluorine, chlorine, bromine or C₁-C₄-alkyl,
represents phenoxy-C₁-C₅-alkyl which is optionally mono- or disubstituted by fluorine, chlorine, bromine or C₁-C₄-alkyl or
represents pyridyloxy-C₁-C₅-alkyl, pyrimidyl-oxy-C₁-C₅-alkyl or thiazolyloxy-C₁-C₅-alkyl, each of which is optionally mono- or disubstituted by fluorine, chlorine, bromine, amino or C₁-C₄-alkyl,
R² particularly preferably represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally mono- to trisubstituted by fluorine or chlorine,
represents C₃-C₇-cycloalkyl which is optionally mono- or disubstituted by fluorine, chlorine, C₁-C₄-alkyl or C₁-C₄-alkoxy or
represents phenyl or benzyl, each of which is optionally mono- to trisubstituted by fluorine, chlorine, bromine, cyano, nitro, C₁-C₄-alkyl, C₁-C₃-alkoxy, C₁-C₃-haloalkyl or C₁-C₃-haloalkoxy,
R³ represents C₁-C₆-alkyl which is optionally mono- to trisubstituted by fluorine or chlorine or represents phenyl or benzyl, each of which is optionally mono- or disubstituted by fluorine, chlorine, bromine, C₁-C₄-alkyl, C₁-C₄-alkoxy, C₁-C₂-haloalkoxy, C₁-C₂-haloalkyl, cyano or nitro,
R⁴ and R⁵ independently of one another represent C₁-C₆-alkyl, C₁-C₆-alkoxy, C₁-C₆-alkylamino, di-(C₁-C₆-alkyl) amino, C₁-C₆-alkylthio or C₃-C₄-alkenylthio, each of which is optionally mono- to trisubstituted by fluorine or chlorine, or represent phenyl, phenoxy or phenylthio, each of which is optionally mono- or disubstituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or C₁-C₃-haloalkyl,
R⁶ and R⁷ independently of one another represent hydrogen, represent C₁-C₆-alkyl, C₃-C₆-cycloalkyl, C₁-C₆-alkoxy, C₃-C₆-alkenyl or C₁-C₆-alkoxy-C₂-C₆-alkyl, each of which is optionally mono- to trisubstituted by fluorine or chlorine, represent phenyl or benzyl, each of which is optionally mono- to trisubstituted by fluorine, chlorine, bromine, C₁-C₅-haloalkyl, C₁-C₅-alkyl or C₁-C₅-alkoxy, or together represent an optionally C₁-C₄-alkyl-substituted C₃-C₆-alkylene radical in which optionally one methylene group is replaced by oxygen or sulfur.

3. Compounds of the formula (I) according to Claim 1 in which
W represents hydrogen, chlorine, bromine, methyl, ethyl, vinyl, ethynyl, propynyl, cyclopropyl, methoxy, ethoxy or trifluoromethyl,
X represents chlorine, bromine, methyl, ethyl, propyl, isopropyl, vinyl, ethynyl, propynyl, cyclopropyl, methoxy, ethoxy, trifluoromethyl, difluoromethoxy, trifluoromethoxy or cyano,
Y and Z independently of one another represent hydrogen, fluorine, chlorine, bromine, methyl, ethyl, vinyl, ethynyl, propynyl, cyclopropyl, methoxy, trifluoromethyl, trifluoromethoxy, cyano or a phenyl radical, 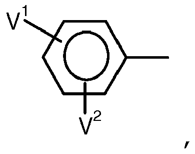 where in the case of phenyl only one of the radicals Y or Z may represent phenyl,
V¹ represents hydrogen, fluorine, chlorine, bromine, methyl, ethyl, n-propyl, isopropyl, tert-butyl, methoxy, ethoxy, n-propoxy, isopropoxy, trifluoromethyl or trifluoromethoxy,
V² represents hydrogen, fluorine, chlorine, methyl, ethyl, n-propyl, isopropyl, methoxy, ethoxy or trifluoromethyl,
A represents methoxy, ethoxy, propoxy, butoxy or isobutoxy, each of which is mono- to trisubstituted by fluorine and/or chlorine, or represents cyclopropylmethoxy or cyclopropylethoxy, each of which is mono- to trisubstituted by fluorine and/or chlorine,
D represents NH (1) or oxygen (2),
Q¹ and Q² represent hydrogen,
m represents the number 1,
G represents hydrogen (a) or one of the groups 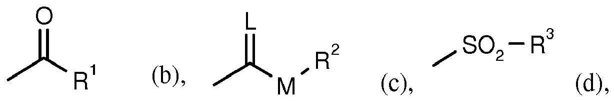 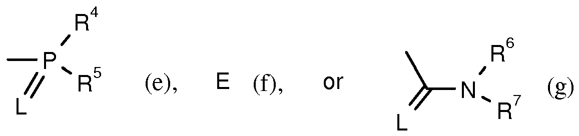 in which
E represents a metal ion or an ammonium ion,
L represents oxygen or sulfur and
M represents oxygen or sulfur,
R¹ represents C₁-C₁₀-alkyl, C₂-C₁₀-alkenyl, C₁-C₄-alkoxy-C₁-C₂-alkyl, C₁-C₄-alkylthio-C₁-C₂-alkyl, each of which is optionally mono- to trisubstituted by fluorine or chlorine, or represents C₃-C₆-cycloalkyl which is optionally monosubstituted by fluorine, chlorine, methyl, ethyl or methoxy,
represents phenyl which is optionally mono- or disubstituted by fluorine, chlorine, bromine, cyano, nitro, methyl, ethyl, n-propyl, isopropyl, methoxy, ethoxy, trifluoromethyl or trifluoromethoxy,
represents furanyl, thienyl or pyridyl, each of which is optionally monosubstituted by chlorine, bromine or methyl,
R² represents C₁-C₁₀-alkyl, C₂-C₁₀-alkenyl or C₁-C₄-alkoxy-C₂-C₄-alkyl, each of which is optionally mono- to trisubstituted by fluorine or chlorine,
represents cyclopentyl or cyclohexyl,
or represents phenyl or benzyl, each of which is optionally mono- or disubstituted by fluorine, chlorine, cyano, nitro, methyl, ethyl, methoxy, trifluoromethyl or trifluoromethoxy,
R³ represents methyl, ethyl, propyl or isopropyl, each of which is optionally mono- to trisubstituted by fluorine or chlorine, or represents phenyl which is optionally monosubstituted by fluorine, chlorine, bromine, methyl, ethyl, isopropyl, tert-butyl, methoxy, ethoxy, isopropoxy, trifluoromethyl, trifluoromethoxy, cyano or nitro,
R⁴ and R⁵ independently of one another represent C₁-C₄-alkoxy or C₁-C₄-alkylthio or represent phenyl, phenoxy or phenylthio, each of which is optionally monosubstituted by fluorine, chlorine, bromine, nitro, cyano, methyl, methoxy, trifluoromethyl or trifluoromethoxy,
R⁶ and R⁷ independently of one another represent hydrogen, C₁-C₄-alkyl, C₃-C₆-cycloalkyl, C₁-C₄-alkoxy, C₃-C₄-alkenyl or C₁-C₄-alkoxy-C₂-C₄-alkyl, represent phenyl which is optionally mono- or disubstituted by fluorine, chlorine, bromine, methyl, methoxy or trifluoromethyl, or together represent a C₅-C₆-alkylene radical in which optionally one methylene group is replaced by oxygen or sulfur.

4. Compounds of the formula (I) according to Claim 1 in which
W represents hydrogen, chlorine, bromine, methyl, ethyl or methoxy,
X represents chlorine, bromine, methyl, ethyl, methoxy or ethoxy,
Y and Z independently of one another represent hydrogen, chlorine, bromine, methyl, methoxy, cyclopropyl or represent the radicals 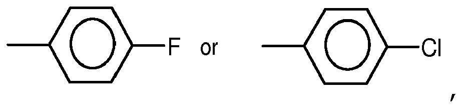 where in this case only one of the radicals Y or Z may represent a substituted phenyl radical,
A represents methoxy, ethoxy or propoxy, each of which is mono- to trisubstituted by fluorine and/or chlorine,
D represents NH (1) or oxygen (2),
Q¹ and Q² represent hydrogen,
m represents the number 1,
G represents hydrogen (a) or one of the groups 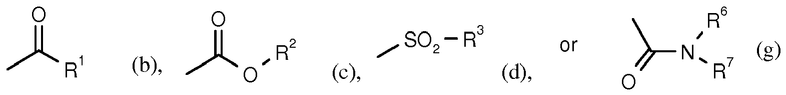
R¹ represents C₁-C₁₀-alkyl, C₁-C₄-alkoxy-C₁-C₂-alkyl, C₃-C₆-cycloalkyl,
represents phenyl which is optionally monosubstituted by chlorine, or represents thienyl,
R² represents C₁-C₁₀-alkyl, C₂-C₁₀-alkenyl, or represents benzyl,
R³ represents methyl,
R⁶ and R⁷ together represent a C₅-C₆-alkylene radical in which optionally one methylene group is replaced by oxygen or sulfur.

5. Process for the preparation of compounds of the formula (I) according to Claim 1, **characterized in that**, to obtain
(A) compounds of the formula (I-1-a) 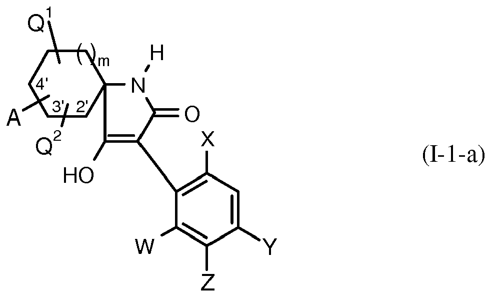 in which
A, Q¹, Q², m, W, X, Y and Z have the meanings given above,
compounds of the formula (II) 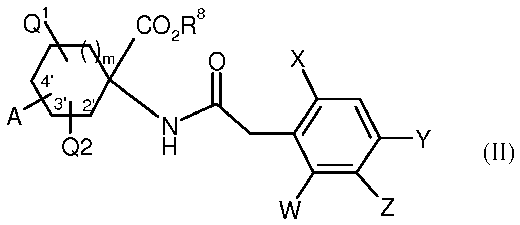 in which
A, Q¹, Q², m, W, X, Y and Z have the meanings given above,
and
R⁸ represents alkyl,
are subjected to an intramolecular condensation reaction in the presence of a diluent and in the presence of a base,
(B) compounds of the formula (I-2-a) 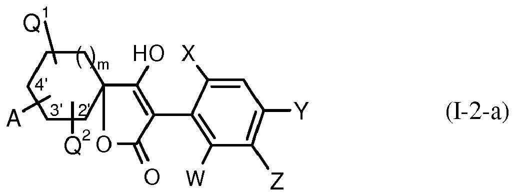 in which
A, Q¹, Q², m, W, X, Y and Z have the meanings given above,
compounds of the formula (III) 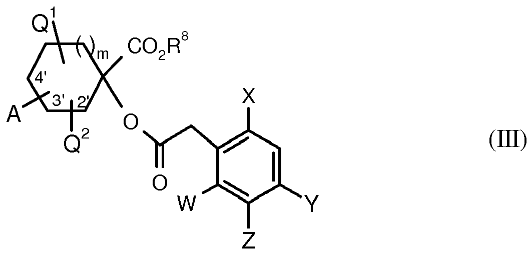 in which
A, Q¹, Q², m, W, X, Y, Z and R⁸ have the meanings given above,
are subjected to an intramolecular condensation reaction in the presence of a diluent and in the presence of a base,
(C) compounds of the formulae (I-1-b) to (I-2-b) shown above in which R¹, A, Q¹, Q², m, W, X, Y and Z have the meanings given above, compounds of the formulae (I-1-a) to (I-2-a) in which A, Q¹, Q², m, W, X, Y and Z have the meanings given above are in each case
α) reacted with compounds of the formula (IV) 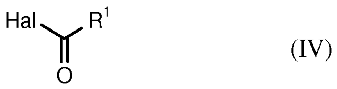 in which
R¹ has the meaning given above and
Hal represents halogen
or
β) with carboxylic anhydrides of the formula (V)
R¹-CO-O-CO-R¹ (V)
in which
R¹ has the meaning given above,
if appropriate in the presence of a diluent and if appropriate in the presence of an acid binder;
(D) compounds of the formulae (I-1-c) to (I-2-c) shown above in which R², A, Q¹, Q², m, W, M, X, Y and Z have the meanings given above, and L represents oxygen, compounds of the formulae (I-1-a) to (I-2-a) shown above in which A, Q¹, Q², m, W, X, Y and Z have the meanings given above are in each case
reacted with chloroformic esters or chloroformic thioesters of the formula (VI)
R²-M-CO-Cl (VI)
in which
R² and M have the meanings given above,
if appropriate in the presence of a diluent and if appropriate in the presence of an acid binder;
(E) compounds of the formulae (I-1-c) to (I-2-c) shown above in which R², A, Q¹, Q², m, W, M, X, Y and Z have the meanings given above, and L represents sulfur, compounds of the formulae (I-1-a) to (I-2-a) shown above in which A, Q¹, Q², m, W, X, Y and Z have the meanings given above are in each case
reacted with chloromonothioformic esters or chlorodithioformic esters of the formula (VII) 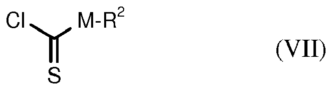 in which
M and R² have the meanings given above,
if appropriate in the presence of a diluent and if appropriate in the presence of an acid binder,
(F) compounds of the formulae (I-1-d) to (I-2-d) shown above in which R³, A, W, Q¹, Q², m, X, Y and Z have the meanings given above, compounds of the formulae (I-1-a) to (I-2-a) shown above in which A, B, Q¹, Q², m, W, X, Y and Z have the meanings given above are in each case
reacted with sulfonyl chlorides of the formula (VIII)
R³-SO₂-Cl (VIII)
in which
R³ has the meaning given above,
if appropriate in the presence of a diluent and if appropriate in the presence of an acid binder,
(G) compounds of the formulae (I-1-e) to (I-2-e) shown above in which L, R⁴, R⁵, A, Q¹, Q², m, W, X, Y and Z have the meanings given above, compounds of the formulae (I-1-a) to (I-2-a) shown above in which A, Q¹, Q², m, W, X, Y and Z have the meanings given above are in each case
reacted with phosphorus compounds of the formula (IX) 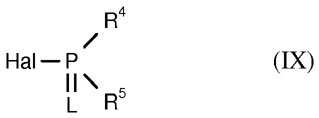 in which
L, R⁴ and R⁵ have the meanings given above
and
Hal represents halogen
if appropriate in the presence of a diluent and if appropriate in the presence of an acid binder,
(H) compounds of the formulae (I-1-f) to (I-2-f) shown above in which E, A, Q¹, Q², m, W, X, Y and Z have the meanings given above, compounds of the formulae (I-1-a) to (I-2-a) in which A, Q¹, Q², m, W, X, Y and Z have the meanings given above are in each case
reacted with metal compounds or amines of the formulae (X) or (XI)
Me(OR¹⁰)ₜ (X) 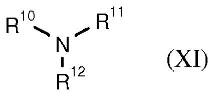
in which
Me represents a monovalent or divalent metal,
t represents the number 1 or 2 and
R¹⁰, R¹¹, R¹² independently of one another represent hydrogen or alkyl,
if appropriate in the presence of a diluent,
(I) compounds of the formulae (I-1-g) to (I-2-g) shown above in which L, R⁶, R⁷, A, Q¹, Q², m, W, X, Y L, R⁶, R⁷, A, Q¹, Q², M, W, X, Y and Z have the meanings given above, compounds of the formulae (I-1-a) to (I-2-a) shown above in which A, Q¹, Q², m, W, X, Y and Z have the meanings given above are in each case
α) reacted with isocyanates or isothiocyanates of the formula (XII)
R⁶-N=C=L (XII)
in which
R⁶ and L have the meanings given above,
if appropriate in the presence of a diluent and if appropriate in the presence of a catalyst, or
β) reacted with carbamoyl chlorides or thiocarbamoyl chlorides of the formula (XIII) 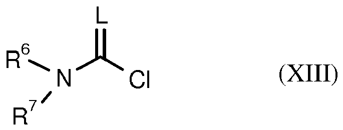 in which
L, R⁶ and R⁷ have the meanings given above,
if appropriate in the presence of a diluent and if appropriate in the presence of an acid binder,
(Jα) compounds of the formulae (I-1-a) to (I-1-g) shown above in which A, D, G, Q¹, Q², m, W, X, Y and Z have the meaning given above, compounds of the formulae (I-1-a') to (1-2-g') in which A, D, G, Q¹, Q², m, W, X and Y have the meaning given above and Z' represents bromine or iodine 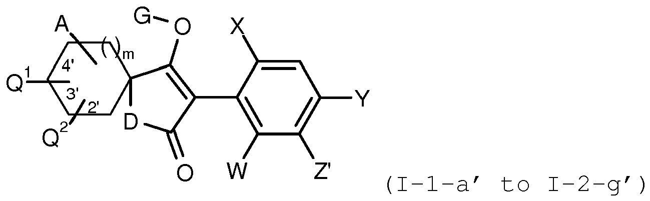 and
(Jβ) compounds of the formulae (I-1-a) to (I-2-g) shown above in which A, D, G, Q¹, Q², m, W, X, Y and Z have the meaning given above, compounds of the formulae (I-1-a") to (1-2-g") in which A, D, G, Q¹, Q², m, W, X and Z have the meaning given above and Y' represents bromine or iodine 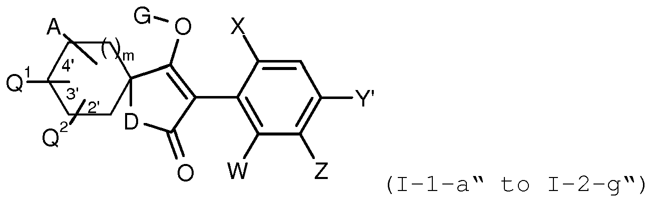 are coupled with (het)aryl derivatives of the formulae (XVα) and (XVβ) 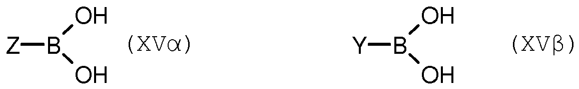 or esters thereof, in the presence of a solvent, in the presence of a catalyst and in presence of a base.

6. Composition for controlling pests and/or unwanted vegetation, **characterized in that** it comprises at least one compound of the formula (I) according to Claim 1.

7. Method of controlling animal pests and/or unwanted vegetation, **characterized in that** compounds of the formula (I) according to Claim 1 are allowed to act on pests, unwanted vegetation and/or their habitat, with the exception of methods for the surgical or therapeutic treatment of the human or animal body.

8. Compounds of the formula (I) according to Claim 1 for controlling animal pests and/or unwanted vegetation.

9. Process for preparing compositions for controlling pests and/or unwanted vegetation, **characterized in that** compounds of the formula (I) according to Claim 1 are mixed with extenders and/or surfactants.

10. Use of compounds of the formula (I) according to Claim 1 for preparing compositions for controlling pests and/or unwanted vegetation.

11. Composition comprising an effective amount of an active compound combination comprising, as components,
(a') at least one compound of the formula (I) in which A, D, G, Q¹, Q^{G}, m, W, X, Y and Z have the meaning given above
and
(b') at least one crop plant compatibility-improving compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl - cf. also related compounds in EP-A-86750, EP-A-94349, EP-A-191736, EP-A-492366), 3-(2-chlorobenzyl)-1-(1-methyl-1-phenylethyl)urea (cumyluron), α-(cyanomethox-imino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methylphenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichlorophenyl)-4,5-dihydro-5-methyl-1H-pyrazole-3,5-dicarboxylate (mefenpyrdiethyl - cf. also related compounds in WO-A-91/07874), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate (cf. also related compounds in EP-A-269806 and EP-A-333131), ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate (cf. also related compounds in WO-A-91/08202), 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (cf. also related compounds in EP-A-582198), 4-carboxychroman-4-ylacetic acid (AC-304415, cf. EP-A-613618), 4-chlorophenoxyacetic acid, 3,3'-dimethyl-4-methoxybenzophenone, 1-bromo-4-chloromethylsulfonylbenzene, 1-[4-(N-2-methoxybenzoylsulfamoyl)phenyl]-3-methylurea (also known as N-(2-methoxybenzoyl)-4-[(methylaminocarbonyl)amino]benzenesulfonamide), 1-[4-(N-2-methoxybenzoylsulfamoyl)phenyl]-3,3-dimethylurea, 1-[4-(N-4,5-dimethylbenzoylsulfamoyl)phenyl]-3-methylurea, 1-[4-(N-naphthylsulfamoyl)phenyl]-3,3-dimethylurea, N-(2-methoxy-5-methylbenzoyl)-4-(cyclopropylaminocarbonyl)benzenesulfonamide,
and/or one of the following compounds, defined by general formulae,
of the general formula (IIa) 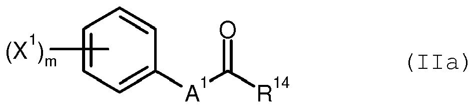 or of the general formula (IIb) 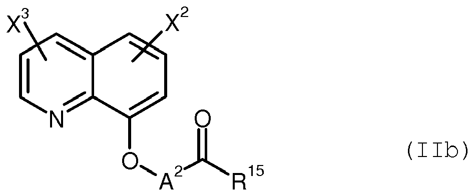 or of the formula (IIc) 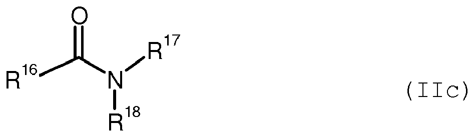 where
m represents a number 0, 1, 2, 3, 4 or 5,
A¹ represents one of the divalent heterocyclic groupings shown below, 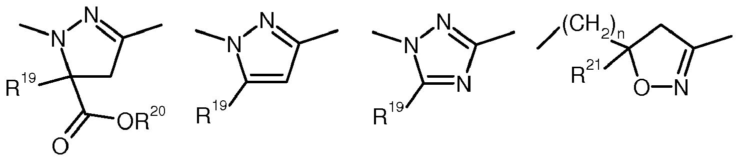
n represents a number 0, 1, 2, 3, 4 or 5,
A² represents optionally C₁-C₄-alkyl- and/or C₁-C₄-alkoxycarbonyl- and/or C₁-C₄-alkenyloxycarbonyl-substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di (C₁-C₄-alkyl) amino,
R¹⁵ represents hydroxyl, mercapto, amino, C₁-C₇-alkoxy, C₁-C₆-alkylthio, C₁-C₆-alkenyloxy, C₁-C₆-alkenyloxy-C₁-C₆-alkoxy, C₁-C₆-alkylamino or di(C₁-C₄-alkyl)amino,
R¹⁶ represents optionally fluorine-, chlorine- and/or bromine-substituted C₁-C₄-alkyl,
R¹⁷ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl, R¹⁷ and R¹⁸ also together represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are attached, form a 5- or 6-membered carbocycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy-substituted C₁-C₆-alkyl, C₃-C₆-cycloalkyl or tri-(C₁-C₄-alkyl)silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds, defined by general formulae,
of the general formula (IId) 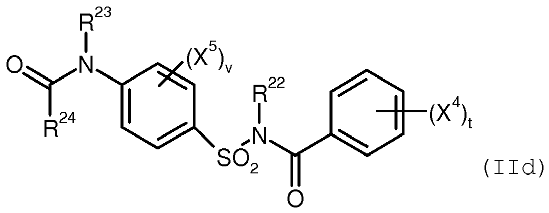 or of the general formula (IIe) 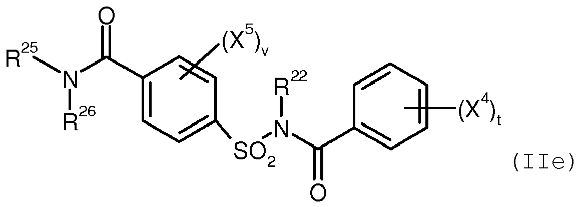 where
t represents a number 0, 1, 2, 3, 4 or 5,
v represents a number 0, 1, 2, 3, 4 or 5,
R²² represents hydrogen or C₁-C₄-alkyl,
R²³ represents hydrogen or C₁-C₄-alkyl,
R²⁴ represents hydrogen, in each case optionally cyano-, halogen- or C₁-C₄-alkoxy-substituted C₁-C₆-alkyl, C₁-C₆-alkoxy, C₁-C₆-alkylthio, C₁-C₆-alkylamino or di (C₁-C₄-alkyl) amino, or in each case optionally cyano-, halogen- or C₁-C₄-alkyl-substituted C₃-C₆-cycloalkyl, C₃-C₆-cycloalkyloxy, C₃-C₆-cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano-, halogen- or C₁-C₄-alkyl-substituted C₃-C₆-cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆-cycloalkyl, or optionally nitro-, cyano-, halogen-, C₁-C₄-alkyl-, C₁-C₄-haloalkyl-, C₁-C₄-alkoxy- or C₁-C₄-haloalkoxy-substituted phenyl, or together with R²⁵ represents in each case optionally C₁-C₄-alkyl-substituted C₂-C₆-alkanediyl or C₂-C₅-oxaalkanediyl,
X⁴ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy.

12. The composition according to Claim 11 where the crop plant compatibility-improving compound is selected from the following group of compounds:
cloquintocet-mexyl, fenchlorazole-ethyl, isoxadifen-ethyl, mefenpyr-diethyl, furilazole, fenclorim, cumyluron, dymron or the compounds 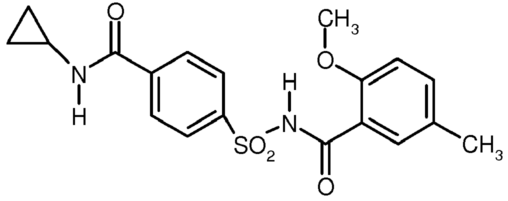 and

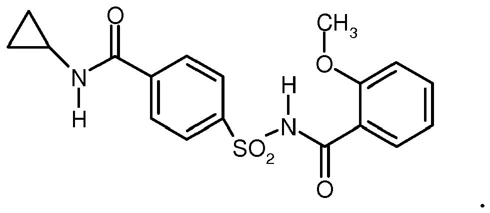

13. Compositions according to Claim 11 or 12 where the crop plant compatibility-improving compound is cloquintocet-mexyl.

14. Compositions according to Claim 11 or 12 where the crop plant compatibility-improving compound is mefenpyr-diethyl.

15. Method for controlling unwanted vegetation, **characterized in that** a composition according to Claim 11 is allowed to act on the plants or their surroundings.

16. Use of a composition according to Claim 11 for controlling unwanted vegetation.

17. Method for controlling unwanted vegetation, **characterized in that** a compound of the formula (I) according to Claim 1 and the crop plant compatibility-improving compound according to Claim 11 are allowed to act separately, in close temporal succession on the plants or their surroundings.

18. Composition, comprising
- at least one compound of the formula (I) according to Claim 1 or a composition according to Claim 11 and
- at least one salt of the formula (III') 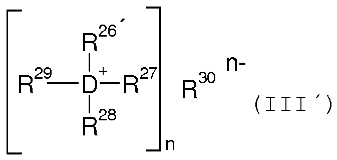 in which
D represents nitrogen or phosphorus,
R²⁶, R²⁷, R²⁸ and R²⁹ independently of one another represent hydrogen or in each case optionally substituted C₁-C₈-alkyl or mono- or polyunsaturated, optionally substituted C₁-C₈-alkylene, the substituents being selectable from halogen, nitro and cyano,
n represents 1, 2, 3 or 4,
R represents an inorganic or organic anion.

19. Composition according to Claim 18, **characterized in that** it comprises at least one penetrant.

20. Method of increasing the activity of pesticides and/or herbicides comprising an active compound of the formula (I) according to Claim 1 or a composition according to Claim 11, **characterized in that** the ready-to-use material (spray liquor) is prepared using a salt of the formula (III') according to Claim 18.

21. Method according to Claim 20, **characterized in that** the spray liquor is prepared using a penetrant.

22. Compounds of the formula (II) 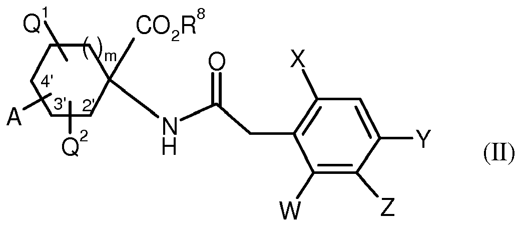 in which
A, m, Q¹, Q², W, X, Y and Z have the meaning given above.

23. Compounds of the formula (III) 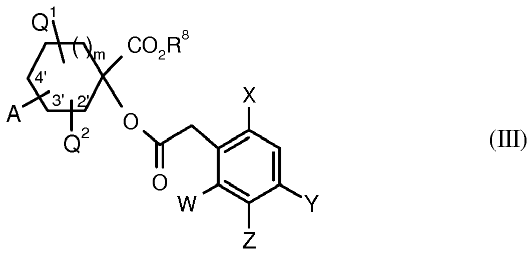 in which
A, m, Q¹, Q², W, X, Y, Z and R⁸ have the meaning given above.

24. Compounds of the formula (XVI) 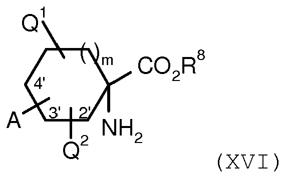 in which
A, m, Q¹, Q² and R⁸ have the meaning given above.

25. Compounds of the formula (XVIII) 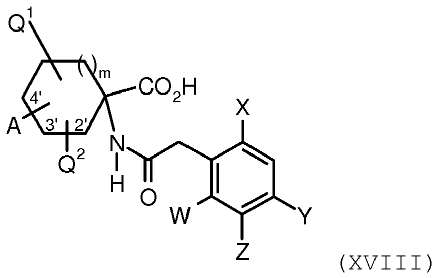 in which
A, Q¹, Q², m, W, X, Y and Z have the meaning given above.

26. Compounds of the formula (XIX) 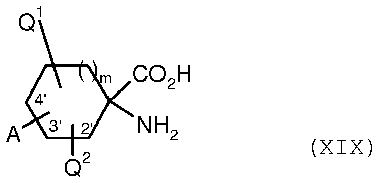 in which
A, m, Q¹ and Q² have the meaning given above.

27. Compounds of the formula (XXI) 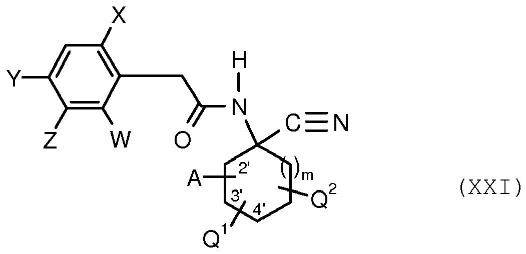 in which
A, m, Q¹, Q², W, X, Y and Z have the meaning given above.

28. Compounds of the formula (XXII) 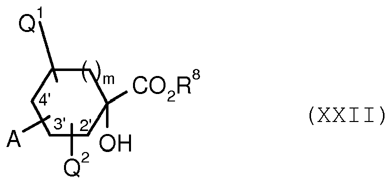 in which
A, m, Q¹, Q² and R⁸ have the meaning given above.

29. Compounds of the formula (XXIII-1)

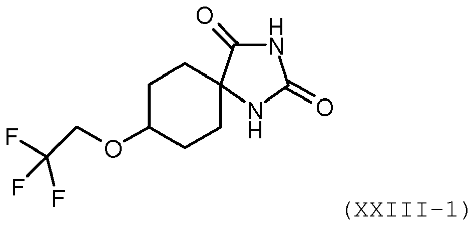

## Revendications

1. Composés de formule (I) 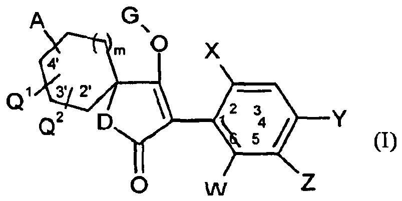 dans laquelle
W représente hydrogène, C₁-C₆-alkyle, C₂-C₆-alcényle, C₂-C₆-alcynyle ; C₃-C₆-cycloalkyle le cas échéant monosubstitué à disubstitué par C₁-C₂-alkyle, C₁-C₂-alcoxy, fluor, chlore, trifluorométhyle ou C₃-C₆-cycloalkyl ; halogène, C₁-C₆-alcoxy, C₁-C₄-halogénoalkyle, C₁-C₄-halogénoalcoxy ou cyano,
X représente halogène, C₁-C₆-alkyle, C₂-C₆-alcényle, C₂-C₆-alcynyle ; C₃-C₆-cycloalkyle le cas échéant monosubstitué à disubstitué par C₁-C₂-alkyle, C₁-C₂-alcoxy, fluor, chlore, trifluorométhyle ou C₃-C₆-cycloalkyl ; C₁-C₆-halogénoalkyle, C₁-C₆-alcoxy, C₃-C₆-alcényloxy, C₁-C₆-alkylthio, C₁-C₆-alkylsulfinyle, C₁-C₆-alkylsulfonyle, C₁-C₆-halogénoalcoxy, C₃-C₆-halogénoalcényloxy, nitro ou cyano,
Y et Z représentent, indépendamment l'un de l'autre, hydrogène, halogène, C₁-C₆-alkyle, C₂-C₆-alcényle, C₂-C₆-alcynyle ; C₃-C₆-cycloalkyle le cas échéant monosubstitué à disubstitué par C₁-C₂-alkyle, C₁-C₂-alcoxy, fluor, chlore, trifluorométhyle ou C₃-C₆-cycloalkyl ; C₁-C₆-alcoxy, C₁-C₆-halogénoalkyle, C₁-C₆-halogénoalcoxyle, cyano, C₂-C₆-alcényle, C₂-C₆-alcynyle ou un des radicaux (Het)-aryle 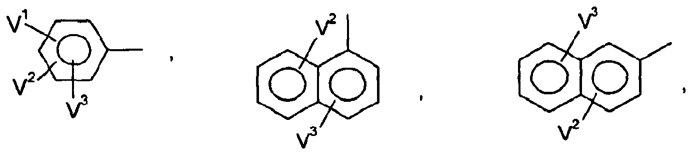 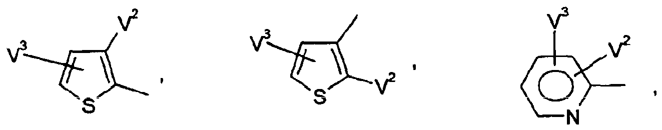 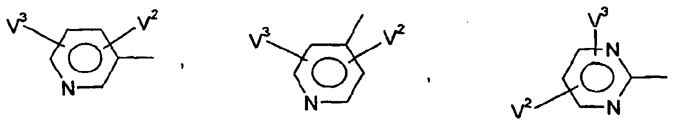 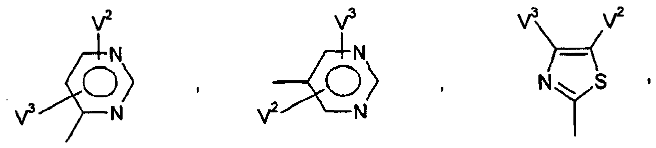 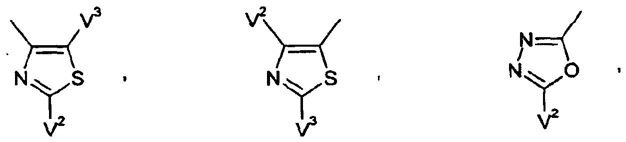 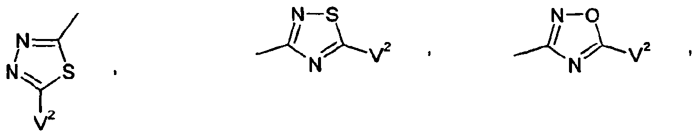 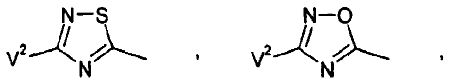 où, dans le cas de (Het)-aryle, seul un des radicaux Y ou Z peut représenter (Het)-aryle,
V¹ représente hydrogène, halogène, C₁-C₁₂-alkyle, C₁-C₆-alcoxy, C₁-C₆-alkylthio, C₁-C₆-alkylsulfinyle, C₁-C₆-alkylsulfonyle, C₁-C₄-halogénoalkyle, C₁-C₄-halogénoalcoxy, nitro, cyano ; ou phényle, phénoxy, phénoxy-C₁-C₄-alkyle, phényl-C₁-C₄-alcoxy, phénylthio-C₁-C₄-alkyle ou phényl-C₁-C₄-alkylthio à chaque fois le cas échéant monosubstitué ou polysubstitué par halogène, C₁-C₆-alkyle, C₁-C₆-alcoxy, C₁-C₄-halogénoalkyle, C₁-C₄-halogénoalcoxy, nitro ou cyano,
V² et V³ représentent, indépendamment l'un de l'autre, hydrogène, halogène, C₁-C₆-alkyle, C₁-C₆-alcoxy, C₁-C₄-halogénoalkyle ou C₁-C₄-halogénoalcoxy,
A représente C₁-C₄-alcoxy monosubstitué à heptasubstitué par fluor, chlore, brome et/ou iode ; ou C₃-C₆-cycloalkyl-C₁-C₂-alcoxy monosubstitué à pentasubstitué par fluor, chlore et/ou brome, qui peut le cas échéant être substitué par C₁-C₂-alkyle, C₁-C₂-halogénoalkyle ou C₁-C₂-alcoxy,
D représente NH (1) ou oxygène (2),
Q¹ et Q² représentent, indépendamment l'un de l'autre, hydrogène, C₁-C₆-alkyle, C₁-C₂-halogénoalkyle ou C₁-C₄-alcoxy,
m vaut le nombre 0 ou 1,
G représente hydrogène (a) ou un des groupes 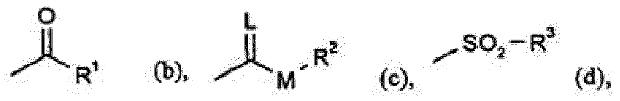 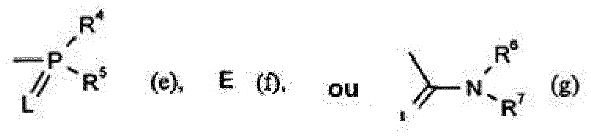 dans lesquels
E représente un ion métallique ou un ion d'ammonium,
L représente oxygène ou soufre et
M représente oxygène ou soufre,
R¹ représente C₁-C₂₀-alkyle, C₂-C₂₀-alcényle, C₁-C₈-alcoxy-C₁-C₈-alkyle, C₁-C₈-alkylthio-C₁-C₈-alkyle, poly-C₁-C₈-alcoxy-C₁-C₈-alkyle à chaque fois le cas échéant substitué par halogène ou cyano ; ou C₃-C₈-cycloalkyle le cas échéant substitué par halogène, C₁-C₆-alkyle ou C₁-C₆-alcoxy, dans lequel le cas échéant un ou deux groupes méthylène non directement adjacents sont remplacés par oxygène et/ou soufre,
représente phényle, le cas échéant substitué par halogène, cyano, nitro, C₁-C₆-alkyle, C₁-C₆-alcoxy, C₁-C₆-halogénoalkyle, C₁-C₆-halogénoalcoxy, C₁-C₆-alkylthio ou C₁-C₆-alkylsulfonyle, représente phényl-Co-Co,-alkyle le cas échéant substitué par halogène, nitro, cyano, C₁-C₆-alkyle, C₁-C₆-alcoxy, C₁-C₆-halogénoalkyle ou C₁-C₆-halogénoalcoxy,
représente hétaryle de 5 ou 6 chaînons, le cas échéant substitué par halogène ou C₁-C₆-alkyle, présentant un ou deux hétéroatomes de la série oxygène, soufre et azote,
représente phénoxy-C₁-C₆-alkyle le cas échéant substitué par halogène ou C₁-C₆-alkyle, ou
représente hétaryloxy-C₁-C₆-alkyle de 5 ou 6 chaînons, le cas échéant substitué par halogène, amino ou C₁-C₆-alkyle, présentant un ou deux hétéroatomes de la série oxygène, soufre et azote,
R² représente C₁-C₂₀-alkyle, C₂-C₂₀-alcényle, C₁-C₈-alcoxy-C₂-C₈-alkyle ou poly-C₁-C₈-alcoxy-C₂-C₈-alkyle à chaque fois le cas échéant substitué par halogène ou cyano,
représente C₃-C₈-cycloalkyle le cas échéant substitué par halogène, C₁-C₆-alkyle ou C₁-C₆-alcoxy ou
représente phényle ou benzyle à chaque fois le cas échéant substitué par halogène, cyano, nitro, C₁-C₆-alkyle, C₁-C₆-alcoxy, C₁-C₆-halogénoalkyle ou C₁-C₆-halogénoalcoxy,
R³ représente C₁-C₈-alkyle le cas échéant substitué par halogène ; ou phényle ou benzyle à chaque fois le cas échéant substitué par halogène, C₁-C₆-alkyle, C₁-C₆-alcoxy, C₁-C₄-halogénoalkyle, C₁-C₄-halogénoalcoxy, cyano ou nitro,
R⁴ et R⁵ représentent, indépendamment l'un de l'autre, C₁-C₈-alkyle, C₁-C₈-alcoxy, C₁-C₈-alkylamino, di-(C₁-C₈-alkyl)amino, C₁-C₈-alkylthio ou C₃-C₈-alcénylthio à chaque fois le cas échéant substitué par halogène ; ou phényle, phénoxy ou phénylthio à chaque fois le cas échéant substitué par halogène, nitro, cyano, C₁-C₄-alcoxy, C₁-C₄-halogénoalcoxy, C₁-C₄-alkylthio, C₁-C₄-halogénoalkylthio, C₁-C₄-alkyle ou C₁-C₄-halogénoalkyle,
R⁶ et R⁷ représentent, indépendamment l'un de l'autre, hydrogène ; C₁-C₈-alkyle, C₃-C₈-cycloalkyle, C₁-C₈-alcoxy, C₃-C₈-alcényle ou C₁-C₈-alcoxy-C₂-C₈-alkyle à chaque fois le cas échéant substitué par halogène ou cyano ; phényle ou benzyle à chaque fois le cas échéant substitué par halogène, C₁-C₈-alkyle, C₁-C₈-halogénoalkyle ou C₁-C₈-alcoxy ; ou ensemble un radical C₃-C₆-alkylène le cas échéant substitué par C₁-C₆-alkyle, dans lequel le cas échéant un groupe méthylène est remplacé par oxygène ou soufre.

2. Composés de formule (I) selon la revendication 1, où
W représente hydrogène, chlore, brome, C₁-C₄-alkyle, C₂-C₄-alcényle, C₂-C₄-alcynyle ; C₃-C₆-cycloalkyle le cas échéant monosubstitué par méthyle, éthyle, méthoxy, fluor, chlore, trifluorométhyle ou cyclopropyle ; C₁-C₄-alcoxy, C₁-C₂-halogénoalkyle ou C₁-C₂-halogénoalcoxy,
X représente chlore, brome, C₁-C₄-alkyle, C₂-C₄-alcényle, C₂-C₄-alcynyle ; C₃-C₆-cycloalkyle le cas échéant monosubstitué par méthyle, éthyle, méthoxy, fluor, chlore, trifluorométhyle ou cyclopropyle ; C₁-C₄-alcoxy, C₁-C₄-halogénoalkyle, C₁-C₄-halogénoalcoxy ou cyano,
Y et Z représentent, indépendamment l'un de l'autre, hydrogène, fluor, chlore, brome, C₁-C₄-alkyle, C₂-C₄-alcényle, C₂-C₄-alcynyle ; C₃-C₆-cycloalkyle le cas échéant monosubstitué par méthyle, éthyle, méthoxy, fluor, chlore, trifluorométhyle ou cyclopropyle ; C₁-C₆-alcoxy, C₁-C₄-halogénoalkyle, C₁-C₄-halogénoalcoxy, cyano, C₂-C₄-alcényle, C₂-C₄-alcynyle ou un des radicaux (Het)-aryle, 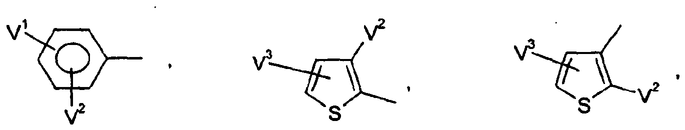 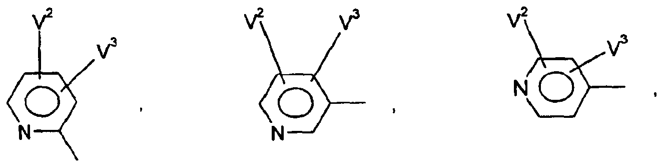 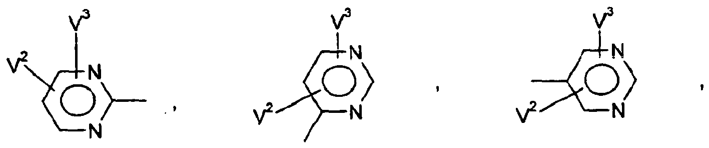 où, dans le cas de (Het)-aryle, seul un des radicaux Y ou Z peut représenter (Het)-aryle ;
V¹ représente hydrogène, fluor, chlore, brome, C₁-C₆-alkyle, C₁-C₄-alcoxy, C₁-C₂-halogénoalkyle, C₁-C₂-halogénoalcoxy, nitro, cyano ; ou phényle à chaque fois le cas échéant monosubstitué à disubstitué par fluor, chlore, brome, C₁-C₄-alkyle, C₁-C₄-alcoxy, C₁-C₂-halogénoalkyle, C₁-C₂-halogénoalcoxy, nitro ou cyano,
V² et V³ représentent, indépendamment l'un de l'autre, hydrogène, fluor, chlore, brome, C₁-C₄-alkyle, C₁-C₄-alcoxy, C₁-C₂-halogénoalkyle ou C₁-C₂-halogénoalcoxy,
A représente C₁-C₄-alcoxy monosubstitué à pentasubstitué par fluor, chlore et/ou brome ; ou C₃-C₆-cycloalkyl-C₁-C₂-alcoxy monosubstitué à trisubstitué par fluor et/ou chlore,
D représente NH (1) ou oxygène (2),
Q¹ et Q² représentent, indépendamment l'un de l'autre, hydrogène, méthyle, éthyle, trifluorométhyle, méthoxy ou éthoxy,
m vaut le nombre 0 ou 1,
G représente hydrogène (a) ou un des groupes 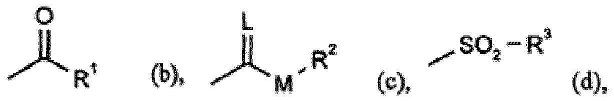 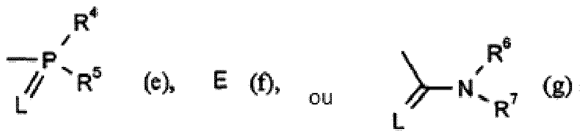 dans lesquels
E représente un ion métallique ou un ion d'ammonium,
L représente oxygène ou soufre et
M représente oxygène ou soufre,
R¹ représente C₁-C₁₆-alkyle, C₂-C₁₆-alcényle, C₁-C₆-alcoxy-C₁-C₄-alkyle, C₁-C₆-alkylthio-C₁-C₄-alkyle ou poly-C₁-C₆-alcoxy-C₁-C₄-alkyle à chaque fois le cas échéant monosubstitué à trisubstitué par fluor ou chlore ; ou C₃-C₇-cycloalkyle le cas échéant monosubstitué à disubstitué par fluor, chlore, C₁-C₅-alkyle ou C₁-C₅-alcoxy, dans lequel le cas échéant un ou deux groupes méthylène non directement adjacents sont remplacés par oxygène et/ou soufre,
représente phényle le cas échéant monosubstitué à trisubstitué par fluor, chlore, brome, cyano, nitro, C₁-C₄-alkyle, C₁-C₄-alcoxy, C₁-C₃-halogénoalkyle, C₁-C₃-halogénoalcoxy, C1-C₄-alkylthio ou Cₗ-C₄-alkylsulfonyle,
représente phényl-C₁-C₄-alkyle le cas échéant monosubstitué à disubstitué par fluor, chlore, brome, C₁-C₄-alkyle, C₁-C₄-alcoxy, C₁-C₃-halogénoalkyle ou C₁-C₃-halogénoalcoxy, représente pyrazolyle, thiazolyle, pyridyle, pyrimidyle, furannyle ou thiényle à chaque fois le cas échéant monosubstitué à disubstitué par fluor, chlore, brome ou C₁-C₄-alkyle,
représente phénoxy-C₁-C₅-alkyle le cas échéant monosubstitué à disubstitué par fluor, chlore, brome ou C₁-C₄-alkyle ou
représente pyridyloxy-C₁-C₅-alkyle, pyrimidyloxy-C₁-C₅-alkyle ou thiazolyloxy-C₁-C₅-alkyle à chaque fois le cas échéant monosubstitué à disubstitué par fluor, chlore, brome, amino ou C₁-C₄-alkyle,
R² représente de manière particulièrement préférée C₁-C₁₆-alkyle, C₂-C₁₆-alcényle, C₁-C₆-alcoxy-C₂-C₆-alkyle ou poly-C₁-C₆-alcoxy-C₂-C₆-alkyle à chaque fois le cas échéant monosubstitué à trisubstitué par fluor ou chlore,
représente C₃-C₇-cycloalkyle le cas échéant monosubstitué à disubstitué par fluor, chlore, C₁-C₄-alkyle ou C₁-C₄-alcoxy ou
représente phényle ou benzyle à chaque fois le cas échéant monosubstitué à trisubstitué par fluor, chlore, brome, cyano, nitro, C₁-C₄-alkyle, C₁-C₃-alcoxy, C₁-C₃-halogénoalkyle ou C₁-C₃-halogénoalcoxy,
R³ représente C₁-C₆-alkyle le cas échéant monosubstitué à trisubstitué par fluor ou chlore ; ou phényle ou benzyle à chaque fois le cas échéant monosubstitué à disubstitué par fluor, chlore, brome, C₁-C₄-alkyle, C₁-C₄-alcoxy, C₁-C₂-halogénoalkyle, C₁-C₂-halogénoalcoxy, cyano ou nitro,
R⁴ et R⁵ représentent, indépendamment l'un de l'autre, C₁-C₆-alkyle, C₁-C₆-alcoxy, C₁-C₆-alkylamino, di-(C₁-C₆-alkyl)amino, C₁-C₆-alkylthio ou C₃-C₄-alcénylthio à chaque fois le cas échéant monosubstitué à trisubstitué par fluor ou chlore ; ou phényle, phénoxy ou phénylthio à chaque fois le cas échéant monosubstitué à disubstitué par fluor, chlore, brome, nitro, cyano, C₁-C₃-alcoxy, C₁-C₃-halogénoalcoxy, C₁-C₃-alkylthio, C₁-C₃-halogénoalkylthio, C₁-C₃-alkyle ou C₁-C₃-halogénoalkyle,
R⁶ et R⁷ représentent, indépendamment l'un de l'autre, hydrogène ; C₁-C₆-alkyle, C₃-C₆-cycloalkyle, C₁-C₆-alcoxy, C₃-C₆-alcényle ou C₁-C₆-alcoxy-C₂-C₆-alkyle à chaque fois le cas échéant monosubstitué à trisubstitué par fluor ou chlore ; phényle ou benzyle à chaque fois le cas échéant monosubstitué à trisubstitué par fluor, chlore, brome, C₁-C₅-halogénoalkyle, C₁-C₅-alkyle ou C₁-C₅-alcoxy ; ou ensemble un radical C₃-C₆-alkylène le cas échéant substitué par C₁-C₄-alkyle, dans lequel le cas échéant un groupe méthylène est remplacé par oxygène ou soufre.

3. Composés de formule (I) selon la revendication 1, où
W représente hydrogène, chlore, brome, méthyle, éthyle, vinyle, éthynyle, propynyle, cyclopropyle, méthoxy, éthoxy ou trifluorométhyle,
X représente chlore, brome, méthyle, éthyle, propyle, iso-propyle, vinyle, éthynyle, propynyle, cyclopropyle, méthoxy, éthoxy, trifluorométhyle, difluorométhoxy, trifluorométhoxy ou cyano,
Y et Z représentent, indépendamment l'un de l'autre, hydrogène, fluor, chlore, brome, méthyle, éthyle, vinyle, éthynyle, propynyle, cyclopropyle, méthoxy, trifluorométhyle, trifluorométhoxy, cyano ou un radical phényle, 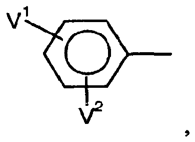 où, dans le cas de phényle, seul un des radicaux Y ou Z peut représenter phényle ;
V¹ représente hydrogène, fluor, chlore, brome, méthyle, éthyle, n-propyle, iso-propyle, tert-butyle, méthoxy, éthoxy, n-propoxy, iso-propoxy, trifluorométhyle ou trifluorométhoxy,
V² représente hydrogène, fluor, chlore, méthyle, éthyle, n-propyle, iso-propyle, méthoxy, éthoxy ou trifluorométhyle,
A représente méthoxy, éthoxy, propoxy, butoxy ou isobutoxy à chaque fois monosubstitué à trisubstitué par fluor et/ou chlore ; ou cyclopropylméthoxy ou cyclopropyléthoxy à chaque fois monosubstitué à trisubstitué par fluor et/ou chlore,
D représente NH (1) ou oxygène (2),
Q¹ et Q² représentent hydrogène,
m vaut le nombre 1,
G représente hydrogène (a) ou un des groupes 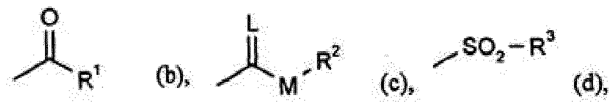 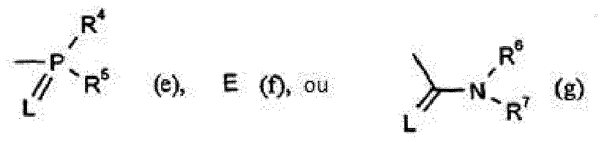 dans lesquels
E représente un ion métallique ou un ion d'ammonium,
L représente oxygène ou soufre et
M représente oxygène ou soufre,
R¹ représente C₁-C₁₀-alkyle, C₂-C₁₀-alcényle, C₁-C₄-alcoxy-C₁-C₂-alkyle, C₁-C₄-alkylthio-C₁-C₂-alkyle à chaque fois le cas échéant monosubstitué à trisubstitué par fluor ou chlore ; ou C₃-C₆-cycloalkyle le cas échéant monosubstitué par fluor, chlore, méthyle, éthyle ou méthoxy, représente phényle le cas échéant monosubstitué à disubstitué par fluor, chlore, brome, cyano, nitro, méthyle, éthyle, n-propyle, i-propyle, méthoxy, éthoxy, trifluorométhyle ou trifluorométhoxy,
représente furannyle, thiényle ou pyridyle à chaque fois le cas échéant monosubstitué par chlore, brome ou méthyle,
R² représente C₁-C₁₀-alkyle, C₂-C₁₀-alcényle ou C₁-C₄-alcoxy-C₂-C₄-alkyle à chaque fois le cas échéant monosubstitué à trisubstitué par fluor ou chlore, représente cyclopentyle ou cyclohexyle ou
représente phényle ou benzyle à chaque fois le cas échéant monosubstitué à disubstitué par fluor, chlore, cyano, nitro, méthyle, éthyle, méthoxy, trifluorométhyle ou trifluorométhoxy,
R³ représente méthyle, éthyle, propyle ou iso-propyle à chaque fois le cas échéant monosubstitué à trisubstitué par fluor ou chlore ; ou phényle à chaque fois le cas échéant monosubstitué par fluor, chlore, brome, méthyle, éthyle, iso-propyle, tert-butyle, méthoxy, éthoxy, iso-propoxy, trifluorométhyle, trifluorométhoxy, cyano ou nitro,
R⁴ et R⁵ représentent, indépendamment l'un de l'autre, C₁-C₄-alcoxy ou C₁-C₄-alkylthio ; ou phényle, phénoxy ou phénylthio à chaque fois le cas échéant monosubstitué par fluor, chlore, brome, nitro, cyano, méthyle, méthoxy, trifluorométhyle ou trifluorométhoxy,
R⁶ et R⁷ représentent, indépendamment l'un de l'autre, hydrogène, C₁-C₄-alkyle, C₃-C₆-cycloalkyle, C₁-C₄-alcoxy, C₃-C₄-alcényle ou C₁-C₄-alcoxy-C₂-C₄-alkyle ; phényle à chaque fois le cas échéant monosubstitué à disubstitué par fluor, chlore, brome, méthyle, méthoxy ou trifluorométhyle ; ou ensemble un radical C₅-C₆-alkylène dans lequel le cas échéant un groupe méthylène est remplacé par oxygène ou soufre.

4. Composés de formule (I) selon la revendication 1, où
W représente hydrogène, chlore, brome, méthyle, éthyle ou méthoxy,
X représente chlore, brome, méthyle, éthyle, méthoxy ou éthoxy,
Y et Z représentent, indépendamment l'un de l'autre, hydrogène, chlore, brome, méthyle, méthoxy, cyclopropyle ou les radicaux 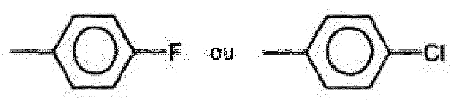 où, dans ce cas, seul un des radicaux Y ou Z peut représenter un radical phényle substitué ;
A représente méthoxy, éthoxy ou propoxy le cas échéant à chaque fois monosubstitué à trisubstitué par fluor et/ou chlore,
D représente NH (1) ou oxygène (2),
Q¹ et Q² représentent hydrogène,
m vaut le nombre 1,
G représente hydrogène (a) ou un des groupes 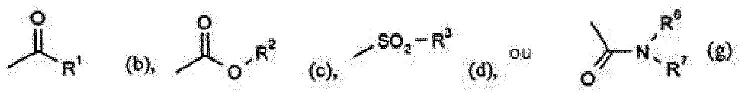
R¹ représente C₁-C₁₀-alkyle, C₁-C₄-alcoxy-C₁-C₂-alkyle, C₃-C₆-cycloalkyle,
représente phényle le cas échéant monosubstitué par chlore ou thiényle,
R² représente C₁-C₁₀-alkyle, C₂-C₁₀-alcényle ou benzyle,
R³ représente méthyle,
R⁶ et R⁷ représentent ensemble un radical C₅-C₆-alkylène, dans lequel le cas échéant un groupe méthylène est remplacé par oxygène ou soufre.

5. Procédé pour la préparation de composés de formule (I) selon la revendication 1, **caractérisé en ce que**, pour obtenir
(A) des composés de formule (I-1-a) 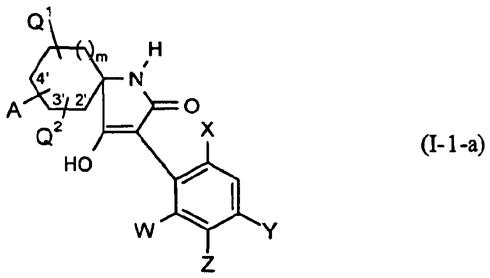 dans laquelle
A, Q¹, Q², m, W, X, Y et Z présentent les significations indiquées ci-dessus,
on condense des composés de formule (II) 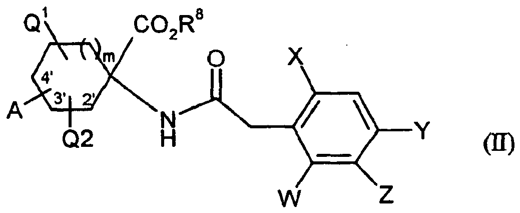 dans laquelle
A, Q¹, Q², m, W, X, Y et Z présentent les significations indiquées ci-dessus et
R⁸ représente alkyle,
de manière intramoléculaire en présence d'un diluant et en présence d'une base,
(B) des composés de formule (I-2-a) 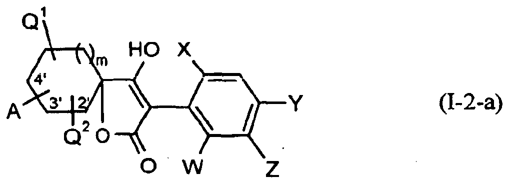 dans laquelle
A, Q¹, Q², m, W, X, Y et Z présentent les significations indiquées ci-dessus,
on condense des composés de formule (III) 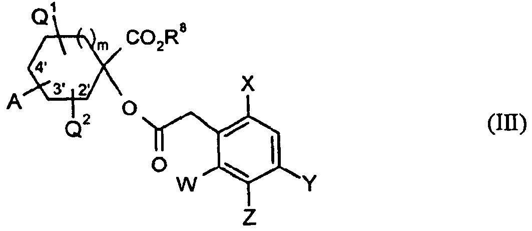 dans laquelle
A, Q¹, Q², m, W, X, Y et Z et R⁸ présentent les significations indiquées ci-dessus,
de manière intramoléculaire en présence d'un diluant et en présence d'une base,
(C) des composés des formules (I-1-b) à (I-2-b) représentées ci-dessus, dans lesquelles R¹, A, Q¹, Q², m, W, X, Y et Z présentent les significations indiquées ci-dessus, on transforme des composés des formules (I-1-a) à (I-2-a) représentées ci-dessus, dans lesquelles A, Q¹, Q², m, W, X, Y et Z présentent les significations indiquées ci-dessus, à chaque fois
α) avec des composés de formule (IV) 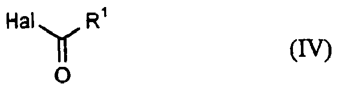 dans laquelle
R¹ présente la signification indiquée ci-dessus et
Hal représente halogène,
soit
β) avec des anhydrides d'acide carboxylique de formule (V)
R¹-CO-O-CO-R¹ (V)
dans laquelle
R¹ présente la signification indiquée ci-dessus,
le cas échéant en présence d'un diluant et le cas échéant en présence d'un liant d'acide ;
(D) des composés des formules (I-1-c) à (I-2-c) représentées ci-dessus, dans lesquelles R², A, Q¹, Q², m, W, M, X, Y et Z présentent les significations indiquées ci-dessus et L représente oxygène, on transforme des composés des formules (I-1-a) à (I-2-a) représentées ci-dessus, dans lesquelles A, Q¹, Q², m, W, X, Y et Z présentent les significations indiquées ci-dessus, à chaque fois
avec des esters de l'acide chloroformique ou des thioesters de l'acide chloroformique de formule (VI)
R²-M-CO-Cl (VI)
dans laquelle
R² et M présentent les significations indiquées ci-dessus,
le cas échéant en présence d'un diluant et le cas échéant en présence d'un liant d'acide ;
(E) des composés des formules (I-1-c) à (I-2-c) représentées ci-dessus, dans lesquelles R², A, Q¹, Q², m, W, M, X, Y et Z présentent les significations indiquées ci-dessus et L représente soufre, on transforme des composés des formules (I-1-a) à (I-2-a) représentées ci-dessus, dans lesquelles A, Q¹, Q², m, W, X, Y et Z présentent les significations indiquées ci-dessus, à chaque fois
avec des esters de l'acide chloromonothioformique ou des esters de l'acide chlorodithioformique de formule (VII) 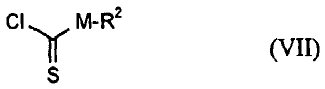 dans laquelle
M et R² présentent les significations indiquées ci-dessus,
le cas échéant en présence d'un diluant et le cas échéant en présence d'un liant d'acide,
(F) des composés des formules (I-1-d) à (I-2-d) représentées ci-dessus, dans lesquelles R³, A, W, Q¹, Q², m, X, Y et Z présentent les significations indiquées ci-dessus, on transforme des composés des formules (I-1-a) à (I-2-a) représentées ci-dessus, dans lesquelles A, B, Q¹, Q², m, W, X, Y et Z présentent les significations indiquées ci-dessus, à chaque fois
avec des chlorures d'acide sulfonique de formule (VIII)
R³-SO₂-Cl (VIII)
dans laquelle
R³ présente la signification susmentionnée,
le cas échéant en présence d'un diluant et le cas échéant en présence d'un liant d'acide,
(G) des composés des formules (I-1-e) à (I-2-e) représentées ci-dessus, dans lesquelles L, R⁴, R⁵, A, Q¹, Q², m, W, X, Y et Z présentent les significations indiquées ci-dessus, on transforme des composés des formules (I-1-a) à (I-2-a) représentées ci-dessus, dans lesquelles A, Q¹, Q², m, W, X, Y et Z présentent les significations indiquées ci-dessus, à chaque fois
avec des composés phosphorés de formule (IX) 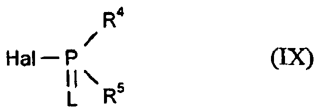 dans laquelle
L, R⁴ et R⁵ présentent les significations indiquées ci-dessus, et
Hal représente halogène,
le cas échéant en présence d'un diluant et le cas échéant en présence d'un liant d'acide,
(H) des composés des formules (I-1-f) à (I-2-f) représentées ci-dessus, dans lesquelles E, A, Q¹, Q², m, W, X, Y et Z présentent les significations indiquées ci-dessus, on transforme des composés des formules (I-1-a) à (I-2-a), dans lesquelles A, Q¹, Q², m, W, X, Y et Z présentent les significations indiquées ci-dessus, à chaque fois avec des composés métalliques ou des amines des formules (X) ou (XI)
**Me(OR¹⁰)ₜ** **(X)** 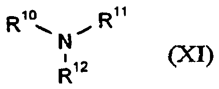
dans lesquelles
Me représente un métal monovalent ou divalent,
t vaut le nombre 1 ou 2 et
R¹⁰, R¹¹, R¹² représentent, indépendamment les uns des autres, hydrogène ou alkyle, le cas échéant en présence d'un diluant,
(I) des composés des formules (I-1-g) à (I-2-g) représentées ci-dessus, dans lesquelles L, R⁶, R⁷, A, Q¹, Q², m, W, X, Y et Z présentent les significations indiquées ci-dessus, on transforme des composés des formules (I-1-a) à (I-2-a) représentées ci-dessus, dans lesquelles A, Q¹, Q², m, W, X, Y et Z présentent les significations indiquées ci-dessus, à chaque fois
α) avec des isocyanates ou des isothiocyanates de formule (XII)
R⁶-N=C=L (XII)
dans laquelle
R⁶ et L présentent les significations indiquées ci-dessus,
le cas échéant en présence d'un diluant et le cas échéant en présence d'un catalyseur ou
β) avec des chlorures d'acide carbamique ou des chlorures d'acide thiocarbamique de formule (XIII) 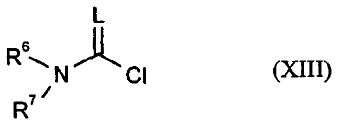 dans laquelle
L, R⁶ et R⁷ présentent les significations indiquées ci-dessus,
le cas échéant en présence d'un diluant et le cas échéant en présence d'un liant d'acide,
(Jα) des composés des formules (I-1a) à (I-1-g) représentées ci-dessus, dans lesquelles A, D, G, Q¹, Q², m, W, X, Y et Z présentent la signification indiquée ci-dessus, on couple des composés des formules (I-1-a') à (I-2-g'), dans lesquelles A, D, G, Q¹, Q², m, W, X, Y et Z présentent la signification mentionnée ci-dessus et Z' représente brome ou iode 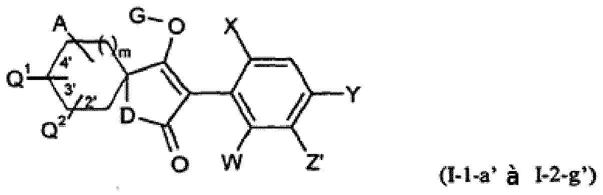 et
(Jβ) des composés des formules (I-1-a) à (I-2-g) représentées ci-dessus, dans lesquelles A, D, G, Q¹, Q², m, W, X, Y et Z présentent la signification indiquée ci-dessus, on couple des composés des formules (I-1-a") à (I-2-g"), dans lesquelles A, D, G, Q¹, Q², m, W, X, Y et Z présentent la signification mentionnée ci-dessus et Y' représente brome ou iode 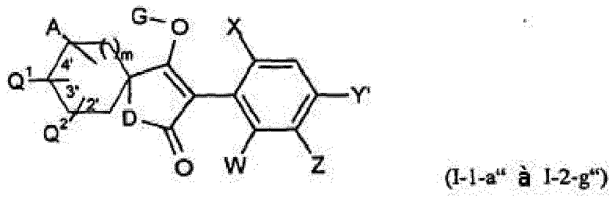 avec des dérivés (Het)-aryle aptes au couplage des formules (XVα) et (XVß) 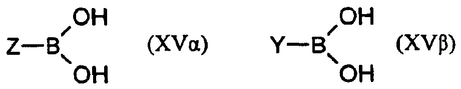 ou leurs esters en présence d'un solvant, en présence d'un catalyseur et en présence d'une base.

6. Agent pour lutter contre des organismes nuisibles et/ou une croissance non souhaitée de plantes, **caractérisé par** une teneur en au moins un composé de formule (I) selon la revendication 1.

7. Procédé pour lutter contre des organismes nuisibles animaux et/ou une couverture végétale non souhaitée, **caractérisé en ce qu'**on laisse agir des composés de formule (I) selon la revendication 1 sur des organismes nuisibles, sur une croissance non souhaitée des plantes et/ou sur leur espace de vie, à l'exception de procédés de traitement chirurgical ou thérapeutique du corps humain et animal.

8. Composés de formule (I) selon la revendication 1 pour lutter contre des organismes nuisibles animaux et/ou une couverture végétale non souhaitée.

9. Procédé pour la préparation d'agents destinés à lutter contre des organismes nuisibles et/ou une couverture végétale non souhaitée, **caractérisé en ce qu'**on mélange des composés de formule (I) selon la revendication 1 avec des agents d'allongement et/ou des substances tensioactives.

10. Utilisation de composés de formule (I) selon la revendication 1 pour la préparation d'agents destinés à lutter contre des organismes nuisibles et/ou une croissance non souhaitée des plantes.

11. Agent contenant une teneur active en une combinaison de substances actives comprenant, comme composants
(a') au moins un composé de formule (I), dans laquelle A, D, G, Q¹, Q², m, W, X, Y et Z présentent la signification indiquée ci-dessus et
(b') au moins un composé améliorant la tolérance des plantes de culture du groupe suivant de composés :
4-dichloroacétyl-1-oxa-4-azaspiro[4,5]-décane (AD-67, MON-4660), 1-dichloroacétylhexahydro-3,3,8a-triméthylpyrrolo[1,2-a]-pyrimidin-6(2H)-one (Dicyclonon, BAS-145138), 4-dichloroacétyl-3,4-dihydro-3-méthyl-2H-1,4-benzoxazine (Benoxacor), ester 1-méthylhexylique de l'acide 5-chloroquinoléin-8-oxyacétique (Cloquintocet-mexyl - cf. également les composés apparentés dans EP-A-86750, EP-A-94349, EP-A-191736, EP-A-492366), 3-(2-chlorobenzyl)-1-(1-méthyl-1-phényléthyl)-urée (Cumyluron), α-(cyanométhoximino)-phénylacétonitrile (Cyometrinil), acide 2,4-dichlorophénoxyacétique (2,4-D), acide 4-(2,4-dichlorophénoxy)-butyrique (2,4-DB), 1-(1-méthyl-1-phényléthyl)-3-(4-méthylphényl)-urée (Daimuron, Dymron), acide 3,6-dichloro-2-méthoxybenzoïque (Dicamba), ester S-1-méthyl-1-phényléthylique de l'acide pipéridine-1-thiocarboxylique (Dimepiperate), 2,2-dichloro-N-(2-oxo-2-(2-propénylamino)-éthyl)-N-(2-propényl)-acétamide (DKA-24), 2,2-dichloro-N,N-di-2-propénylacétamide (Dichlormid), 4,6-dichloro-2-phénylpyrimidine (Fenclorim), ester éthylique de l'acide 1-(2,4-dichlorophényl)-5-trichlorométhyl-1H-1,2,4-triazole-3-carboxylique (Fenchlorazol-éthyl - cf. également les composés apparentés dans EP-A-174562 et EP-A-346620), ester phénylméthylique de l'acide 2-chloro-4-trifluorométhylthiazole-5-carboxylique (Flurazole), 4-chloro-N-(1,3-dioxolan-2-ylméthoxy)-α-trifluoroacétophénonoxime (Fluxofenim), 3-dichloroacétyl-5-(2-furannyl)-2,2-diméthyloxazolidine (Furilazole, MON-13900), 4,5-dihydro-5,5-diphényl-3-isoxazole-carboxylate d'éthyle (Isoxadifen-éthyl - cf. également les composés apparentés dans WO-A-95/07897), 3,6-dichloro-2-méthoxybenzoate de 1-(éthoxycarbonyl)-éthyle (Lactidichlor), acide (4-chloro-o-toluyloxy)-acétique (MCPA), acide 2-(4-chloro-o-toluyloxy)-propionique (Mecoprop), 1-(2,4-dichlorophényl)-4,5-dihydro-5-méthyl-1H-pyrazole-3,5-dicarboxylate de diéthyle (Mefenpyrdiéthyl-cf. également les composés apparentés dans WO-A-91/07874), 2-dichlorométhyl-2-méthyl-1,3-dioxolane (MG-191), 1-oxa-4-azaspiro[4,5]décane-4-carbodithioate de 2-propényle (MG-838), anhydride de l'acide 1,8-naphtalénique, α-(1,3-dioxolan-2-ylméthoximino)-phénylacétonitrile (Oxabetrinil), 2,2-dichloro-N-(1,3-dioxolan-2-ylméthyl)-N-(2-propényl)-acétamide (PPG-1292), 3-dichloroacétyl-2,2-diméthyloxazolidine (R-28725), 3-dichloroacétyl-2,2,5-triméthyloxazolidine (R-29148), acide 4-(4-chloro-o-toluyl)-butyrique, acide 4-(4-chlorophénoxy)-butyrique, acide diphénylméthoxyacétique, ester méthylique de l'acide diphénylméthoxyacétique, ester éthylique de l'acide diphénylméthoxyacétique, ester méthylique de l'acide 1-(2-chlorophényl)-5-phényl-1H-pyrazole-3-carboxylique, ester éthylique de l'acide 1-(2,4-dichlorophényl)-5-méthyl-1H-pyrazole-3-carboxylique, ester éthylique de l'acide 1-(2,4-dichlorophényl)-5-isopropyl-1H-pyrazole-3-carboxylique, ester éthylique de l'acide 1-(2,4-dichlorophényl)-5-(1,1-diméthyléthyl)-1H-pyrazole-3-carboxylique, ester éthylique de l'acide 1-(2,4-dichlorophényl)-5-phényl-1H-pyrazole-3-carboxylique (cf. également les composés apparentés dans EP-A-269806 et EP-A-333131), ester éthylique de l'acide 5-(2,4-dichlorobenzyl)-2-isoxazoline-3-carboxylique, ester éthylique de l'acide 5-phényl-2-isoxazoline-3-carboxylique, ester éthylique de l'acide 5-(4-fluorophényl)-5-phényl-2-isoxazoline-3-carboxylique (cf. également les composés apparentés dans WO-A-91/08202), ester 1,3-diméthylbut-1-ylique de l'acide 5-chloroquinoléin-8-oxyacétique, ester 4-allyloxybutylique de l'acide 5-chloroquinoléin-8-oxyacétique, ester 1-allyloxyprop-2-ylique de l'acide 5-chloroquinoléin-8-oxyacétique, ester méthylique de l'acide 5-chloroquinoxalin-8-oxyacétique, ester éthylique de l'acide 5-chloroquinoléin-8-oxyacétique, ester allylique de l'acide 5-chloroquinoxalin-8-oxyacétique, ester 2-oxoprop-1-ylique de l'acide 5-chloroquinoléin-8-oxyacétique, ester diéthylique de l'acide 5-chloroquinoléin-8-oxymalonique, ester diallylique de l'acide 5-chloroquinoxalin-8-oxymalonique, ester diéthylique de l'acide 5-chloroquinoléin-8-oxymalonique (cf. également les composés apparentés dans EP-A-582198), acide 4-carboxychroman-4-ylacétique (AC-304415 - cf. EP-A-613618), acide 4-chlorophénoxyacétique, 3,3'-diméthyl-4-méthoxybenzophénone, 1-bromo-4-chlorométhylsulfonylbenzène, 1-[4-(N-2-méthoxybenzoylsulfamoyl)-phényl]-3-méthylurée (alias N-(2-méthoxybenzoyl)-4-[(méthylaminocarbonyl)-amino]-benzènesulfonamide), 1-[4-(N-2-méthoxybenzoylsulfamoyl)-phényl]-3,3-diméthylurée, 1-[4-(N-4,5-diméthylbenzoylsulfamoyl)-phényl]-3-méthylurée, 1-[4-(N-naphtylsulfamoyl)-phényl]-3,3-diméthylurée, N-(2-méthoxy-5-méthylbenzoyl)-4-(cyclopropylaminocarbonyl)-benzènesulfonamide
et/ou un des composés suivants, définis par des formules générales, de formule générale (IIa) 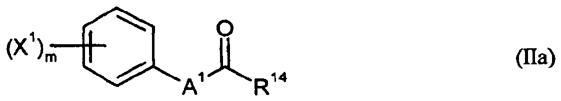 ou de formule générale (IIb) 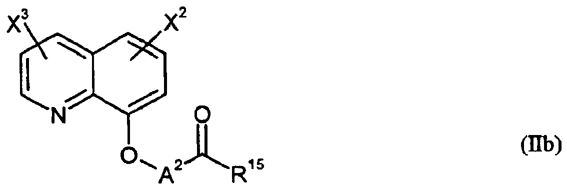 ou de formule (IIc) 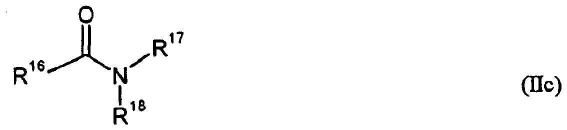 où
m vaut le nombre 0, 1, 2, 3, 4 ou 5,
A¹ représente un des groupes hétérocycliques divalents esquissés ci-après, 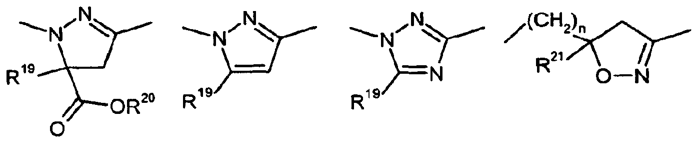
n vaut le nombre 0, 1, 2, 3, 4 ou 5,
A² représente alcanediyle, comprenant 1 ou 2 atomes de carbone, le cas échéant substitué par C₁-C₄-alkyle et/ou C₁-C₄-alcoxycarbonyle et/ou C₁-C₄-alcényloxycarbonyle,
R¹⁴ représente hydroxy, mercapto, amino, C₁-C₆-alcoxy, C₁-C₆-alkylthio, C₁-C₆-alkylamino ou di-(C₁-C₄-alkyl)-amino,
R¹⁵ représente hydroxy, mercapto, amino, C₁-C₇-alcoxy, C₁-C₆-alkylthio, C₁-C₆-alcényloxy, C₁-C₆-alcényloxy-C₁-C₆-alcoxy, C₁-C₆-alkylamino ou di-(C₁-C₄-alkyl)-amino,
R¹⁶ représente C₁-C₄-alkyle le cas échéant substitué par fluor, chlore et/ou brome,
R¹⁷ représente hydrogène ; C₁-C₆-alkyle, C₂-C₆-alcényle ou C₂-C₆-alcynyle à chaque fois le cas échéant substitué par fluor, chlore et/ou brome ; C₁-C₄-alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou par C₁-C₄-alkyle,
R¹⁸ représente hydrogène ; C₁-C₆-alkyle, C₂-C₆-alcényle ou C₂-C₆-alcynyle à chaque fois le cas échéant substitué par fluor, chlore et/ou brome ; C₁-C₄-alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou C₁-C₄-alkyle,
R¹⁷ et R¹⁸ représentent également, ensemble, C₃-C₆-alcanediyle ou C₂-C₅-oxaalcanediyle à chaque fois le cas échéant substitué par C₁-C₄-alkyle, phényle, furyle, un cycle benzène annelé ou par deux substituants, qui forment, ensemble avec l'atome de carbone auquel ils sont liés, un carbocycle de 5 ou 6 chaînons,
R¹⁹ représente hydrogène, cyano, halogène ; ou C₁-C₄-alkyle, C₃-C₆-cycloalkyle ou phényle à chaque fois le cas échéant substitué par fluor, chlore et/ou brome,
R²⁰ représente hydrogène ; C₁-C₆-alkyle, C₃-C₆-cycloalkyle ou tri-(C₁-C₄-alkyl)-silyle à chaque fois le cas échéant substitué par hydroxy, cyano, halogène ou C₁-C₄-alcoxy,
R²¹ représente hydrogène, cyano, halogène ; ou C₁-C₄-alkyle, C₃-C₆-cycloalkyle ou phényle à chaque fois le cas échéant substitué par fluor, chlore et/ou brome,
X¹ représente nitro, cyano, halogène, C₁-C₄-alkyle, C₁-C₄-halogénoalkyle, C₁-C₄-alcoxy ou C₁-C₄-halogénoalcoxy,
X² représente hydrogène, cyano, nitro, halogène, C₁-C₄-alkyle, C₁-C₄-halogénoalkyle, C₁-C₄-alcoxy ou C₁-C₄-halogénoalcoxy,
X³ représente hydrogène, cyano, nitro, halogène, C₁-C₄-alkyle, C₁-C₄-halogénoalkyle, C₁-C₄-alcoxy ou C₁-C₄-halogénoalcoxy,
et/ou les composés suivants, définis par des formules générales, de formule générale (IId) 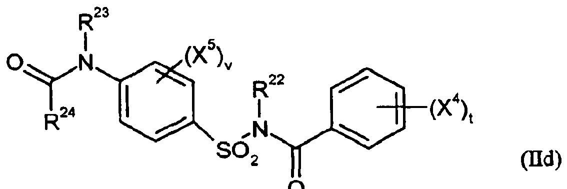 ou de formule générale (IIe) 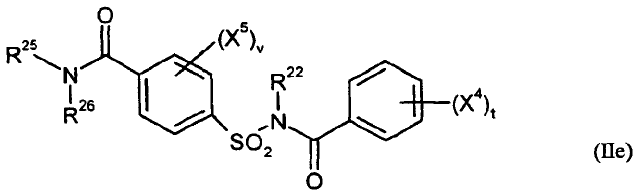 où
t vaut le nombre 0, 1, 2, 3, 4 ou 5,
v vaut le nombre 0, 1, 2, 3, 4 ou 5,
R²² représente hydrogène ou C₁-C₄-alkyle,
R²³ représente hydrogène ou C₁-C₄-alkyle,
R²⁴ représente hydrogène ; C₁-C₆-alkyle, C₁-C₆-alcoxy, C₁-C₆-alkylthio, C₁-C₆-alkylamino ou di-(C₁-C₄-alkyl)-amino à chaque fois le cas échéant substitué par cyano, halogène ou C₁-C₄-alcoxy ; ou C₃-C₆-cycloalkyle, C₃-C₆-cycloalkyloxy, C₃-C₆-cycloalkylthio ou C₃-C₆-cycloalkylamino à chaque fois le cas échéant substitué par cyano, halogène ou C₁-C₄-alkyle,
R²⁵ représente hydrogène ; C₁-C₆-alkyle le cas échéant substitué par cyano, hydroxy, halogène ou C₁-C₄-alcoxy ; C₃-C₆-alcényle ou C₃-C₆-alcynyle à chaque fois le cas échéant substitué par cyano ou halogène ; ou C₃-C₆-cycloalkyle le cas échéant substitué par cyano, halogène ou C₁-C₄-alkyle,
R²⁶ représente hydrogène ; C₁-C₆-alkyle le cas échéant substitué par cyano, hydroxy, halogène ou C₁-C₄-alcoxy ; C₃-C₆-alcényle ou C₃-C₆-alcynyle à chaque fois le cas échéant substitué par cyano ou halogène ; C₃-C₆-cycloalkyle le cas échéant substitué par cyano, halogène ou C₁-C₄-alkyle ; ou phényle le cas échéant substitué par nitro, cyano, halogène, C₁-C₄-alkyle, C₁-C₄-halogénoalkyle, C₁-C₄-alcoxy ou C₁-C₄-halogénoalcoxy ; ou, ensemble avec R²⁵, C₂-C₆-alcanediyle ou C₂-C₅-oxaalcanediyle à chaque fois le cas échéant substitué par C₁-C₄-alkyle,
X⁴ représente nitro, cyano, carboxy, carbamoyle, formyle, sulfamoyle, hydroxy, amino, halogène, C₁-C₄-alkyle, C₁-C₄-halogénoalkyle, C₁-C₄-alcoxy ou C₁-C₄-halogénoalcoxy, et
X⁵ représente nitro, cyano, carboxy, carbamoyle, formyle, sulfamoyle, hydroxy, amino, halogène, C₁-C₄-alkyle, C₁-C₄-halogénoalkyle, C₁-C₄-alcoxy ou C₁-C₄-halogénoalcoxy.

12. Agent selon la revendication 11, dans lequel le composé améliorant la tolérance des plantes de culture est choisi dans le groupe suivant de composés :
Cloquintocetmexyl, Fenchlorazole-ethyl, Isoxadifen-ethyl, Mefenpyr-diethyl, Furilazole, Fenclorim, Cumyluron, Dymron ou les composés 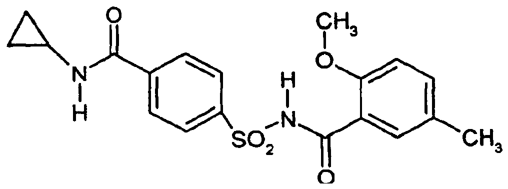 et

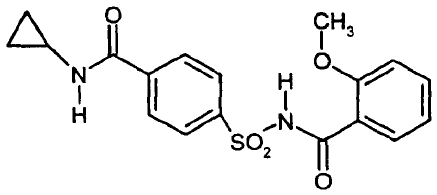

13. Agent selon l'une quelconque des revendications 11 ou 12, dans lequel le composé améliorant la tolérance des plantes de culture est le Cloquintocet-mexyl.

14. Agent selon l'une quelconque des revendications 11 ou 12, dans lequel le composé améliorant la tolérance des plantes de culture est le Mefenpyr-diethyl.

15. Procédé pour lutter contre la croissance non souhaitée de plantes, **caractérisé en ce qu'**on laisse agir un agent selon la revendication 11 sur les plantes ou leur environnement.

16. Utilisation d'un agent selon la revendication 11 pour lutter contre la croissance non souhaitée de plantes.

17. Procédé pour lutter contre la croissance non souhaitée de plantes, **caractérisé en ce qu'**on laisse agir un composé de formule (I) selon la revendication 1 et le composé améliorant la tolérance des plantes de culture selon la revendication 11 séparément, dans une succession proche dans le temps, sur les plantes ou leur environnement.

18. Composition comprenant
- au moins un composé de formule (I) selon la revendication 1 ou un agent selon la revendication 11 et
- au moins un sel de formule (III') 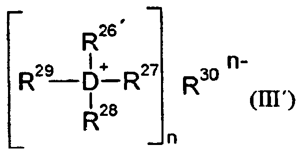 dans laquelle
D représente azote ou phosphore,
R²⁶, R²⁷, R²⁸ et R²⁹ représentent, indépendamment les uns des autres, hydrogène ; ou C₁-C₈-alkyle à chaque fois le cas échéant substitué ou C₁-C₈-alkylène monoinsaturé ou polyinsaturé, le cas échéant substitué, les substituants pouvant être choisis parmi halogène, nitro et cyano,
n vaut 1, 2, 3 ou 4,
R³⁰ représente un anion inorganique ou organique.

19. Composition selon la revendication 18, **caractérisée en ce qu'**elle contient au moins un agent favorisant la pénétration.

20. Procédé pour augmenter l'action d'agents de lutte contre les organismes nuisibles et/ou d'herbicides contenant une substance active de formule (I) selon la revendication 1 ou un agent selon la revendication 11, **caractérisé en ce que** l'agent prêt à l'emploi (bouillon à pulvériser) est préparé avec utilisation d'un sel de formule (III') selon la revendication 18.

21. Procédé selon la revendication 20, **caractérisé en ce que** le bouillon à pulvériser est préparé avec utilisation d'un agent favorisant la pénétration.

22. Composés de formule (II) 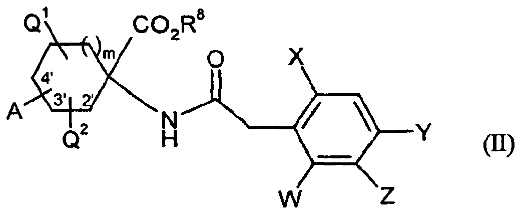 dans laquelle
A, m, Q¹, Q², W, X, Y et Z présentent la signification indiquée ci-dessus,

23. Composés de formule (III) 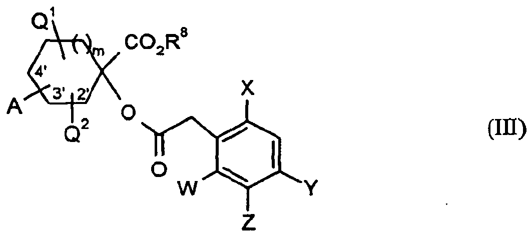 dans laquelle
A, m, Q¹, Q², W, X, Y, Z et R⁸ présentent la signification indiquée ci-dessus.

24. Composés de formule (XVI) 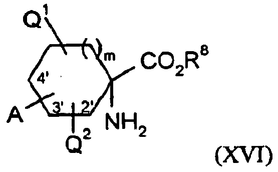 dans laquelle
A, m, Q¹, Q² et R⁸ présentent la signification indiquée ci-dessus.

25. Composés de formule (XVIII) 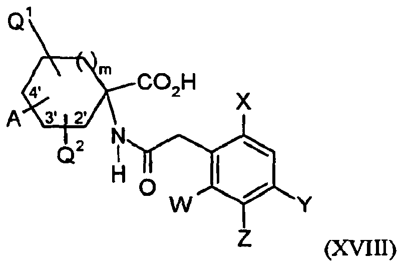 dans laquelle
A, Q¹, Q², m, W, X, Y et Z présentent la signification indiquée ci-dessus.

26. Composés de formule (XIX) 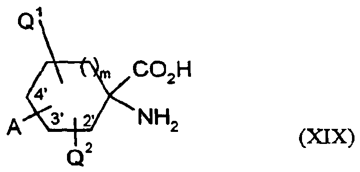 dans laquelle
A, m, Q¹ et Q² présentent la signification indiquée ci-dessus.

27. Composés de formule (XXI) 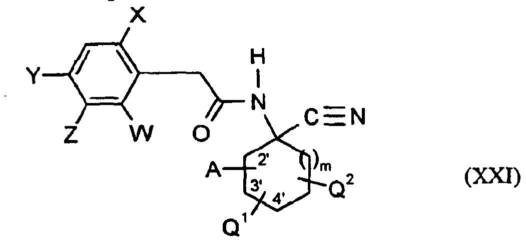 dans laquelle
A, m, Q¹, Q², W, X, Y et Z présentent la signification indiquée ci-dessus,

28. Composés de formule (XXII) 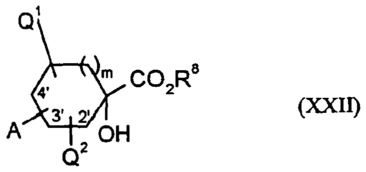 dans laquelle
A, m, Q¹, Q² et R⁸ présentent la signification indiquée ci-dessus.

29. Composés de formule (XXIII-1)
